# EUROPEAN PATENT SPECIFICATION

(11) **EP 2 148 865 B1**
(45) Date of publication and mention of the grant of the patent: **17.05.2017**
(21) Application number: 08747088.6
(22) Date of filing: 29.04.2008
(51) Int. Cl.: C07D 231/06, A61K 31/415, C07D 417/12, A61P 35/00

(54) **PYRAZOLE INHIBITORS OF WNT SIGNALING**
PYRAZOLINHIBITOREN DER WNT-SIGNALVERMITTLUNG
INHIBITEURS PYRAZOLÉS DE LA SIGNALISATION DES WNT

(30) Priority: 30.04.2007 US 915029 P
(43) Date of publication of application: 03.02.2010
(73) Proprietor: Genentech, Inc., South San Francisco, CA 94080 (US); Curis, Inc., Cambridge, MA 02138 (US)
(72) Inventor: BRUNTON, Shirley Ann, Windsor Berkshire UK SL4 3JX (GB); GUNZNER, Janet L., Berkeley, California 94705 (US); COULTER, Tom, Wantage Oxfordshire UK OX12 7PA (GB); STIBBARD, John H. A., Didcot Oxfordshire UK OX11 8QG (GB); MARSTERS, JR., James C., Oakland, California 94611 (US)
(74) Representative: Wytenburg, Wilhelmus Johannes
(86) International application number: PCT/US2008/061864
(87) International publication number: WO 2008/137408

(56) References cited:
- WO-A1-2005/079803
- WO-A1-2007/019933
- WO-A2-2005/033048
- WO-A2-2007/038425

## Description

### FIELD OF THE INVENTION

The present invention relates to organic compounds useful for therapy and/or prophylaxis in a mammal, in particular to compounds that inhibit the Wnt signaling pathway and are useful in the treatment of hyperproliferative diseases.

### BACKGROUND OF THE INVENTION

Human Wnt is a family of small (39-46 kD) secreted glycoproteins involved in embryogenesis through regulation of cell-to-cell interactions, control of cell proliferation and cell fate determination. For example, Wnt signaling is involved in the initial formation of the neural plate and in subsequent patterning decisions in the embryonic nervous system, including formation of the neural crest and contributes to the development of tissues and organs such as limbs, brain, reproductive tract and kidney during embryognenesis (Peifer and Polakis 2000 Science 287(5458): 1606). In addition, Wnt signaling is involved in hair follicle morphogenesis and is required for the initiation of hair follicle placode formation (Andl et al 2002 Dev Cell. (5):643-53.

There are at least 19 members of Wnt family including: Wnt-1 (RefSeq.: NM_005430), Wnt-2 (RefSeq.:NM_003391), Wnt-2B (Wnt-13) (RefSeq.: NM_004185), Wnt-3 (ReSeq. :NM_030753), Wnt-3A (RefSeq.: NM_033131), Wnt-4 (RefSeq.: NMR_030761), Wnt-5A (RefSeq.: NM_003392), Wnt-5B (RefSeq.: NM_032642), Wnt-6 (RefSeq.: NM_006522), Wnt-7A (RefSeq.: NM_004625), Wnt-7B (RefSeq.: NM_058238), Wnt-8A (RefSeq.: NM_058244), Wnt-8B (RefSeq.: NM_003393), Wnt-9A (Wnt-14) (RefSeq.: NM_003395), Wnt-9B (Wnt-15) (RefSeq.: NM_003396), Wnt-10A (RefSeq.: NM_025216), Wnt-10B (RefSeq.: NM_003394), Wnt-11 (RefSeq.: NM_004626), and Wnt-16 (RefSeq.: NM_016087). Each member is palmitoylated in its active form and has varying degrees of sequence identity but all contain 23-24 conserved cysteine residues which show highly conserved spacing. McMahon et al 1992 Trends Genet. 8:236-242; Miller 2002 Genome Biol. 3(1):3001.1-3001.15. Wnt proteins are ligands of seven-pass transmembrane receptors known as Frizzled (Fz). Ingham 1996 Trends Genet. 12:382-384; YangSnyder et al 1996 Curr. Biol. 6:1302-1306; Bhanot et al 1996 Nature 382:225-230. There are ten known members of the Fz family, Fz-1 through Fz-10, each characterized by the presence of a cysteine rich domain (CRD). Huang et al 2004 Genome Biol. 5:234.1-234.8. There is a great degree of promiscuity between the various Wnt-Frizzled interactions.

There are two Wnt signaling pathways. Activation of the non-canonical Wnt signaling pathway stimulates intracellular Ca²⁺ release and activates the kinases CamKII and PKC (Kuhl et al 2000 Trends Genet 16(7):279-83). The canonical Wnt signaling pathway is initiated by binding of Wnt ligands to Fz receptors and LRP co-receptors (LDL receptor related proteins e.g. LRP5 or LRP6). The subsequent recruitment of the cytoplasmic protein Dishevelled (Dsh) to Fz and the scaffold protein Axin to phosphorylated LRP result in stabilization and nuclear translocation of ß-catenin (Peifer et al 1994 Development 120:369-380; Papkoff et al 1996 Mol. Cell Biol. 16:2128-2134; Tamai et al 2004 Mol. Cell 13:149-156) which has been implicated in the development of cancers (Ilyas 2005 J. Pathol. 205:130-144). In the absence of Wnt signals, tumor suppressor gene adenomatous polyposis coli (APC) simultaneously interacts with the serine kinase glycogen synthase kinase (GSK)-3β, Axin and β-catenin (Su et al 1993 Science 262:1734-1737, Yost et al 1996 Genes Dev. 10:1443-1454; Hayashi et al 1997 Proc. Natl. Acad. Sci. USA, 94: 242-247; Sakanaka et al 1998 Proc. Natl. Acad. Sci. USA 95:3020-3023; Sakanaka and William 1999 J. Biol. Chem 274:14090-14093). Phosphorylation of APC and β-catenin by GSK-3β regulates the interaction of APC with β-catenin and targets phosphorylated β-catenin to proteasome for degradation (B. Rubinfeld et al 1996 Science 272:1023; Logan and Nusse 2004 Annu. Rev. Cell Dev. Biol. 20: 781-810).

Wnt signaling stabilization of β-catenin allows its translocation to the nucleus where it interacts with members of the lymphoid enhancer factor (LEF1)/T-cell factor (TCF4) family of transcription factors resulting in upregulation of target genes involved in carcinogenesis (Behrens et al 1996 Nature 382:638-642; Hsu et al 1998 Mol. Cell. Biol. 18:4807-4818; Roose et al 1999 Science 285:1923-1926), such as c-myc, cyclin D1 and metalloproteinase (He et al 1998 Science 281:1509-1512; Kolligs et al 1999 Mol. Cell. Biol. 19:5696-5706; Crawford et al 1999 Oncogene 18:2883-2891; Shtutman et al 1999 Proc. Natl. Acad. Sci. USA 11:5522-5527; Tetsu and McCormick 1999 Nature 398:422-426). The aberrant activation of the Wnt is implicated in driving the formation of various human cancers. There have been numerous reports indicating that Wnt ligands, Fz family members and Dsh may be overexpressed and that SFRP (secreted Frizzled related protein), DKK may be underexpressed in a number of major cancer types, including breast, ovarian, non-small cell lung and colon cancers (Ramaswamy et al., 2001 Proc. Natal. Acad. Sci. USA 98:15149; Bafico et al., 2004 Cancer Cell 6: 497-506; Liang et al., 2004 Oncogene 23: 6170-6174; Suzuki et al., 2004 Nature Genetics 36: 417-422; Uematsu et al., 2003 Oncogene 22: 7218-7221). Inhibition of aberrant Wnt signaling in certain cancer cell lines efficiently blocks their growth, highlighting its great potential as anti-cancer therapeutics (Barker and Clevers 2006 Nat. Rev. Drug Discovery 5: 997-1014; He et al., 2005 Oncogene 24: 3054-3058; Kawano et al., 2006 Oncogene 25: 6528-6537; Chen et al, 2001 J. Cell. Biol. 152: 87-96).

Recently studies have emerged that implicate abnormally active Wnt signaling in diseases and conditions other than cancer, including osteoarthritis, high bone mass, polycystic kidney disease, fibromatosis, pulmonary fibrosis, cardiovascular disease, autism and schizophrenia (Loughlin et al., 2004 Proc. Natl. Acad. Sci. USA 101:9757-9762; Boyden et al., 2002 N. Engl. J. Med. 346:1513-1521; Rodova et al., 2002 J. Biol. Chem. 277: 29577-29583; Cheon et al, 2002 Proc. Natl. Acad. Sci. USA 99: 6973-6978; Morrisey 2003 Am. J. Pathol. 162:1393-1397; van Gijn et al., 2002 Cardiovasc. Res. 55: 16-24; Ferrari and Moon 2006 Oncogene 25: 7545-7553; Miyaoka et al., 1999 Schizophr. Res. 38: 1-6). Inhibition of Wnt signaling is therefore an attractive approach for developing therapeutics in these fields.

WO2005/033048 describes heterocyclic compounds which are useful for the inhibition of an altered growth state of a cell (normal cells or cancer cells) having a Wnt receptor.

WO 2007/019933 A1 (Merck Patent GMBH; 22 February 2007) describes certain 1-acyl-dihydro-pyrazole derivatives of the following general formula which allegedly are inhibitors of tyrosine kinases (in particular, Met kinase) and are useful for the treatment of tumours.

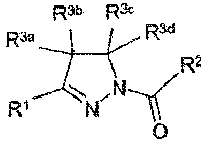

WO 2007/038425 A2 (Regents of the University of Minnesota; 05 April 2007) describes certain pyrazole derivatives of the following general formula which allegedly which suppress viral RNA synthesis and are useful for the treatment of viral infections.

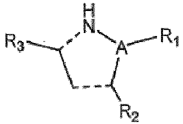

WO 2005/079803 A1 (Pfizer Products, Inc.; 01 September 2005) describes certain sodium-hydrogen exchanger type 1 (NHE-1) inhibitors of the following formula which allegedly are useful, in combination with a Ca²⁺ overload inhibitor, for the treatment of cardiovascular disease.

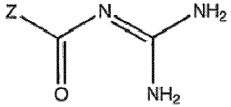

### SUMMARY OF THE INVENTION

A first aspect of the invention is a compound of formula (I): 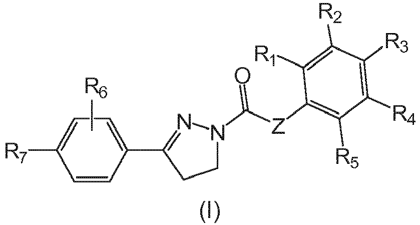 wherein:
R₁, R₄, and R₅ are H; or
R₂ and R₅ are H;
the others of R₁, R₂, R₃, R₄, and R₅ are independently halogen or alkoxy;
Z is -CH₂-;
R₆ is H, alkyl, or halogen;
R₇ is OH, -NH-Y, -NR₈-C(O)-Y, -NR₈-C(O)-NR₈-Y, or-NR₈-S(O)₂-Y;
R₈ is H or alkyl; and
Y is H, cyano, alkyl, a carbocycle or a heterocyle; wherein said alkyl is optionally substituted with halogen, hydroxyl, alkoxy, amino, nitro, cyano, carboxyl, sulfonyl, a carbocycle or a heterocycle; wherein said carbocycles and heterocycles are optionally substituted with hydroxyl, halogen, alkyl or haloalkyl.

In one embodiment, R₁, R₄, and R₅ are H.

In one embodiment, R₂ and R₅ are H.

In one embodiment,
R₁ is halogen; and
R₃ and R₄ are alkoxy.

In one embodiment, the others of R₁, R₂, R₃, R₄, and R₅ are independently chloro, fluoro, bromo, methoxy, or ethoxy.

In one embodiment, R₆ is H, Me, or F.

In one embodiment, R₆ is H.

In one embodiment, R₇ is -NR₈-C(O)-Y.

In one embodiment, R₇ is -NR₈-C(O)-NR₈-Y.

In one embodiment, R₇ is -NR₈-S(O)₂-Y.

In one embodiment, R₈ is H or Me.

In one embodiment, R₇ is -NH-Y.

In one embodiment, Y is H, alkyl, cycloalkyl, heteroaryl or alkoxyakyl.

In one embodiment, Y is H.

In one embodiment, Y is alkyl.

In one embodiment, Y is methyl.

In one embodiment, Y is cycloalkyl.

In one embodiment, Y is heteroaryl

In one embodiment, R₇ is OH.

A second aspect of the invention is a pharmaceutical composition comprising a compound of the first aspect, and a pharmaceutically acceptable carrier, diluent or excipient.

A third aspect of the invention is a compound of the first aspect, for use in a method of treatment of the human or animal body by therapy.

In one embodiment, the method is a method of treatment of a disease or condition associated with Wnt pathway signaling in a mammal, cancer, colorectal cancer, or breast cancer.

A fourth aspect of the invention is use of a compound of the first aspect in the manufacture of a medicament for the treatment of a disease or condition associated with Wnt pathway signaling in a mammal, cancer, colorectal cancer, or breast cancer.

### DETAILED DESCRIPTION OF THE PREFERRED EMBODIMENTS

"Acyl" means a carboxyl containing substituent represented by the formula -C(O)-R in which R is H, alkyl, a carbocycle, a heterocycle, carbocycle-substituted alkyl or heterocycle-substituted alkyl wherein the alkyl, alkoxy, carbocycle and heterocycle are as defined herein. Acyl groups include alkanoyl (e.g. acetyl), aroyl (e.g. benzoyl), and heteroaroyl.

"Alkyl," means a branched or unbranched, saturated or unsaturated (i.e. alkenyl, alkynyl) aliphatic hydrocarbon group, having up to 12 carbon atoms unless otherwise specified. When used as part of another term, for example "alkylamino", the alkyl portion is preferably a saturated hydrocarbon chain, however also includes unsaturated hydrocarbon carbon chains such as "alkenylamino" and "alkynylamino. "Alkylphosphinate" means a -P(O)R-alkyl group wherein R is H, alkyl, carbocycle-alkyl or heterocycle-alkyl. Examples of preferred alkyl groups include methyl, ethyl, n-propyl, isopropyl, n-butyl. iso-butyl, sec-butyl, tert-butyl, n-pentyl, 2-methylbutyl, 2,2-dimethylpropyl, n-hexyl, 2-methylpentyl, 2,2-dimethylbutyl, n-heptyl, 3-heptyl, 2-methylhexyl, and the like. The terms "lower alkyl," "C₁-C₄ alkyl." and "alkyl of 1 to 4 carbon atoms" are synonymous and used interchangeably to mean methyl, ethyl, 1-propyl, isopropyl, cyclopropyl, 1-butyl, sec-butyl or t-butyl. Unless specified, substituted, alkyl groups may contain one (preferably), two, three or four substituents which may be the same or different. Examples of the above substituted alkyl groups include, but are not limited to; cyanomethyl, nitromethyl, hydroxymethyl, trityloxymethyl, propionyloxymethyl, aminomethyl, carboxymethyl, carboxyethyl, carboxypropyl, alkyloxycarbonylmethyl, allyloxycarbonylaminomethyl, carbamoyloxymethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichloro(n-butyl), 2-amino(iso-propyl), 2-carbamoyloxyethyl and the like. The alkyl group may also be substituted with a carbocycle group. Examples include cyclopropylmethyl, cyclobutylmethyl, cyclopentylmethyl, and cyclohexylmethyl groups, as well as the corresponding -ethyl, -propyl, -butyl, -pentyl, -hexyl groups, etc. Preferred substituted alkyls are substituted methyls e.g. a methyl group substituted by the same substituents as the "substituted Cₙ-Cₘ alkyl," group. Examples of the substituted methyl group include groups such as hydroxymethyl, protected hydroxymethyl (e.g. tetrahydropyranyloxymethyl), acetoxymethyl, carbamoyloxymethyl, trifluoromethyl, chloromethyl, carboxymethyl, bromomethyl and iodomethyl.

"Amidine" or "amidino" means the group -C(NH)-NRR wherein each R is independently H, OH, alkyl, alkoxy, a carbocycle, a heterocycle, a carbocycle-substituted alkyl or a heterocycle-substituted alkyl; or both R groups together form a heterocycle. A preferred amidine is the group -C(NH)-NH₂.

"Amino" means primary (i.e. -NH₂), secondary (i.e. -NRH) and tertiary (i.e. -NRR) amines wherein R is independently alkyl, a carbocycle (e.g. aryl), a heterocycle (e.g. heteroaryl), carbocycle-substituted alkyl (e.g. benzyl) or a heterocycle-substituted alkyl or alternatively two R groups together with the nitrogen atom from which they depend form a heterocycle. Particular secondary and tertiary amines are alkylamine, dialkylamine, arylamine, diarylamine, aralkylamine and diaralkylamine. Particular secondary and tertiary amines are methylamine, ethylamine, propylamine, isopropylamine, phenylamine, benzylamine dimethylamine, diethylamine, dipropylamine and diisopropylamine.

"Amino-protecting group" as used herein refers to a derivative of the groups commonly employed to block or protect an amino group while reactions are carried out on other functional groups on the compound. Examples of such protecting groups include carbamates, amides, alkyl and aryl groups, imines, as well as many N-heteroatom derivatives which can be removed to regenerate the desired amine group. Preferred amino protecting groups are Boc, Fmoc and Cbz. Further examples of these groups are found in T. W. Greene and P. G. M. Wuts, "Protective Groups in Organic Synthesis", 2nd ed., John Wiley & Sons, Inc., New York, NY, 1991, chapter 7; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected amino" refers to an amino group substituted with one of the above amino-protecting groups.

"Aryl" when used alone or as part of another term means a carbocyclic aromatic group whether or not fused having the number of carbon atoms designated or if no number is designated, up to 14 carbon atoms. Aryl groups include phenyl, naphthyl, biphenyl, phenanthrenyl, naphthacenyl, and the like (see e.g. Lang's Handbook of Chemistry (Dean, J. A., ed) 13th ed. Table 7-2 [1985]). In a particular embodiment aryl may be phenyl. Substituted phenyl or substituted aryl denotes a phenyl group or aryl group substituted with one, two, three, four or five, such as 1-2, 1-3 or 1-4 substituents chosen, unless otherwise specified, from halogen (F, Cl, Br, I), hydroxy, protected hydroxy, cyano, nitro, alkyl (for example C₁-C₆ alkyl), alkoxy (for example C₁-C₆ alkoxy), benzyloxy, carboxy, protected carboxy, carboxymethyl, protected carboxymethyl, hydroxymethyl, protected hydroxymethyl, aminomethyl, protected aminomethyl, trifluoromethyl, alkylsulfonylamino, arylsulfonylamino, heterocyclylsulfonylamino, heterocyclyl, aryl, or other groups specified. One or more methyne (CH) and/or methylene (CH₂) groups in these substituents may in turn be substituted with a similar group as those denoted above. Examples of the term "substituted phenyl" includes but is not limited to a mono- or di(halo)phenyl group such as 2-chlorophenyl, 2-bromophenyl, 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono or di(alkoxy)phenyl group, for example, 3,4-dimethoxyphenyl, 3-methoxy-4-benzyloxyphenyl, 3-methoxy-4-(1-chloromethyl)benzyloxy-phenyl, 3-ethoxyphenyl, 4-(isopropoxy)phenyl, 4-(t-butoxy)phenyl, 3-ethoxy-4-methoxyphenyl and the like; 3- or 4-trifluoromethylphenyl; a mono- or dicarboxyphenyl or (protected carboxy)phenyl group such 4-carboxyphenyl, ; a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))phenyl. Also, the term "substituted phenyl" represents disubstituted phenyl groups where the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl, and the like, as well as trisubstituted phenyl groups where the substituents are different, for example 3-methoxy-4-benzyloxy-6-methyl sulfonylamino, 3-methoxy-4-benzyloxy-6-phenyl sulfonylamino, and tetrasubstituted phenyl groups where the substituents are different such as 3-methoxy-4-benzyloxy-5-methyl-6-phenyl sulfonylamino. Substituted phenyl groups include 2-chlorophenyl, 2-aminophenyl, 2-bromophenyl, 3-methoxyphenyl, 3-ethoxy-phenyl, 4-benzyloxyphenyl, 4-methoxyphenyl, 3-ethoxy-4-benzyloxyphenyl, 3,4-diethoxyphenyl, 3-methoxy-4-benzyloxyphenyl, 3-methoxy-4-(1-chloromethyl)benzyloxy-phenyl, 3-methoxy-4-(1-chloromethyl)benzyloxy -6- methyl sulfonyl aminophenyl groups. Fused aryl rings may also be substituted with any (for example 1, 2 or 3) of the substituents specified herein in the same manner as substituted alkyl groups.

"Carbamoyl" means an aminocarbonyl containing substituent represented by the formula-C(O)N(R)₂ in which R is H, hydroxyl, alkoxy, alkyl, a carbocycle, a heterocycle, carbocycle-substituted alkyl or alkoxy, or heterocycle-substituted alkyl or alkoxy wherein the alkyl, alkoxy, carbocycle and heterocycle are as herein defined. Carbamoyl groups include alkylaminocarbonyl (e.g. ethylaminocarbonyl, Et-NH-CO-), arylaminocarbonyl (e.g. phenylaminocarbonyl), aralkylaminocarbonyl (e.g. benzoylaminocarbonyl) a heterocycleaminocarbonyl (e.g. piperizinylaminocarbonyl), and in particular a heteroarylaminocarbonyl (e.g. pyridylaminocarbonyl).

"Carbocyclyl", "carbocyclic", "carbocycle" and "carbocyclo" alone and when used as a moiety in a complex group such as a carbocycloalkyl group, refers to a mono-, bi-, or tricyclic aliphatic ring having 3 to 14 carbon atoms and preferably 3 to 7 carbon atoms which may be saturated or unsaturated, aromatic or non-aromatic. Preferred saturated carbocyclic groups include cyclopropyl, cyclobutyl, cyclopentyl and cyclohexyl groups and more preferred are cyclopropyl and cyclohexyl and most preferred is cyclohexyl. Preferred unsaturated carbocycles are aromatic e.g. aryl groups as previously defined, the most preferred being phenyl. The terms "substituted carbocyclyl", "substituted carbocycle" and "substituted carbocyclo" unless otherwise specified mean these groups substituted by the same substituents as the "substituted alkyl" group.

"Carboxy-protecting group" as used herein refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, alkyl such as t-butyl or t-amyl, trityl, 4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, beta-(trimethylsilyl)ethyl, beta-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the molecule and can be removed at the appropriate point without disrupting the remainder of the molecule. In particular, it is important not to subject a carboxy-protected molecule to strong nucleophilic bases, such as lithium hydroxide or NaOH, or reductive conditions employing highly activated metal hydrides such as LiAlH₄. (Such harsh removal conditions are also to be avoided when removing amino-protecting groups and hydroxy-protecting groups, discussed below.) Preferred carboxylic acid protecting groups are the alkyl (e.g. methyl, ethyl, t-butyl), allyl, benzyl and p-nitrobenzyl groups. Similar carboxy-protecting groups used in the cephalosporin, penicillin and peptide arts can also be used to protect a carboxy group substituents. Further examples of these groups are found in T. W. Greene and P. G. M. Wuts, "Protective Groups in Organic Synthesis", 2nd ed., John Wiley & Sons, Inc., New York, N.Y., 1991, chapter 5; E. Haslam, "Protective Groups in Organic Chemistry", J. G. W. McOmie, Ed., Plenum Press, New York, N.Y., 1973, Chapter 5, and T.W. Greene, "Protective Groups in Organic Synthesis", John Wiley and Sons, New York, NY, 1981, Chapter 5. The term "protected carboxy" refers to a carboxy group substituted with one of the above carboxy-protecting groups.

"Guanidine" means the group -NH-C(NH)-NHR wherein R is H, alkyl, a carbocycle, a heterocycle, a carbocycle-substituted alkyl, or a heterocycle-substituted alkyl. A particular guanidine group is -NH-C(NH)-NH₂.

"Heterocyclic group", "heterocyclic", "heterocycle", "heterocyclyl", or "heterocyclo" alone and when used as a moiety in a complex group such as a heterocycloalkyl group, are used interchangeably and refer to any mono-, bi-, or tricyclic, saturated or unsaturated, aromatic (heteroaryl) or non-aromatic ring having the number of atoms designated, generally from 5 to about 14 ring atoms, where the ring atoms are carbon and at least one heteroatom (nitrogen, sulfur or oxygen) and preferably 1 to 4 heteroatoms. "Heterocyclosulfonyl" means a -SO₂-heterocycle group; "heterocyclosulfinyl" means a -SO-heterocycle group. Typically, a 5-membered ring has 0 to 2 double bonds and 6- or 7-membered ring has 0 to 3 double bonds and the nitrogen or sulfur heteroatoms may optionally be oxidized (e.g. SO, SO₂), and any nitrogen heteroatom may optionally be quaternized. Preferred non-aromatic heterocycles include morpholinyl (morpholino), pyrrolidinyl, oxiranyl, oxetanyl, tetrahydrofuranyl, 2,3-dihydrofuranyl, 2H-pyranyl, tetrahydropyranyl, thiiranyl, thietanyl, tetrahydrothietanyl, aziridinyl, azetidinyl, 1-methyl-2-pyrrolyl, piperazinyl and piperidinyl. A "heterocycloalkyl" group is a heterocycle group as defined above covalently bonded to an alkyl group as defined above. Preferred 5-membered heterocycles containing a sulfur or oxygen atom and one to three nitrogen atoms include thiazolyl, in particular thiazol-2-yl and thiazol-2-yl N-oxide, thiadiazolyl, in particular 1,3,4-thiadiazol-5-yl and 1,2,4-thiadiazol-5-yl, oxazolyl, preferably oxazol-2-yl, and oxadiazolyl, such as 1,3,4-oxadiazol-5-yl, and 1,2,4-oxadiazol-5-yl. Preferred 5-membered ring heterocycles containing 2 to 4 nitrogen atoms include imidazolyl, preferably imidazol-2-yl; triazolyl, preferably 1,3,4-triazol-5-yl; 1,2,3-triazol-5-yl, 1,2,4-triazol-5-yl, and tetrazolyl, preferably 1H-tetrazol-5-yl. Preferred benzo-fused 5-membered heterocycles are benzoxazol-2-yl, benzthiazol-2-yl and benzimidazol-2-yl. Preferred 6-membered heterocycles contain one to three nitrogen atoms and optionally a sulfur or oxygen atom, for example pyridyl, such as pyrid-2-yl, pyrid-3-yl, and pyrid-4-yl; pyrimidyl, preferably pyrimid-2-yl and pyrimid-4-yl; triazinyl, preferably 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl groups, are a preferred group. Substituents for optionally substituted heterocycles, and further examples of the 5- and 6-membered ring systems discussed above can be found in W. Druckheimer et al., U.S. Patent No. 4,278,793.

"Heteroaryl" alone and when used as a moiety in a complex group such as a heteroaralkyl group, refers to any mono-, bi-, or tricyclic aromatic ring system having the number of atoms designated where at least one ring is a 5-, 6- or 7-membered ring containing from one to four heteroatoms selected from the group nitrogen, oxygen, and sulfur, and preferably at least one heteroatom is nitrogen *(Lang's Handbook of Chemistry, supra).* Included in the definition are any bicyclic groups where any of the above heteroaryl rings are fused to a benzene ring. Heteroaryls in which nitrogen or oxygen is the heteroatom are preferred. The following ring systems are examples of the heteroaryl (whether substituted or unsubstituted) groups denoted by the term "heteroaryl": thienyl, furyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, thiazinyl, oxazinyl, triazinyl, thiadiazinyl, oxadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl, tetrazolo[1,5-b]pyridazinyl and purinyl, as well as benzo-fused derivatives, for example benzoxazolyl, benzofuryl, benzothiazolyl, benzothiadiazolyl, benzotriazolyl, benzoimidazolyl and indolyl. A particularly preferred group of "heteroaryl" include; 1,3-thiazol-2-yl, 4-(carboxymethyl)-5-methyl-1,3-thiazol-2-yl, 4-(carboxymethyl)-5-methyl-1,3-thiazol-2-yl sodium salt, 1,2,4-thiadiazol-5-yl, 3-methyl-1,2,4-thiadiazol-5-yl, 1,3,4-triazol-5-yl, 2-methyl-1,3,4-triazol-5-yl, 2-hydroxy-1,3,4-triazol-5-yl, 2-carboxy-4-methyl-1,3,4-triazol-5-yl sodium salt, 2-carboxy-4-methyl-1,3,4-triazol-5-yl, 1,3-oxazol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-thiol-1,3,4-thiadiazol-5-yl, 2-(methylthio)-1,3,4-thiadiazol-5-yl, 2-amino-1,3,4-thiadiazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 2-methyl-1H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(n-oxide)-pyridaz-3-yl, 6-hydroxypyridaz-3-yl, 1-methylpyrid-2-yl, 1-methylpyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-astriazin-3-yl, 2,5-dihydro-5-oxo-6-hydroxy-as-triazin-3-yl sodium salt, 2,5-dihydro-5-oxo-6-hydroxy-2-methyl-astriazin-3-yl sodium salt, 2,5-dihydro-5-oxo-6-hydroxy-2-methyl-as-triazin-3-yl, 2,5-dihydro-5-oxo-6-methoxy-2-methyl-as-triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5-b]pyridazin-6-yl and 8-aminotetrazolo[1,5-b]-pyridazin-6-yl. An alternative group of "heteroaryl" includes; 4-(carboxymethyl)-5-methyl-1,3-thiazol-2-yl, 4-(carboxymethyl)-5-methyl-1,3-thiazol-2-yl sodium salt, 1,3,4-triazol-5-yl, 2-methyl-1,3,4-triazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 1,2,3-triazol-5-yl, 1,4,5,6-tetrahydro-5,6-dioxo-4-methyl-as-triazin-3-yl, 1,4,5,6-tetrahydro-4-(2-formylmethyl)-5,6-dioxo-as-triazin-3-yl, 2,5-dihydro-5-oxo-6-hydroxy-2-methyl-as-triazin-3-yl sodium salt, 2,5-dihydro-5-oxo-6-hydroxy-2-methyl-as-triazin-3-yl, tetrazolo[1,5-b]pyridazin-6-yl, and 8-aminotetrazolo[1,5-b]pyridazin-6-yl.

"Hydroxy-protecting group" as used herein refers to a derivative of the hydroxy group commonly employed to block or protect the hydroxy group while reactions are carried out on other functional groups on the compound. Examples of such protecting groups include tetrahydropyranyloxy, benzoyl, acetoxy, carbamoyloxy, benzyl, and silylethers (e.g. TBS, TBDPS) groups. Further examples of these groups are found in T. W. Greene and P. G. M. Wuts, "Protective Groups in Organic Synthesis", 2nd ed., John Wiley & Sons, Inc., New York, NY, 1991, chapters 2-3; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected hydroxy" refers to a hydroxy group substituted with one of the above hydroxy-protecting groups.

"Optionally substituted" unless otherwise specified means that a group may be substituted by one or more (e.g. 0, 1, 2, 3 or 4) of the substituents listed for that group in which said substituents may be the same or different. In an embodiment an optionally substituted group has 1 substituent. In another embodiment an optionally substituted group has 2 substituents. In another embodiment an optionally substituted group has 3 substituents.

"Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, TEA, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic non-toxic bases are isopropylamine, diethylamine, ethanolamine, trimethamine, dicyclohexylamine, choline, and caffeine.

"Phosphinate" means -P(O)R-OR wherein each R is independently H, alkyl, carbocycle, heterocycle, carbocycloalkyl or heterocycloalkyl. Particular phosphinate groups are alkylphosphinate (i.e. -P(O)R-O-alkyl), for example -P(O)Me-OEt.

"Sulfamoyl" means -SO₂-N(R)₂ wherein each R is independently H, alkyl, carbocycle, heterocycle, carbocycloalkyl or heterocycloalkyl. Particular sulfamoyl groups are alkylsulfamoyl, for example methylsulfamoyl (-SO₂-NHMe); arylsulfamoyl, for example phenylsulfamoyl; aralkylsulfamoyl, for example benzylsulfamoyl.

"Sulfide" means -S-R wherein R is H, alkyl, carbocycle, heterocycle, carbocycloalkyl or heterocycloalkyl. Particular sulfide groups are mercapto, alkylsulfide, for example methylsulfide (-S-Me); arylsulfide, for example phenylsulfide; aralkylsulfide, for example benzylsulfide.

"Sulfinyl" means a -SO-R group wherein R is alkyl, carbocycle, heterocycle, carbocycloalkyl or heterocycloalkyl. Particular sulfinyl groups are alkylsulfinyl (i.e. -SO-alkyl), for example methylsulfinyl; arylsulfinyl (i.e. -SO-aryl) for example phenylsulfinyl; aralkylsulfinyl, for example benzylsulfinyl.

"sulfonamide" means -NR-SO₂-R wherein each R is independently H, alkyl, carbocycle, heterocycle, carbocycloalkyl or heterocycloalkyl), a carbocycle or a heterocycle. Particular sulfonamide groups are alkylsulfonamide (e.g. -NH-SO₂-alkyl), for example methylsulfonamide; arylsulfonamide (i.e. -NH-SO₂-aryl) for example phenylsulfonamide; aralkylsulfonamide, for example benzylsulfonamide.

"sulfonyl" means a -SO₂-R group wherein R is alkyl, carbocycle, heterocycle, carbocycloalkyl or heterocycloalkyl. Particular sulfonyl groups are alkylsulfonyl (i.e. -SO₂-alkyl), for example methylsulfonyl; arylsulfonyl, for example phenylsulfonyl; aralkylsulfonyl, for example benzylsulfonyl.

The phrase "and salts and solvates thereof" as used herein means that compounds of the invention may exist in one or a mixture of salts and solvate forms. For example a compound of the invention may be substantially pure in one particular salt or solvate form or else may be mixtures of two or more salt or solvate forms.

The present invention provides a compounds having the general formula I: 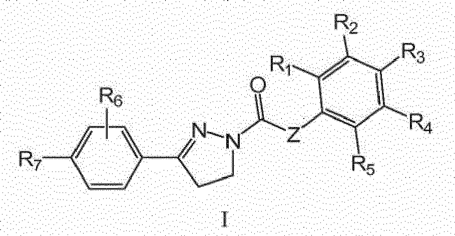 wherein R₁ to R₇ and Z are as defined herein.

Each of R₁ to R₅, when other than H, is independently halogen, or alkoxy. Each of R₁ to R₅ when other than H, is independently chloro, fluoro, bromo, methoxy, or ethoxy.

In a particular embodiment R₁, R₄ and R₅ are H and R₂ and R₃ are other than H as defined herein, In a particular embodiment R₂ and R₅ are H and R₁, R₃ and R₄ are other than H as defined herein. In a particular embodiment R₂ and R₅ are H; R₁ is halogen (e.g. chloro or promo); and R₃ and R₄ are Alkoxy (e.g. methoxy or ethoxy).

In a particular embodiment R₆ is H. In a particular embodiment R₆ is alkyl (e.g. methyl) or halogen (e.g. F). In a particular embodiment R₆ is ortho (adjacent) relative to R₇. In a particular embodiment R₆ is meta relative to R₇.

R₇ is OH or -X. In a particular embodiment R₇ is OH. In a particular embodiment R₇ is X.

In a particular embodiment X is -NH-Y. In a particular embodiment X is -NR₈-C(O)-Y wherein R₈ is H or alkyl (e.g. methyl). In a particular embodiment X is -NH-C(O)-Y. In a particular embodiment X is -NR₈-C(O)-NR₈-Y wherein R₈ is H or alkyl (e.g. methyl). In a particular embodiment X is -NHC(O)NH-Y. In a particular embodiment X is -NR₈-S(O)₂-Y wherein R₈ is H or alkyl (e.g. methyl). In a particular embodiment X is -NH-S(O)₂-Y.

Y is H, cyano, alkyl, a carbocycle or a heterocyle; wherein said alkyl is optionally substituted with halogen, hydroxyl, Alkoxy, amino, nitro, cyano, carboxyl, sulfonyl, a carbocycle or a heterocycle wherein said carbocycles and heterocycles are optionally substituted with hydroxyl, halogen, alkyl or haloalkyl. In a particular embodiment, Y is H, alkyl, cycloalkyl, heteroaryl or alkoxyalkyl. In a particular embodiment Y is H, cyano, methyl, ethyl, ethynyl, 2-propyl, propynyl, methoxyethenyl, cyclopropyl or 1,2,3-thiadiazole. In a particular embodiment, Y is alkyl (e.g. methyl, ethyl, ethynyl, propyl, 2-propyl or propynyl). In a particular embodiment Y is methyl. In a particular embodiment Y is H. In a particular embodiment Y is cyano. In a particular embodiment Y is heteroaryl (e.g. thiadiazole). In a particular embodiment, Y is cycloalkyl (e.g. cyclopropyl, cyclobutyl, cyclopentyl or cyclohexyl).

Z is -CH₂-.

Particular compounds of the invention include, but are not limited to the following:

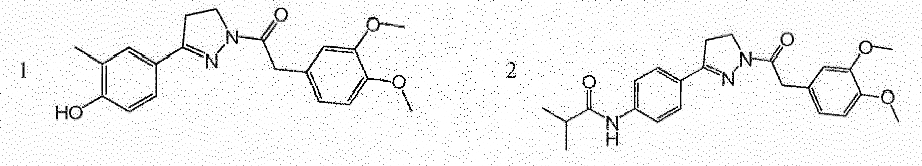

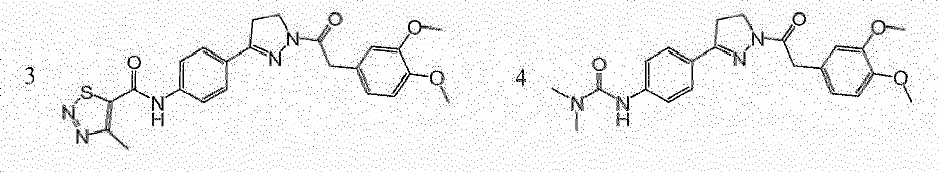

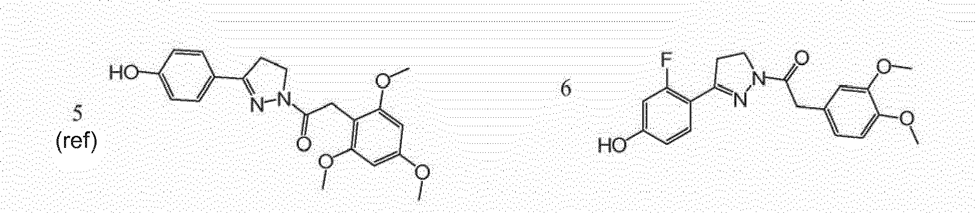

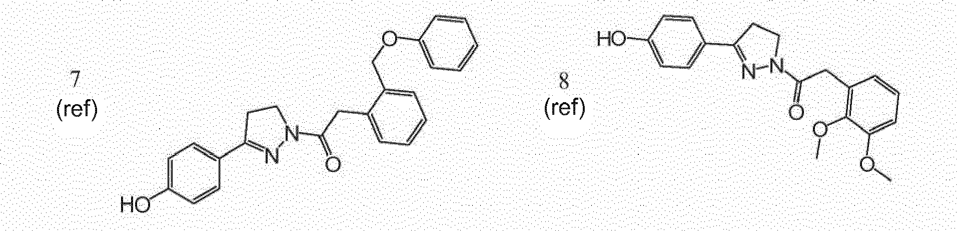

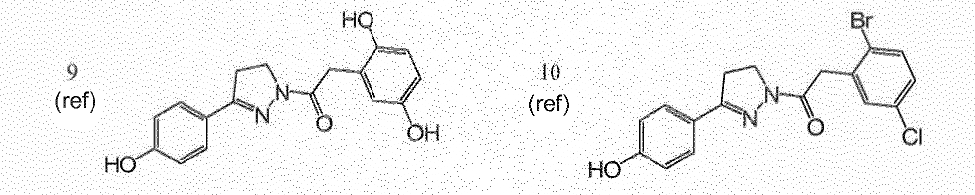

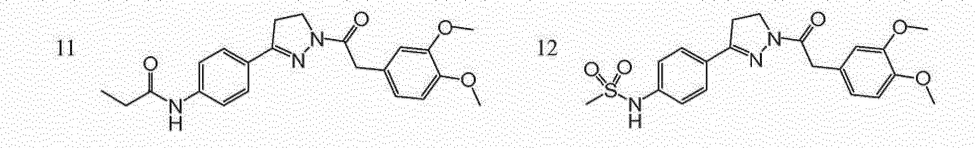

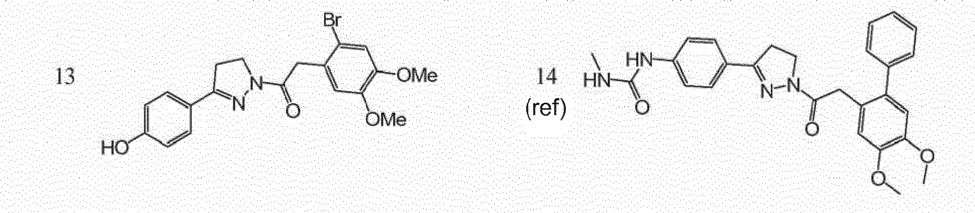

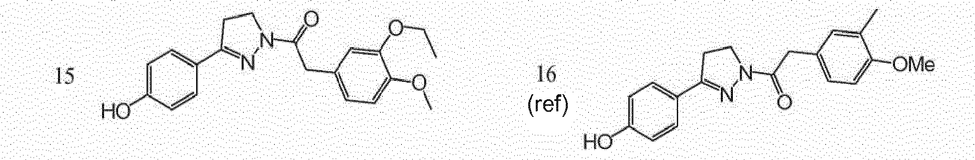

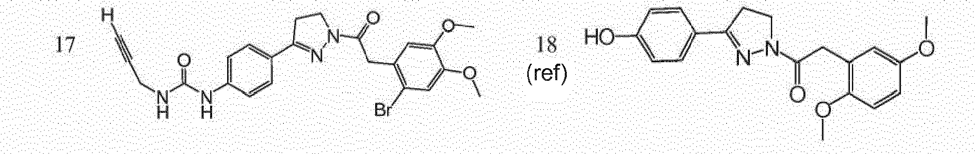

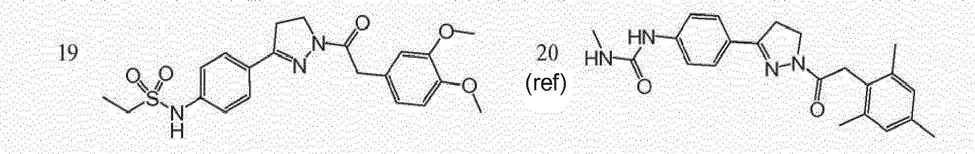

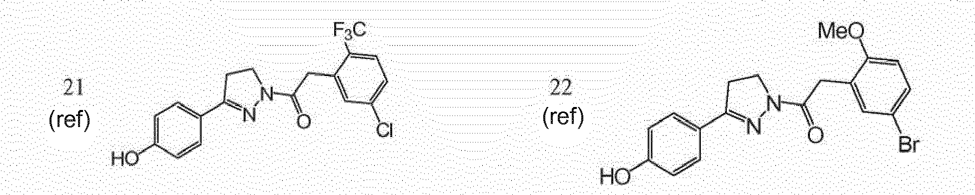

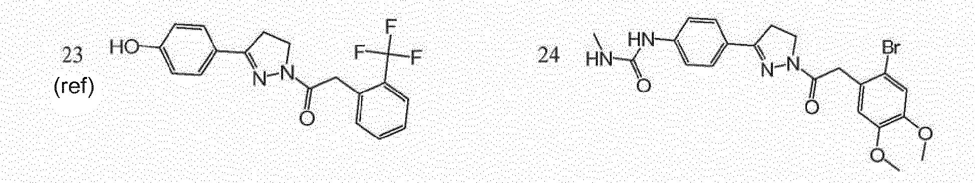

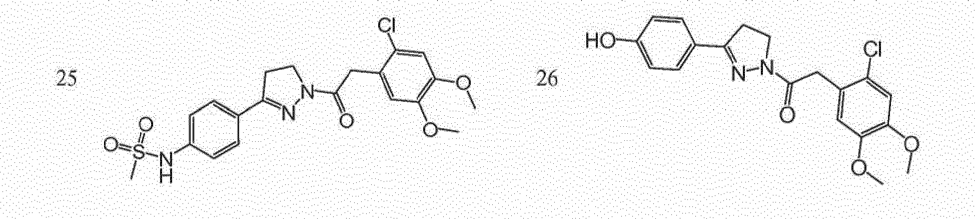

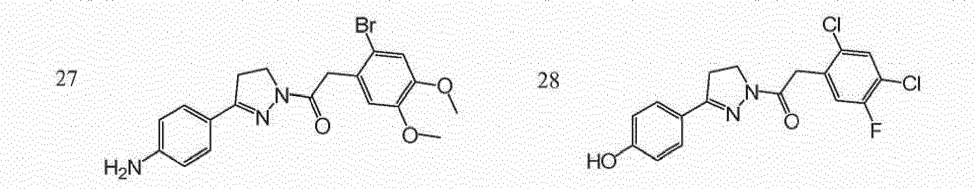

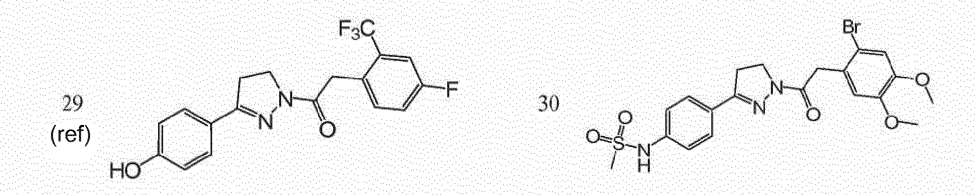

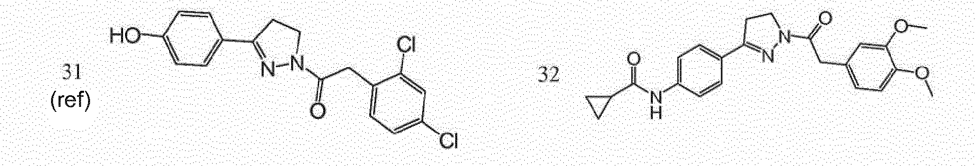

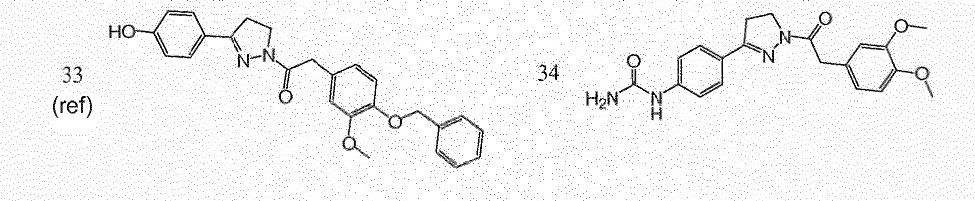

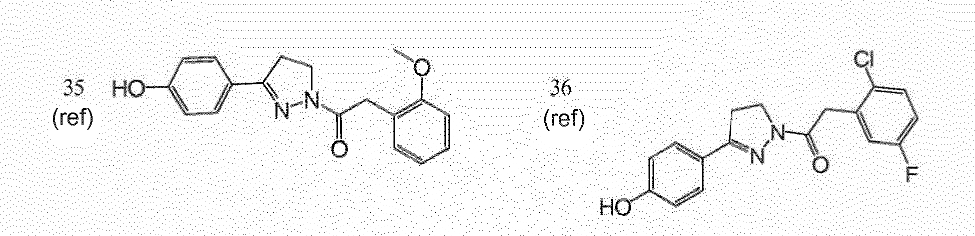

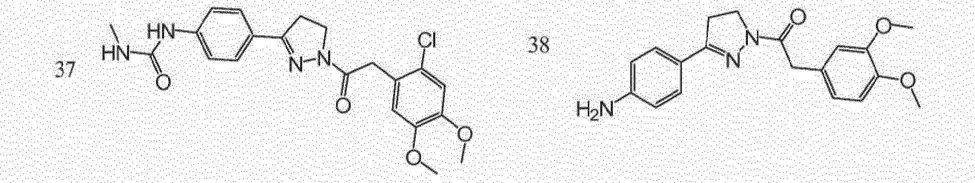

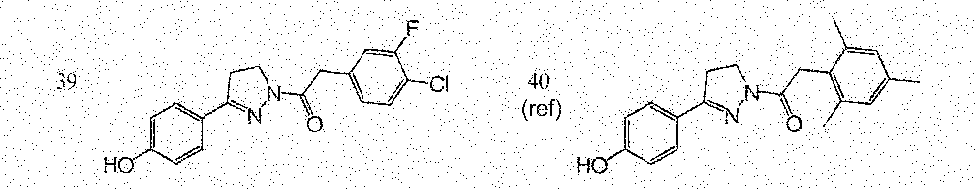

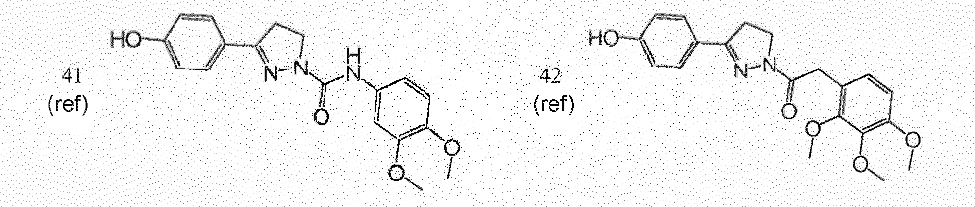

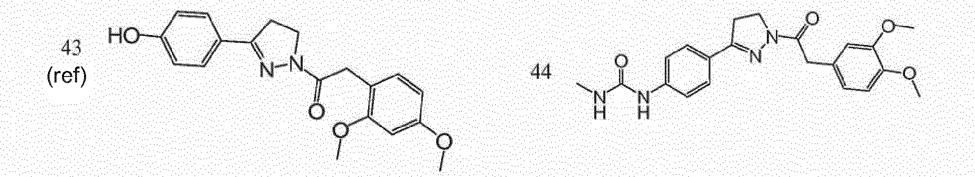

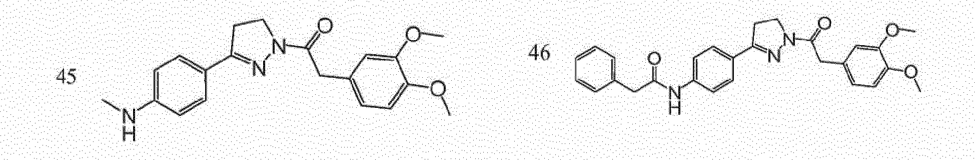

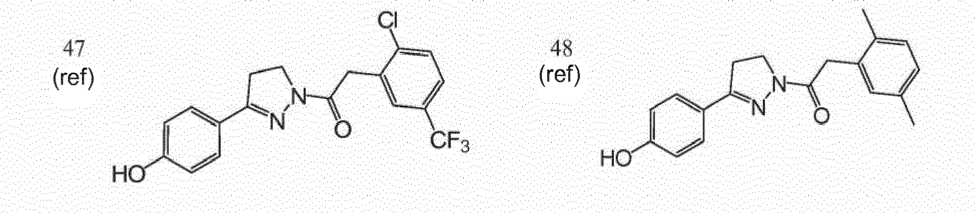

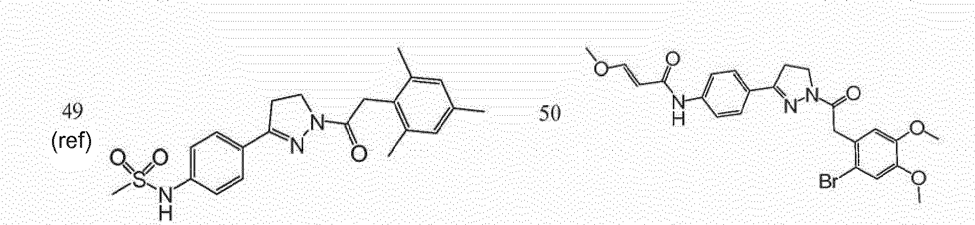

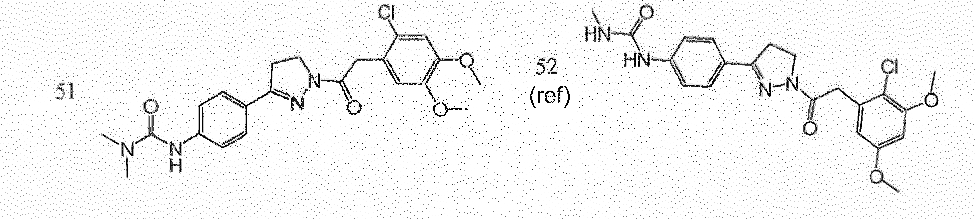

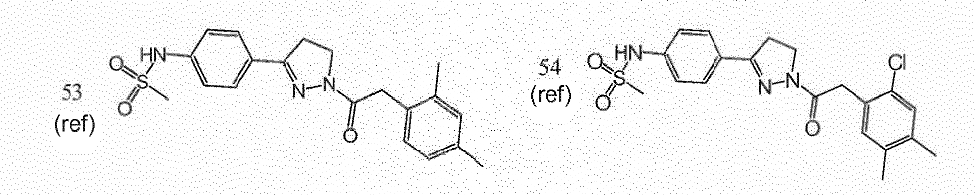

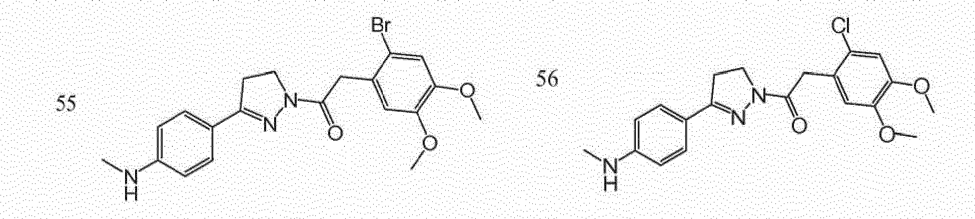

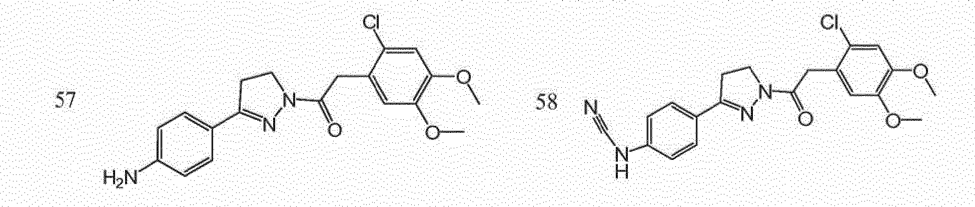

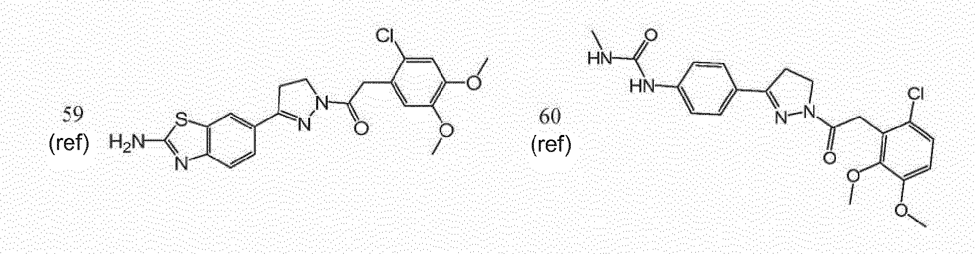

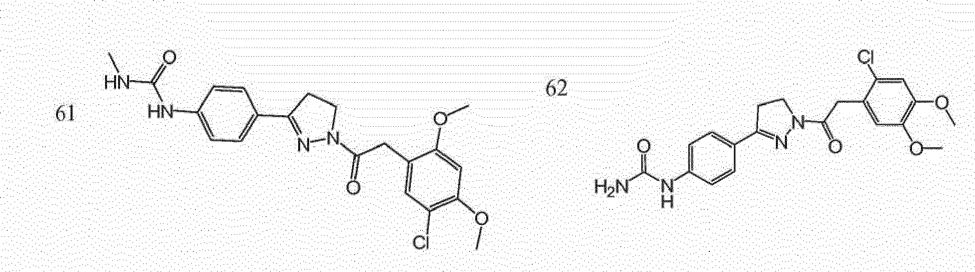

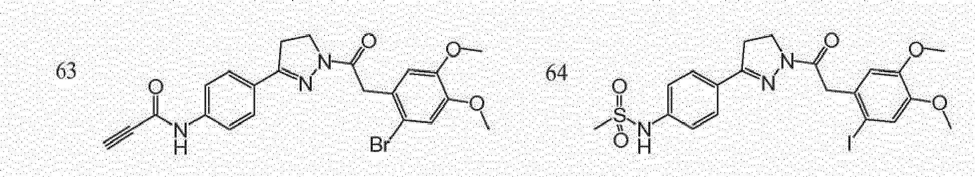

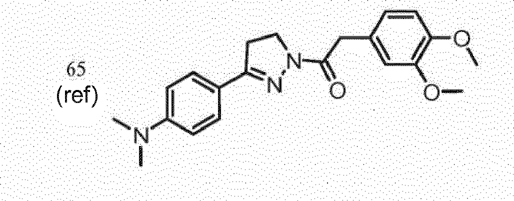

Compounds of the invention are prepared using standard organic synthetic techniques from commercially available starting materials and reagents. It will be appreciated that synthetic procedures employed in the preparation of compounds of the invention will depend on the particular substituents present in a compound and that various protection and deprotection procedures may be required as is standard in organic synthesis. Compounds may be prepared according to the following general scheme 1:

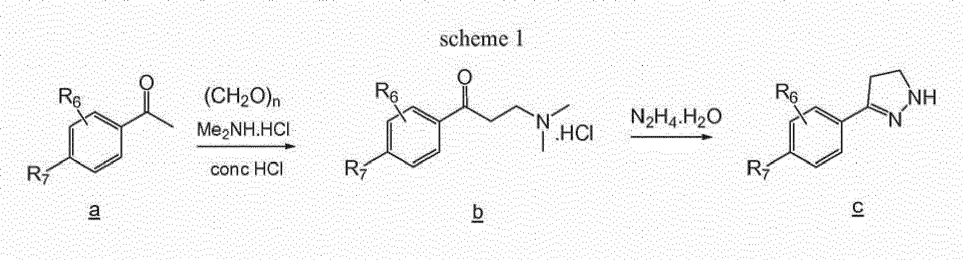

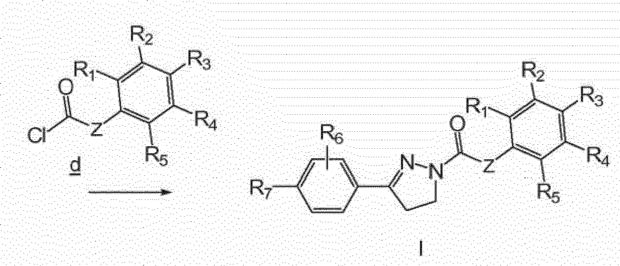

A mixture of acetophenone a, paraformaldehyde and dimethylamine is added to concentrated HCl to give dimethylamino-phenylpropanone b which is cyclized with hydrazine hydrate to give dihydropyrazole c. Acid halide d is reacted with dihydropyrazole c to give the final compound I.

Alternatively compounds of the formula I may be prepared according to scheme 2 in which dihydropyrazole b is reacted with phenyl acetic acid a in a dichloromethane solution with oxalyl chloride and catalyzed with DMF.

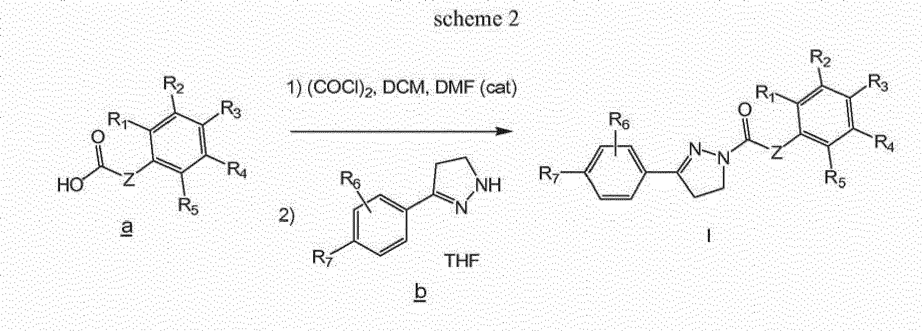

Compounds in which R₇ is -C(O)-Y, -C(O)-O-Y or -S(O)₂-Y may be prepared according to the procedures of scheme 3 by reacting amine a with the corresponding acid chloride, sulfonyl chloride or chloroformate.

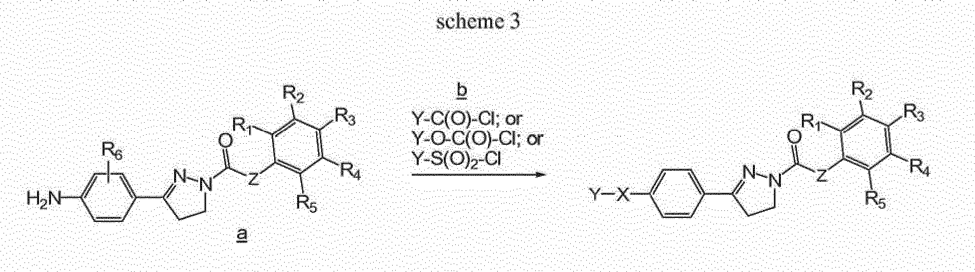

Compounds incorporating a urea moiety at R₇ (i.e. R₇ is -X-Y in which X is - NHC(O)NH-) may be prepared according to general procedure 4:

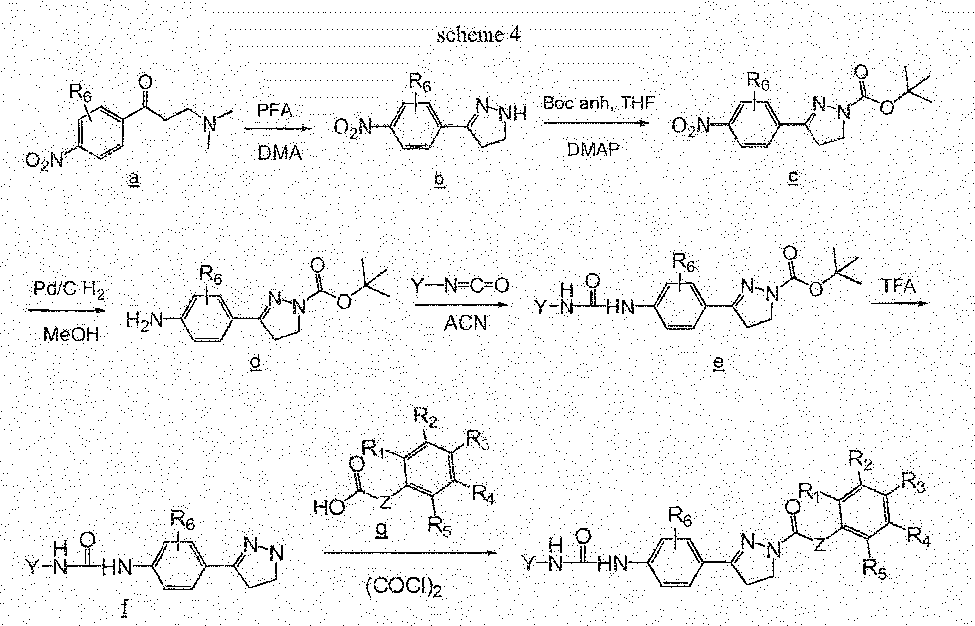

Starting amine a prepared from treating 4-nitroacetophenone with paraformaldehyde and dimethylamine is reacted with hydrazine hydrate to give dihydropyrazote b. The dihydropyrazole b is protected with bloc anhydride in THF, catalyzed by dimethylaminopyridine, and subsequently the nitro groups is reduced with palladium catalyst under hydrogen atmosphere to give amine d. Amine d is then reacted with appropriate isocyanate Y-N-C=O in acetonitrile to give urea e which is boc-deprotected with TFA. Deprotected urea f is reacted with carboxylic acid g treated with oxalylchloride to give final urea compound.

Compounds of the invention may contain one or more asymmetric carbon atoms. Accordingly, the compounds may exist as diastereomers, enantiomers or mixtures thereof. The syntheses of the compounds may employ racemates, diastereomers or enantiomers as starting materials or as intermediates. Diastereomeric compounds may be separated by chromatographic or crystallization methods. Similarly, enantiomeric mixtures may be separated using the same techniques or others know in the art. Each of the asymmetric carbon atoms may be in the R or S configuration and both of these configurations are within the scope of the invention.

Also described herein are prodrugs of the compounds described above. Suitable prodrugs include known amino-protecting and carboxy-protecting groups which are released, for example hydrolyzed, to wield the parent compound under physiologic conditions. A particular class of prodrugs are compounds in which a nitrogen atom in an amino, amidino, aminoalkyleneamino, iminoalkyleneamino or guanidino group is substituted with a hydroxy (OH) group, an alkylcarbanyl (-CO-R) group, an alkoxycarbonyl (-CO-OR), an acyloxyalkyl-alkoxycarbonyl (-CO-O-R-O-CO-R) group where R is a monovalent or divalent group and as defined abode or a group having the formula -C(O)-O-CP1P2-haloalkyl, where P1 and P2 are the same or different and are H, lower alkyl, lower alkoxy, cyano, halo lower alkyl or aryl. Prodrug compounds may be prepared by reacting the compounds of the invention described above with an activated acyl compound to bond a nitrogen atom in the compound of the invention to the carbonyl of the activated acyl compound. Suitable activated carbonyl compounds contain a good leaving group bonded to the carbonyl carbon and include acyl halides, acyl amines, acyl pyridinium salts, acyl alkoxides, in particular acyl phenoxides such as p-nitrophenoxy acyl, dinitrophenoxy acyl, fluorophenoxy acyl, and difluorophenoxy acyl. The reactions are generally exothermic and are carried out in inert solvents at reduced temperatures such as -78 to about 50 °C. The reactions are usually also carried out in the presence of an inorganic base such as potassium carbonate or sodium bicarbonate, or an organic base such as an amine, including pyridine, TEA, etc. One manner of preparing prodrugs is described in USSN 08/843,369 filed April 15, 1997 (corresponding to PCT publication WO9846576).

### INDICATIONS

The compounds of the invention inhibit the Wnt signaling. Accordingly described herein is a method for treating cancer comprising administering an effective amount of a compound of the invention to a mammal in need thereof. In a particular embodiment, the cancers are associated with aberrant Wnt signaling. In a particular embodiment the cancers are associated with overexpression of Wnt ligands. In a particular embodiment the cancer type is carcinoma, lymphoma, blastoma, or leukemia. Particular cancers include, but are not limited to: chronic lymphocytic leukemia (CLL), lung, including non small cell (NSCLC), breast, ovarian, cervical, endometrial, prostate, colorectal, intestinal carcinoid, bladder, gastric, pancreatic, hepatic (hepatocellular), hepatoblastoma, esophageal, pulmonary adenocarcinoma, mesothelioma, synovial sarcoma, osteosarcoma, head and neck squamous cell carcinoma, juvenile nasopharyngeal angiofibromas, liposarcoma, thyroid, melanoma, basal cell carcinoma (BCC), medulloblastoma and desmoid. In a particular embodiment the cancer is colon cancer. In a particular embodiment the cancer is breast cancer.

Compounds of the invention may be administered prior to, concomitantly with, or following administration of other anticancer treatments such as radiation therapy or chemotherapy. Suitable cytostatic chemotherapy compounds include, but are not limited to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphteria toxin; (Vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate, ET-18- OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists. In a particular embodiment, compounds of the present invention are coadministered with a cytostatic compound selected from the group consisting of cisplatin, doxorubicin, taxol, taxotere and mitomycin C.

The compounds of the present invention can be also used in combination with radiation therapy. The phrase "radiation therapy" refers to the use of electromagnetic or particulate radiation in the treatment of neoplasia. Radiation therapy is based on the principle that high-dose radiation delivered to a target area will result in the death of reproducing cells in both tumor and normal tissues. The radiation dosage regimen is generally defined in terms of radiation absorbed dose (rad), time and fractionation, and must be carefully defined by the oncologist. The amount of radiation a patient receives will depend on various consideration including the location of the tumor in relation to other organs of the body, and the extent to which the tumor has spread. Examples of radiotherapeutic agents are provided in, but not limited to, radiation therapy and is known in the art (Hellman, Principles of Radiation Therapy, Cancer, in Principles I and Practice of Oncology, 24875 (Devita et al., 4th ed., vol 1, 1993). Recent advances in radiation therapy include three-dimensional conformal external beam radiation, intensity modulated radiation therapy (IMRT), stereotactic radiosurgery and brachytherapy (interstitial radiation therapy), the latter placing the source of radiation directly into the tumor as implanted "seeds". These newer treatment modalities deliver greater doses of radiation to the tumor, which accounts for their increased effectiveness when compared to standard external beam radiation therapy.

Ionizing radiation with beta-emitting radionuclides is considered the most useful for radiotherapeutic applications because of the moderate linear energy transfer (LET) of the ionizing particle (electron) and its intermediate range (typically several millimeters in tissue). Gamma rays deliver dosage at lower levels over much greater distances. Alpha particles represent the other extreme, they deliver very high LET dosage, but have an extremely limited range and must, therefore, be in intimate contact with the cells of the tissue to be treated. In addition, alpha emitters are generally heavy metals, which limits the possible chemistry and presents undue hazards from leakage of radionuclide from the area to be treated. Depending on the tumor to be treated all kinds of emitters are conceivably within the scope of the present invention. Furthermore, the present invention encompasses types of non-ionizing radiation like e.g. ultraviolet (UV) radiation, high energy visible flight, microwave radiation (hyperthermia therapy), infrared (IR) radiation and lasers. In a particular embodiment of the present invention UV radiation is applied.

Also described herein are pharmaceutical compositions or medicaments containing the compounds of the invention and a therapeutically inert carrier, diluent or excipient, as well as methods of using the compounds of the invention to prepare such compositions and medicaments. Typically, the compounds of the invention used in the methods are formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but may range from about 3 to about 8. A particular formulation is an acetate buffer at pH 5. The compounds for use herein may be in a sterile formulation. The compound may be stored as a solid composition, although lyophilized formulations or aqueous solutions are acceptable.

The composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to decrease Wnt pathway signaling or else is the minimum amount necessary to cause reduction in size, volume or mass of a tumor that is responsive to Wnt signaling, or a reduction in the increase in size, volume or mass of such a tumor relative to the increase in the absence of administering the compound of the invention.

Alternatively "effective amount" of the compound means the amount necessary to reduce the number of malignant cells or the rate in increase of the number of malignant cells. Alternatively, effective amount" is the amount of the compound of the invention required to increase survival of patients afflicted with an anti-Wnt pathway sensitive tumor. Such amount may be below the amount that is toxic to normal cells, or the mammal as a whole. With respect to non-malignant indications, "effective amount" means the amount of compound of the invention required to decrease severity of the particular indication or symptoms thereof.

Also described herein is a method for treating non-malignant indications involving aberrant Wnt signaling comprising administering an effective amount of a compound of the invention to a mammal in need thereof. Also described herein is a method of treating bone disorders such as osteoarthritis and high bone mass. In another embodiment, the compounds of the invention may be used to treat cardiovascular indications including, but not limited to, pulmonary hypertension, cardiac hypertrophy, pulmonary fibrosis and cardiovascular disease. In another embodiment, the compounds of` the invention may be used to treat neurological conditions including Alzheimer's, autism and schizophrenia. Also described herein is a method of treating renal disorders such polycystic kidney disease or renal fibrosis.

In addition, the compounds of the invention may be administered under conditions wherein Wnt signaling is operating in a normal, nonpathological fashion. In a particular embodiment, the compounds of the invention may be applied topically to prevent initiation of hair follicle formation and development, thereby inhibiting hair growth. In such an application, an "effect amount" is an amount sufficient to prevent hair growth but below an amount that would be toxic to the mammal.

Generally, the initial pharmaceutically effective amount of the compound of the invention administered parenterally per dose will be in the range of about 0.01 to about 100 mg/kg, for example about 0.1 to about 20 mg/kg of patient body weight per day, for example about 0.3 to about 15 mg/kg/day. Oral unit dosage forms, such as tablets and capsules, may contain from about 25 to about 1000 mg of the compound of the invention.

The compound of the invention may be administered by any suitable means, including oral, topical, transdermal, parenteral, subcutaneous, rectal, intraperitoneal, intrapulmonary, and intranasal, and, if desired for local treatment, intralesional administration. Parenteral infusions include intramuscular, intravenous, intraarterial, intraperitoneal, or subcutaneous administration. An example of a suitable oral dosage form is a tablet containing about 25mg, 50mg, 100mg, 250mg, or 500mg of the compound of the invention compounded with about 90-30 mg anhydrous lactose, about 5-40 mg sodium croscarmellose, about 5-30mg polyvinylpyrrolidone (PVP) K30, and about 1-10mg magnesium stearate. The powdered ingredients are first mixed together and then mixed with a solution of the PVP. The resulting composition can be dried, granulated, mixed with the magnesium stearate and compressed to tablet form using conventional equipment. An aerosol formulation can be prepared by dissolving the compound, for example 5-400 mg, of the invention in a suitable buffer solution, e.g. a phosphate buffer, adding a tonicifier, e.g. a salt such sodium chloride, if desired. The solution is typically filtered, e.g. using a 0.2 micron filter, to removed impurities and contaminants. Topical formulations include ointments, creams, lotions, powders, solutions, pessaries, sprays, aerosols and capsules. Ointments and creams may be formulated with an aqueous or oily base with the addition of suitable thickening and/or gelling agents and/or solvents. Such bases may include water and/or an oil such a liquid paraffin or a vegetable oil such as arachis oil or castor oil or a solvent such as a polyethylene glycol. Thickening agents which may be used include soft paraffin, aluminum stearate, cetostearyl alcohol, polyethylene glycols, microcrystalline wax and beeswax. Lotions may be formulated with an aqueous or oily base and may contain one or more emulsifying agents, stabilizing agents, dispersing agents, suspending agents or thickening agents. Powders for external application may be formed with the aid of any suitable powder base e.g. talc, lactose or starch. Drops may be formulated with an aqueous or nonaqueous base also comprising one or more dispersing agents, solubilizing agents or suspending agents.

### EXAMPLES

The invention will be more fully understood by reference to the following examples. They should not, however, be construed as limiting. Abbreviations used herein are as follows:
- Ac: acetyl
- ACN: acetonitrile
- BOC, Boc: *tert*-butoxycarbonyl
- br: broad (spectral)
- °C: degrees Celcius
- cat.: catalytic
- conc.: concentrated
- *δ*: chemical shift in parts per million downfield from tetramethylsilane
- d: days; doublet (spectral)
- *d*: density
- DCM: dichloromethane
- DIPEA: *N,N-*diisopropylethylamine
- DMF: *N,N-*dimethylformamide
- DMSO: dimethyl sulfoxide
- dppf: 1,1'-bis(diphenylphosphino)ferrocene
- equiv.: equivalent
- ES+: electrospray ionisation
- Et: ethyl
- EtOAc: Ethyl acetate
- EtOH: Ethanol
- g: gram(s)
- h: hours
- HPLC: high performance liquid chromatography
- Hz: hertz
- L: litre(s)
- LC: liquid chromatography
- LHMDS: lithium hexamethyldisilazide
- µ: micro
- m: multiplet (spectral); milli
- M: molar (moles per litre); parent molecular ion (spectral - MS); mega
- Me: methyl
- min: minute(s)
- mol: mole(s)
- MS: mass spectrometry
- MTBE: methyl *tert*-butyl ether
- *m*/*z*: mass-to-charge ratio
- NMP: *N*-methylpyrrolidone
- NMR: nuclear magnetic resonance
- obs: obscured (spectral)
- PFA: paraformaldehyde
- Ph: phenyl
- ppm: part(s) per million
- q: quartet (spectral)
- RT: ambient (room) temperature
- s: singlet (spectral)
- t: triplet (spectral)
- TFA: trifluoroacetic acid
- THF: tetrahydrofuran
- TLC: thin layer chromatography
- *tᵣ*: retention time (in chromatography)
- vol: volume (1 vol = 1 mL:1 g)

### Example 1 general procedures

### Method A

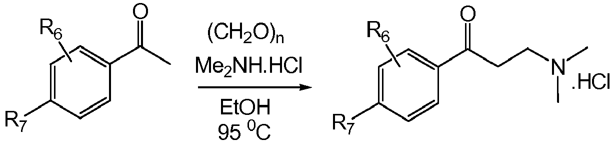

To a stirred suspension of the acetophenone (1 equiv), paraformaldehyde (2 - 2.7 equiv) and dimethylamine hydrochloride (2 equiv) in EtOH (6 vol) was added 2 drops of conc. HCl and the resulting suspension was heated to 95 °C for 18 h. The reaction mixture was allowed to cool to RT and the precipitated solid was filtered, washed with EtOH (1 x 4 vol) then dried to give the desired compound.

### Method B

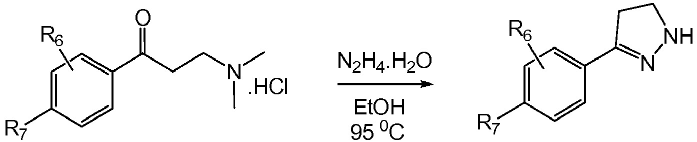

A solution of the amine (1 equiv) and hydrazine hydrate (5 equiv) were heated to 95 °C in EtOH for 4 h. The reaction mixture was cooled to RT and evaporated to a small volume then H₂O (7 vol) was added. The resulting precipitate was filtered and dried to give the desired compound.

### Method C

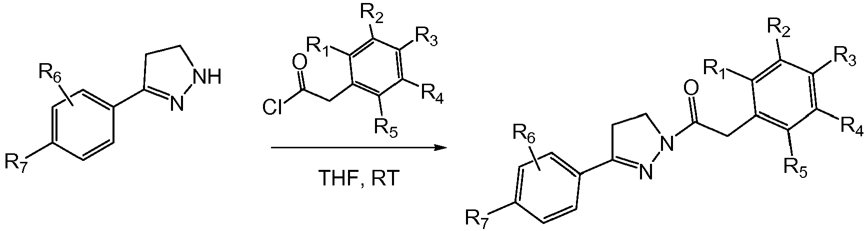

A solution of the dihydropyrazole (1 equiv) and acid chloride (1 equiv) in dry THF (10 vol) was stirred at RT for 2 h. The reaction was quenched with H₂O (70 vol) and the resulting precipitate was filtered. The crude residue was triturated with DCM then MeOH to give the desired compound.

### Method D

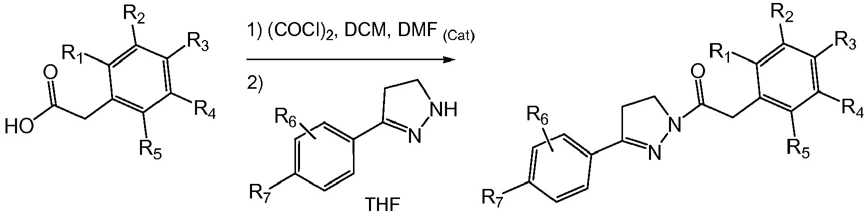

To a solution of the phenyl acetic acid (1 equiv) in DCM (10 vol) was added dropwise oxalyl chloride (2 equiv) and DMF (1 drop). The resulting solution was stirred at RT for 1 hr and evaporated. The residue was redissolved in THF (10 vol), the dihydropyrazole (1.2 equiv) was added and the resulting solution was stirred at RT for 1 h. The reaction mixture was quenched with H₂O (40 vol) and the resulting precipitate was filtered. This was washed with EtOAc (4 vol) and DCM (4 vol) then dried to give the desired compound.

### Method E

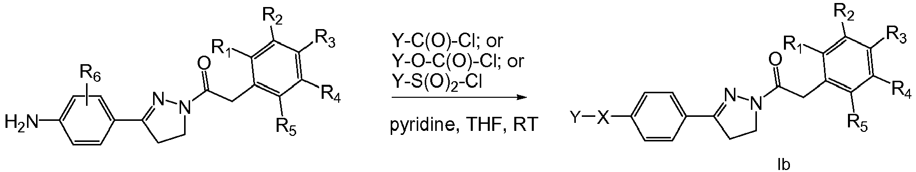

The corresponding acid chloride, sulfonyl chloride or chloroformate (5 equiv) was added to a suspension of the dihydropyrazole (1 equiv) in acetonitrile (20 vol) and pyridine (5 equiv) and the resulting mixture was stirred at RT for 3 h. The reaction mixture was quenched with H₂O (60 equiv) and an oil separated which crystallised overnight. The solid was filtered to give the desired compound.

### Method F

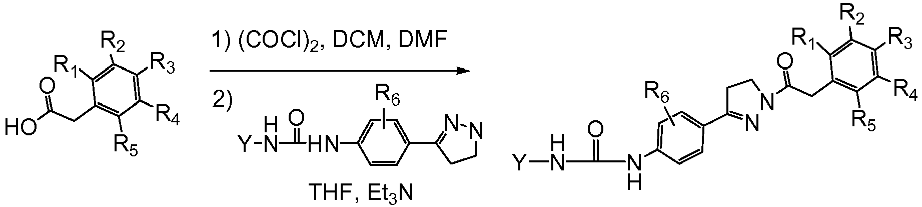

To a solution of the phenyl acetic acid (1 equiv) in DCM (1 mL) was added oxalyl chloride (2 equiv) dropwise and DMF (1 drop). The reaction was stirred at RT for 1hr then evaporated. The residue was redissolved in THF (10 vol) and the dihydro pyrazole (1 equiv) was added. After stirring at RT for 10 min, Et₃N (3 equiv) was added dropwise, slowly until fuming ceased. The reaction was stirred at RT for 1.5 h after which time H₂O (40 vol) was added. The resulting precipitate was filtered and washed with EtOAc (40 vol) and DCM (4 vol) then dried to give the desired compound.

### Method G

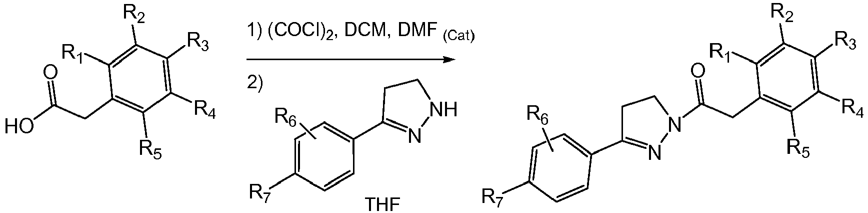

To a stirred solution of the phenyl acetic acid (1 equiv) in THF (20 vol) was added oxalyl chloride (1 equiv) and DMF (1 drop). The solution was stirred at RT for 3 h and then the dihydropyrazole (1 equiv) was added. The resulting suspension was stirred for 4 h at RT, then poured into water (80 vol), the resulting precipitate was filtered and washed with H₂O (80 vol x 2) and acetone (80 vol x 2) then dried to give the desired compound.

### Example 2 2-(3,4-dimethoxyphenyl)-1-(3-(4-hydroxy-3-methylphenyl)-4,5-dihydro-1H-pyrazol-1-yl)ethanone (6)

4-hydroxy-3-methyl acetophenone (450 mg, 3.0 mmol) was treated with paraformaldehyde (240 mg, 8.1 mmol) and dimethylamine hydrochloride (489 mg, 6.0 mmol) using method A to give 3-dimethylamino-1-(4-hydroxy-3-methyl-phenyl)-propan-1-one hydrochloride salt. Yield: 612 mg (84%). ¹H NMR δ_{H} ppm (360 MHz, D₆-DMSO):10.60 (1H, brs), 10.57 (1H, s) 7.80 (1H, brs), 7.74 (1H, dd), 6.97 (1H, d), 3.51 (2H, t), 3.37 (2H, obs), 2.80 (6H, s), 2.19 (3H, s).

A solution of (4) (612 mg, 2.51 mmol) and hydrazine hydrate (0.98 mL, 20.1 mmol) in EtOH (3 mL) was heated to 95 °C for 4 h. The EtOH was evaporated and H₂O (6 mL) was added. An oil separated which was extracted with DCM (3 x 2 mL), washed with H₂O (2 mL) and evaporated to give 4-(4,5-dihydro-1*H-*pyrazol-3-yl)-2-methyl-phenol (5) as a sticky gum. Yield: 139 mg (31%). ¹H NMR δ_{H} ppm (360 MHz, D₆-DMSO): 9.33 (1H, brs), 7.19 (1H, d), 7.10 (1H, dd), 6.61 (1H, d), 3.12 (2H, t), 2.66 (2H, t), 1.98 (3H, s).

4-(4,5-dihydro-1*H*-pyrazol-3-yl)-2-methyl-phenol (5) (42 mg, 0.24 mmol) was treated with 3,4-dimethoxyphenyl acetyl chloride (52 mg, 0.24 mmol) using method C to give final compound 2-(3,4-dimethoxy-phenyl)-1-[3-(4-hydroxy-3-methyl-phenyl)-4,5-dihydro-pyrazol-1-yl]-ethanone (6). Yield: 5 mg (6%). LC/MS *t*ᵣ 3.87 min. MS(ES+) *m*/*z* 355 (M+H). ¹H NMR δ_{H} ppm (360 MHz, D₆-DMSO): 9.91 (1H, brs), 7.56 (1H, d), 7.47 (1H, dd), 6.97 (1H, d),6.88 (1H, d), 6.86 (1H, d), 6.83 (1H, dd), 3.91 (2H, s), 3.86 (2H, t), 3.72 (6H, s), 3.22 (2H, t), 2.18 (3H, t).

### Example 3 2-(3,4-dimethoxyphenyl)-1-(3-(2-fluoro-4-hydroxyphenyl)-4,5-dihydro-1H-pyrazol-1-yl)ethanone (9)

2-Fluoro-4-hydroxy acetophenone (462 mg, 3.0 mmol) was treated with paraformaldehyde (240 mg, 8.1 mmol) and dimethylamine hydrochloride (489 mg, 6.0 mmol) using method A to give 3-dimethylamino-1-(2-fluoro-4-hydroxy-phenyl)-propan-1-one hydrochloride salt (7). Yield: 543 mg (73%). ¹H NMR δ_{H} ppm (360 MHz, D₆-DMSO): 11.15 (1H, brs), 10.30 (1H, brs), 7.81 (1H, m), 6.6 - 6.7 (2H, m), 3.40 (4H, obs), 2.79 (6H, s).

A solution of 3-dimethylamino-1-(2-fluoro-4-hydroxy-phenyl)-propan-1-one hydrochloride (543 mg, 2.19 mmol) and hydrazine hydrate (0.53 mL, 11 mmol) in EtOH (2.7 mL) was heated to 95 °C for 5 h. The EtOH was evaporated and H₂O (3 mL) was added followed by 6M HCl (1 mL). An oil separated which solidified overnight. The solid was filtered, washed with H₂O and dried to give 4-(4,5-dihydro-1*H-*pyrazol-3-yl)-3-fluoro-phenol (8) as a hard, reddish gum. Yield: 176 mg (44%). ¹H NMR δ_{H} ppm (360 MHz, D₆-DMSO): 9.83 (1H, brs), 7.38 (1H, t), 6.70 (1H, brs), 6.4 - 6.5 (2H, m), 3.14 (2H, t), 2.73 (2H, t).

4-(4,5-Dihydro-1*H-*pyrazol-3-yl)-3-fluoro-phenol (8) (54 mg, 0.3 mmol) was treated with 3,4-dimethoxyphenyl acetyl chloride (64 mg, 0.3 mmol) using method C to give 2-(3,4-dimethoxy-phenyl)-1-[3-(4-hydroxy-3-methyl-phenyl)-4,5-dihydro-pyrazol-1-yl]-ethanone (9). Yield: 34 mg (31%). LC/MS *t*ᵣ 3.82 min. MS(ES+) *m*/*z* 359 (M+H). ¹H NMR δ_{H} ppm (360 MHz, D₆-DMSO): 10.43 (1H, brs), 7.67 (1H, t), 6.88 (1H, d), 6.80 (1H, d), 6.76 (1H, dd), 6.5 - 6.7 (2H, m), 3.82 (2H, s), 3.77 (2H, t), 3.65 (6H, s), 3.20 (2H, t).

### Example 4 2-(3-ethoxy-4-methoxyphenyl)-1-(3-(4-hydroxyphenyl)-4,5-dihydro-1H-pyrazol-1-yl)ethanone

(3-Hydroxy-4-methoxy-phenyl)-acetic acid (182 mg, 1 mmol) was dissolved in acetone (3.6 mL, previously dried over potassium carbonate). Cesium carbonate (717 mg, 2.2 mmol) and ethyl iodide (0.18 mL, 2.2 mmol) were added and the reaction heated to 65 °C for 3.5 h. After cooling, H₂O (4 mL) was added and the mixture extracted with EtOAc (2 x 2 mL). The combined organic phases were evaporated and the residue dissolved in THF (2 mL) and H₂O (1 mL). Lithium hydroxide (92 mg, 2.2 mmol) was added and the reaction heated to 70 °C for 2.5 h. The reaction was allowed to cool to RT then diluted with H₂O (2 mL) and extracted with MTBE (2 x 2 mL). The aqueous phase was acidified with 6M HCl then extracted with EtOAc (2 x 2 mL). The combined organic phases were washed with brine (2 mL), dried (MgSO₄), filtered and evaporated to give (3-Ethoxy-4-methoxy-phenyl)-acetic acid (10). Yield: 58 mg (12%). ¹H NMR δ_{H} ppm (360 MHz, D₆-DMSO): 12.23 (1H, brs), 6.88 (1H, d), 6.85 (1H, d), 6.76 (1H, dd), 3.98 (2H, q), 3.74 (3H, s), 3.47 (2H, s), 1.32 (3H, t).

4-hydroxyacetophenone (13.6 g, 100 mmol) was treated with paraformaldehyde (6.0 g, 200 mmol) and dimethylamine hydrochloride (16.3 g, 200 mmol) using method A to give 3-dimethylamino-1-(4-hydroxy-phenyl)-propan-1-one hydrochloride salt (1). Yield: 20.0 g (87%). LC/MS *t*ᵣ 0.48 min. MS(ES+) *m*/*z* 194 (M+H). ¹H NMR δ_{H} ppm (400 MHz, D₆-DMSO): 10.58 (1H, s), 10.37 (1H, brs), 7.88 (2H, d), 6.89 (2H, d), 3.48 (2H, t), 3.37 (2H, t), 2.77 (6H, s).

3-dimethylamino-1-(4-hydroxy-phenyl)-propan-1-one hydrochloride salt (1) (20.0 g, 87 mmol) was treated with hydrazine hydrate (21.8 g, 435 mmol) using method B to give 4-(4,5-dihydro-1*H-*pyrazol-3-yl)-phenol (2) as an off-white solid. Yield: 8.1 g (57%). LC/MS *t*ᵣ 0.36 min. MS(ES+) *m*/*z* 163 (M+H). ¹H NMR δ_{H} ppm (400 MHz, D₆-DMSO): 7.42 (2H, d), 6.74 (2H, d), 3.27 (2H, t), 2.81 (2H, t).

(3-Ethoxy-4-methoxy-phenyl)-acetic acid (10) (42 mg, 0.2 mmol) was treated with oxalyl chloride (19 µL, 0.22 mmol) then 4-(4,5-dihydro-1*H*-pyrazol-3-yl)-phenol (2) (32 mg, 0.2 mmol) using method D to give final compound 2-(3-Ethoxy-4-methoxy-phenyl)-1-[3-(4-hydroxy-phenyl)-4,5-dihydro-pyrazol-1-yl]-ethanone (11). Yield: 23 mg (32%). LC/MS *t*ᵣ 3.86 min. MS(ES+) *m*/*z* 355 (M+H). ¹H NMR δ_{H} ppm (360 MHz, D₆-DMSO): 10.04 (1H, s), 7.69 (sH, d), 7.00 (1H, d), 6.8 - 6.9 (4H, m), 4.00 (2H, q), 3.93 (2H, s), 3.90 (2H, t), 3.76 (3H,s), 3.27 (2H, t), 1.34 (3H, t).

### Example 5 1-[3-(4-Amino-phenyl)-4,5-dihydro-pyrazol-1-yl]-2-(3,4-dimethoxy-phenyl)-ethanone (15)

4-nitroacetophenone (12.26 g, 0.074 mol) was treated with paraformaldehyde (6.0 g, 0.2 mol) and dimethylamine hydrochloride (12.07 g, 0.15 mol) using method A to give 3-dimethylamino-1-(4-nitro-phenyl)-propan-1-one-hydrochloride salt (12) as an off-white solid. Yield: 13.03 g (68%).

3-dimethylamino-1-(4-nitro-phenyl)-propan-1-one-hydrochloride (12) (13.0 g, 0.05 mol) was treated with hydrazine hydrate (19.50 mL, 0.402 mol) using method B to give 3-(4-nitro-phenyl)-4,5-dihydro-1*H-*pyrazole (13) as an orange solid. Yield: 7.87 g (82%). LC/MS *t*ᵣ 1.25 min (96%). MS(ES+) *m*/*z* 192 (M+H).

3-(4-nitro-phenyl)-4,5-dihydro-1*H*-pyrazole (13) (2.21 g, 11.6 mmol) was treated with 3,4-dimethoxyphenyl acetyl chloride (2.48 g, 11.6 mmol) using method C to give 2-(3,4-dimethoxy-phenyl)-1-[3-(4-nitro-phenyl)-4,5-dihydro-pyrazol-1-yl]-ethanone (14). Yield: 3.12 g (100%). LC/MS *t*ᵣ 4.21 min. MS(ES+) *m*/*z* 370 (M+H). ¹H NMR δ_{H} ppm (360 MHz, D₆-DMSO): 8.37 (2H, d), 8.09 (2H, d), 7.00 (1H, d), 6.93 (1H, d), 6.89 (1H, dd), 4.01 (4H, m), 3.76 (6H, s), 3.39 (2H, t).

A suspension of 2-(3,4-dimethoxy-phenyl)-1-[3-(4-nitro-phenyl)-4,5-dihydro-pyrazol-1-yl]-ethanone (14) (3.12 g, 8.46 mmol) and 10% palladium on charcoal (1.25 g, 0.4 weight) in acetonitrile (125 mL) was vigorously stirred under a hydrogen atmosphere for 5 h. The reaction mixture was flushed with nitrogen then filtered through a pad of celite. The residue was then boiled in acetonitrile and filtered while hot (4 x 125 mL). The combined acetonitrile solutions were evaporated to give a yellow / orange solid. This was heated to 50 °C in DCM (25 mL), cooled and filtered to give 1-[3-(4-Amino-phenyl)-4,5-dihydro-pyrazol-1-yl]-2-(3,4-dimethoxy-phenyl)-ethanone (15) as a yellow solid. Yield: 2.61 g (91%). LC/MS *t*ᵣ 3.58 min. MS(ES+) *m*/*z* 340 (M+H). ¹H NMR δ_{H} ppm (360 MHz, D₆-DMSO): 7.57 (2H, d), 7.05 (1H, d), 6.96 (1H, d), 6.91 (1H, dd), 6.70 (2H, d), 5.77 (2H, brs), 3.97 (2H, s), 3.91 (2H, t), 3.80 (6H, s), 3.26 (2H, t).

### Example 6 2-(3,4-dimethoxyphenyl)-1-(3-(4-(methylamino)phenyl)-4,5-dihydro-1H-pyrazol-1-yl)ethanone (16)

1-[3-(4-Amino-phenyl)-4,5-dihydro-pyrazol-1-yl]-2-(3,4-dimethoxy-phenyl)-ethanone (15) (50 mg, 0.15 mmol) was dissolved in acetonitrile (1 mL) and DMF (0.5 mL). Methyl iodide (0.14 mL, 2.25 mmol) was added and the reaction heated to 60 °C for 30 min then allowed to cool to RT and H₂O (3 mL) was added. The resulting precipitate was filtered, washed with H₂O and dried and the resulting residue purified by column chromatography (50 - 100% EtOAc in heptane) to give a mixture of 1-[3-(4-Amino-phenyl)-4,5-dihydro-pyrazol-1-yl]-2-(3,4-dimethoxy-phenyl)-ethanone (15), 2-(3,4-dimethoxyphenyl)-1-(3-(4-(methylamino)phenyl)-4,5-dihydro-1H-pyrazol-1-yl)-ethanone (16) and the corresponding dimethylated aniline. These were separated by preparative HPLC to give the final compound 2-(3,4-dimethoxyphenyl)-1-(3-(4-(methylamino)phenyl)-4,5-dihydro-1H-pyrazol-1-yl)ethanone as the TFA salt. Yield: 5 mg (9%). LC/MS *t*ᵣ 4.03 min. MS(ES+) *m*/*z* 354 (M+H). ¹H NMR δ_{H} ppm (360 MHz, D₄-MeOD): 7.65 (2H, d), 7.01 (1H, d), 6.90 (1H, dd), 6.85 (1H, d), 6.72 (2H, d), 3.97 (2H, s), 3.91 (2H, t), 3.77 (6H, s), 3.24 (2H, t), 2.84 (3H, s).

### Example 7 N-(4-{1-[2-(3,4-Dimethoxy-phenyl)-acetyl]-4,5-dihydro-1H-pyrazol-3-yl}-phenyl)-isobutyramide (17)

1-[3-(4-Amino-phenyl)-4,5-dihydro-pyrazol-1-yl]-2-(3,4-dimethoxy-phenyl)-ethanone (15) (50 mg, 0.15 mmol) was treated with isobutyryl chloride (77 µL, 0.74 mmol) using method E. The crude residue was purified by column chromatography (50 - 100% EtOAc in heptane) to give final compound *N-*(4-{1-[2-(3,4-Dimethoxy-phenyl)-acetyl]-4,5-dihydro-1*H-*pyrazol-3-yl}-phenyl)-isobutyramide (17). Yield: 33 mg (54%). LC/MS *t*ᵣ 4.04 min. MS(ES+) *m*/*z* 410 (M+H). ¹H NMR δ_{H} ppm (360 MHz, CDCl₃): 7.64 (2H, d), 7.55 (2H, d), 7.23 (1H, brs), 6.92 (1H, brs), 6.88 (1H, d), 6.74 (1H, d), 3.96 (4H, m), 3.79 (3H, s), 3.77 (3H, s), 3.13 (2H, t), 2.47 (1H, m), 1.21 (6H, d).

### Example 8 N-(4-{1-[2-(3,4-dimethoxy-phenyl)-acetyl]-4,5-dihydro-1H-pyrazol-3-yl}-phenyl)-propionamide (18)

1-[3-(4-Amino-phenyl)-4,5-dihydro-pyrazol-1-yl]-2-(3,4-dimethoxy-phenyl)-ethanone (15) (50 mg, 0.15 mmol) was treated with propionyl chloride (65 µL, 0.74 mmol) using method E to give final compound *N-*(4-{1-[2-(3,4-dimethoxy-phenyl)-acetyl]-4,5-dihydro-1*H*-pyrazol-3-yl}-phenyl)-propionamide. Yield: 27 mg (45%). LC/MS *t*ᵣ 3.85 min. MS(ES+) *m*/*z* 396 (M+H). ¹H NMR δ_{H} ppm (360 MHz, CDCl₃): 7.64 (2H, d), 7.55 (2H, d), 7.32 (1H, brs), 6.92 (1H, brs), 6.88 (1H, d), 6.74 (1H, d), 3.96 (4H, m), 3.78 (6H, s), 3.13 (2H, t), 2.36 (2H, q), 1.20 (3H, t).

### Example 9 cyclopropanecarboxylic acid (4-{1-[2-(3,4-dimethoxy-phenyl)-acetyl]-4,5-dihydro-1H-pyrazol-3-yl}-phenyl)-amide (19)

1-[3-(4-amino-phenyl)-4,5-dihydro-pyrazol-1-yl]-2-(3,4-dimethoxy-phenyl)-ethanone (15) (50 mg, 0.15 mmol) was treated with cyclopropane carboxylic acid chloride (67 µL, 0.74 mmol) using method E to give final compound cyclopropanecarboxylic acid (4-{1-[2-(3,4-dimethoxy-phenyl)-acetyl]-4,5-dihydro-1*H*-pyrazol-3-yl}-phenyl)-amide (19). Yield: 41 mg (67%). LC/MS *t*ᵣ 3.96 min. MS(ES+) *m*/*z* 408 (M+H). ¹H NMR δ_{H} ppm (360 MHz, CDCl₃): 7.72 (1H, brs), 7.63 (2H, d), 7.54 (2H, d), 6.92 (1H, brs), 6.88 (1H, d), 6.73 (1H, d), 3.96 (4H, m), 3.77 (6H, s), 3.13 (2H, t), 1.47 (1H, m), 1.04 (2H, m), 0.81 (2H, m).

### Example 10 N-(4-{1-[2-(3,4-Dimethoxy-phenyl)-acetyl]-4,5-dihydro-1H-pyrazol-3-yl}-phenyl)-2-phenyl-acetamide (20)

1-[3-(4-Amino-phenyl)-4,5-dihydro-pyrazol-1-yl]-2-(3,4-dimethoxy-phenyl)-ethanone (15) (50 mg, 0.15 mmol) was treated with phenyl acetyl chloride (98 µL, 0.74 mmol) using method E. The crude residue was purified by column chromatography (50-100% EtOAc in heptane) to give final compound *N-*(4-{1-[2-(3,4-dimethoxy-phenyl)-acetyl]-4,5-dihydro-1*H-*pyrazol-3-yl}-phenyl)-2-phenyl-acetamide (20). Yield: 56 mg (81%). LC/MS *t*ᵣ 4.26 min. MS(ES+) *m*/*z* 458 (M+H). ¹H NMR δ_{H} ppm (360 MHz, CDCl₃): 8.02 (1H, brs), 7.67 (2H, d), 7.58 (2H, d), 7.3 - 7.4 (5H, m), 6.99 (1H, d), 6.96 (1H, dd), 6.81 (1H, d), 4.05 (2H, s), 4.01 (2H, t), 3.83 (6H, s), 3.74 (2H, s), 3.18 (2H, t).

### Example 11 4-methyl-[1,2,3]thiadiazole-5-carboxylic acid (4-{1-[2-(3,4-dimethoxy-phenyl)-acetyl]-4,5-dihydro-1H-pyrazol-3-yl}-phenyl)-amide (21)

4-Methyl-1,2,3-thiadiazole-5-carboxylic acid (24 mg, 0.16 mmol), triethylamine (23 µL, 0.16 mmol) and HATU (62 mg, 0.16 mmol) were stirred in DMF (0.5 mL) for 70 min. 1-[3-(4-Amino-phenyl)-4,5-dihydro-pyrazol-1-yl]-2-(3,4-dimethoxy-phenyl)-ethanone (15) (50 mg, 0.15 mmol) was added and the reaction stirred at RT for 2 h then heated to 85 °C for a further 2 h. After cooling to RT, H₂O (3 mL) was added and a solid precipitated. This was filtered, washed with H₂O and dried to give final compound 4-methyl-[1,2,3]thiadiazole-5-carboxylic acid (4-{1-[2-(3,4-dimethoxy-phenyl)-acetyl]-4,5-dihydro-1*H*-pyrazol-3-yl}-phenyl)-amide (21). Yield: 21 mg (30%). LC/MS *t*ᵣ 4.09 min.
MS(ES+) *m*/*z* 466 (M+H). ¹H NMR δ_{H} ppm (360 MHz, CDCl₃): 8.15 (1H, brs), 7.76 (2H, d), 7.68 (2H, d), 6.94 (1H, brs), 6.90 (1H, d), 6.77 (1H, d), 4.01 (4H, m), 3.82 (6H, s), 3.21 (2H, t), 2.96 (3H, s).

### Example 12 N-(4-{1-[2-(3,4-dimethoxy-phenyl)-acetyl]-4,5-dihydro-1H-pyrazol-3-yl}-phenyl)-methanesulfonamide (22)

1-[3-(4-Amino-phenyl)-4,5-dihydro-pyrazol-1-yl]-2-(3,4-dimethoxy-phenyl)-ethanone (15) (100 mg, 0.3 mmol) was treated with methane sulfonyl chloride (114 µL, 1.48 mmol) using method E. The crude residue was triturated with DCM to give final compound *N*-(4-{1-[2-(3,4-dimethoxyphenyl)-acetyl]-4,5-dihydro-1*H*-pyrazol-3-yl}-phenyl)-methanesulfonamide (22). Yield: 58 mg (46%). LC/MS *t*ᵣ 3.68 min. MS(ES+) *m*/*z* 418 (M+H). ¹H NMR δ_{H} ppm (360 MHz, D₆-DMSO): 10.01 (1H, brs), 7.78 (2H, d), 7.30 (2H, d), 6.95 (1H, d), 6.88 (1H, d), 6.84 (1H, dd), 3.92 (2H,s), 3.90 (2H, t), 3.72 (6H, s), 3.26 (2H, t), 3.08 (3H, s).

### Example 13 Ethanesulfonic acid (4-{1-[2-(3,4-dimethoxy-phenyl)-acetyl]-4,5-dihydro-1H-pyrazol-3-yl}-phenyl)-amide (23)

1-[3-(4-Amino-phenyl)-4,5-dihydro-pyrazol-1-yl]-2-(3,4-dimethoxy-phenyl)-ethanone (15) (50 mg, 0.15 mmol) was treated with ethane sulfonyl chloride (70 µL, 0.74 mmol) using method E except that the reaction was heated to 70 °C for 1 h. After this time, H₂O (4 mL) was added and the resulting precipitate was filtered to give the crude residue. This was triturated in DCM to give the final compound ethanesulfonic acid (4-{1-[2-(3,4-dimethoxy-phenyl)-acetyl]-4,5-dihydro-1*H-*pyrazol-3-yl}-phenyl)-amide (23). Yield: 22 mg (34%). LC/MS *t*ᵣ 3.83 min. MS(ES+) *m*/*z* 432 (M+H). ¹H NMR δ_{H} ppm (360 MHz, D₆-DMSO): 9.95 (1H, brs), 7.59 (2H, d), 7.13 (2H, d), 6.77 (1H, brs), 6.70 (1H, d), 6.66 (1H, d), 3.74 (2H, s), 3.71 (2H, t), 3.54 (6H, s), 3.08 (2H, t), 3.00 (2H, q), 1.03 (3H, t).

### Example 14 1-(4-(1-(2-(3,4-dimethoxyphenyl)acetyl)-4,5-dihydro-1H-pyrazol-3-yl)phenyl)-urea (25)

1-[3-(4-Amino-phenyl)-4,5-dihydro-pyrazol-1-yl]-2-(3,4-dimethoxy-phenyl)-ethanone (15) (50 mg, 0.15 mmol) was treated with 4-nitrophenyl chloroformate (149 mg, 0.74 mmol) using method E to give (4-{1-[2-(3,4-dimethoxy-phenyl)-acetyl]-4,5-dihydro-1*H-*pyrazol-3-yl}-phenyl)-carbamic acid 4-nitro-phenyl ester (24). Yield: 38 mg (50%). ¹H NMR δ_{H} ppm (360 MHz, CDCl₃): 8.22 (2H, d), 7.68 (2H, d), 7.48 (3H, m), 7.32 (2H, d), 6.91 (1H, d), 6.88 (1H, dd), 6.73 (1H, d), 3.97 (4H, m), 3.77 (6H, s), 3.15 (2H, t).
(4-{1-[2-(3,4-dimethoxy-phenyl)-acetyl]-4,5-dihydro-1*H*-pyrazol-3-yl}-phenyl)-carbamic acid 4-nitro-phenyl ester (24) (93 mg, 0.185 mmol) and ammonium acetate (57 mg, 0.74 mmol) were dissolved in THF (1.8 mL). Triethylamine (103µL, 0.74 mmol) was added and the resulting suspension stirred at RT for 4 h, after which time H₂O (6 mL) was added in 3 portions. The resulting precipitate was filtered, washed with H₂O then dried to give the crude residue. This was triturated in hot DCM, filtered and dried to give final compound 1-(4-{1-[2-(3,4-dimethoxy-phenyl)-acetyl]-4,5-dihydro-1*H*-pyrazol-3-yl}-phenyl)-urea (25). Yield: 38 mg (53%). LC/MS *t*ᵣ 3.39 min. MS(ES+) *m*/*z* 383 (M+H). ¹H NMR δ_{H} ppm (360 MHz, D₆-DMSO): 8.96 (1H, brs), 7.81 (2H, d), 7.64 (2H, d), 7.09 (1H, brs), 7.01 (1H, d), 6.97 (1H, d), 6.11 (2H, brs), 4.04 (2H, s), 4.00 (2H, t), 3.84 (6H, s), 3.37 (2H, t).

### Example 15 1-(4-{1-[2-(3,4-Dimethoxy-phenyl)-acetyl]-4,5-dihydro-1H-pyrazol-3-yl}-phenyl)-3-methyl-urea (26)

Methyl isocyanate (43 µL, 0.74 mmol) was added to a suspension of the 1-[3-(4-amino-phenyl)-4,5-dihydro-pyrazol-1-yl]-2-(3,4-dimethoxy-phenyl)-ethanone (15) (50 mg, 0.15 mmol) in acetonitrile (1 mL) and the resulting mixture was stirred at 70 °C for 25 h. The reaction mixture was quenched with H₂O (4 mL) and the resulting precipitate was filtered and washed with H₂O (5 mL) then dried to give final compound 1-(4-{1-[2-(3,4-Dimethoxy-phenyl)-acetyl]-4,5-dihydro-1*H*-pyrazol-3-yl}-phenyl)-3-methyl-urea (26). Yield: 13 mg (22%). LC/MS *t*ᵣ 3.53 min. MS(ES+) *m*/*z* 397 (M+H). ¹H NMR δ_{H} ppm (360 MHz, D₆-DMSO): 8.87 (1H, brs), 7.73 (2H, d), 7.56 (2H, d), 7.01 (1H, d), 6.93 (1H, d), 6.89 (1H, dd), 6.16 (1H, q), 3.96 (2H, s), 3.92 (2H, t), 3.77 (6H, s), 3.29 (2H, t), 2.71 (3H, d).

### Example 16 3-(4-{1-[2-(3,4-Dimethoxy-phenyl)-acetyl]-4,5-dihydro-1H-pyrazol-3-yl}-phenyl)-1,1-dimethyl-urea (27)

(4-{1-[2-(3,4-dimethoxy-phenyl)-acetyl]-4,5-dihydro-1*H*-pyrazol-3-yl}-phenyl)-carbamic acid 4-nitro-phenyl ester (24) (38 mg, 0.075 mmol) and dimethylamine hydrochloride (12 mg, 0.15 mmol) were dissolved in THF (0.7 mL). Triethylamine (21 µL, 0.15 mmol) was added and the reaction stirred at RT for 90 min, after which time H₂O (4 mL) was added. The resulting oil was extracted into EtOAc (2 x 3 mL). The combined organic extracts were washed with saturated sodium carbonate (3 mL) and brine (2 mL), dried (MgSO₄), filtered and evaporated to give the crude residue. This was purified by column chromatography (0 - 30% MeOH in EtOAc) to give final compound 3-(4-{1-[2-(3,4-dimethoxy-phenyl)-acetyl]-4,5-dihydro-1*H-*pyrazol-3-yl}-phenyl)-1,1-dimethyl-urea (27). Yield: 12 mg (39%). LC/MS *t*ᵣ 3.72 min. MS(ES+) *m*/*z* 411 (M+H). ¹H NMR δ_{H} ppm (360 MHz, CDCl₃): 7.61 (2H, d), 7.41 (2H, d), 6.93 (1H, brs), 6.89 (1H, d), 6.73 (1H, d), 6.40 (1H, brs), 3.96 (4H, m), 3.78 (3H, s), 3.77 (3H, s), 3.13 (2H, t), 3.00 (6H, s).

### Example 17 1-(4-{1-[2-(2-Bromo-4,5-dimethoxy-phenyl)-acetyl]-4,5-dihydro-1H-pyrazol-3-yl}-phenyl)-3-prop-2-ynyl-urea (70)

A solution of (4-{1-[2-(3,4-dimethoxy-phenyl)-acetyl]-4,5-dihydro-1*H*-pyrazol-3-yl}-phenyl)-carbamic acid 4-nitro-phenyl ester (24) (26 mg, 0.045 mmol) and propargyl amine (6µL, 0.09 mmol) in DCM (1 mL) was stirred at RT for 2 h. The resulting precipitate was filtered, washed with DCM and dried to give final compound 1-(4-{1-[2-(2-Bromo-4,5-dimethoxy-phenyl)-acetyl]-4,5-dihydro-1*H*-pyrazol-3-yl}-phenyl)-3-prop-2-ynyl-urea (70). Yield: 6 mg (27%). LC/MS *t*ᵣ 4.04 min. MS(ES+) *m*/*z* 499/501 (M+H). ¹H NMR δ_{H} ppm (360 MHz, D₆-DMSO): 8.96 (1H, s), 7.74 (2H, d), 7.57 (2H, d), 7.18 (1H, s), 7.10 (1H, s), 6.63 (1H, t), 4.14 (2H, s), 3.96 (4H, m), 3.82 (3H, s), 3.78 (3H, s), 3.33 (2H, t), 3.19 (1H, t).

### Example 18 2-(2-Chloro-4,5-dimethoxy-phenyl)-1-[3-(4-hydroxy-phenyl)-4,5-dihydro-pyrazol-1-yl]-ethanone (28)

A solution of 3,4-dimethoxy phenyl acetic acid (1.96 g, 10 mmol), oxone (6.15 g, 10 mmol) and KCl (1.49 g, 20 mmol) in acetonitrile (20 mL) and H₂O (20 mL) was stirred at RT for 1 hr. The reaction mixture was diluted with EtOAc (75 mL) and H₂O (25 mL) and the organic layer separated and evaporated. The residue was dissolved in EtOAc (50 mL) and washed with brine (3 x 25 mL). The organic layer was separated and evaporated to give 2-chloro-4-5-dimethoxyphenylacetic acid (3). Yield: 1.26 g (quant). LC/MS *t*ᵣ 1.56 min. ¹H NMR δ_{H} ppm (400 MHz, D₆-DMSO): 12.35 (1H, brs), 6.98 (2H, s), 3.74 (3H, s), 3.72 (3H, s) 3.59 (2H, s).

4-(4,5-dihydro-1*H*-pyrazol-3-yl)-phenol (2) (97 mg, 0.6 mmol) was treated with 2-chloro-4-5-dimethoxyphenyl acetic acid (3) (115 mg, 0.5 mmol) using method D to give final compound 2-(2-Chloro-4,5-dimethoxy-phenyl)-1-[3-(4-hydroxy-phenyl)-4,5-dihydro-pyrazol-1-yl]-ethanone (28). Yield: 5 mg (3%). LC/MS *t*ᵣ 3.94 min. MS(ES+) *m*/*z* 275 (M+H). ¹H NMR δ_{H} ppm (400 MHz, D₆-DMSO): 10.02 (1H, s), 7.61 (2B, d), 6.69 (1H. s), 6.97 (1H, s), 6.83 (2H, d), 4.03 (2H, s), 3.86 (2H, t), 3.74 (3H, s), 3.70 (3H, s), 3.22 (2H, t).

### Reference

### Example 19 2-(4-Benzyloxy-3-methoxy-phenyl)-1-[3-(4-hydroxy-phenyl)-4,5-dihydro-pyrazol-1-yl]-ethanone (29)

4-(4,5-dihydro-1*H*-pyrazol-3-yl)-pbenol (2) (97 mg, 0.6 mmol) was treated with 4-benzyloxy-3-methoxy phenyl acetic acid (136 mg, 0.5 mmol) using method D to give final compound 2-(4-Benzyloxy-3-methoxy-phenyl)-1-[3-(4-hydroxy-phenyl)-4,5-dihydro-pyrazol-1-yl]-ethanone (29). Yield: 97 mg (46%). LC/MS *t*ᵣ 4.31 min. MS(ES+) *m*/*z* 417 (M+H). ¹H NMR δ_{H} ppm (400 MHz, D₆-DMSO): 9.98 (1H, s), 7.63 (2H, d), 7.29 - 7.43 (5H, m), 6.76 - 6.96 (5H, m), 5.02 (2H, s), 3.79 - 3.87 (4H, m), 3.71 (3H, s), 3.20 (2H, t).

### Reference

### Example 20 2-(2,4-Dimethoxy-phenyl)-1-[3-(4-hydroxy-phenyl)-4,5-dihydro-pyrazol- 1-yl]-ethanone (30)

4-(4,5-Dihydro-1*H*-pyrazol-3-yl)-phenol (2) (97 mg, 0.6 mmol) was treated with 2-4-dimethoxy phenyl acetic acid (98 mg, 0.5 mmol) using method D to give final compound 2-(2,4-dimethoxyphenyl)-1-[3-(4-hydroxy-phenyl)-4,5-dihydro-pyrazol-1-yl]-ethanone (30). Yield: 52 mg (31%). LC/MS *t*ᵣ 3.88 min. MS(ES+) *m*/*z* 341 (M+H). ¹H NMR δₙ ppm (400 MHz, D₆-DMSO): 9.96 (1H, s), 7.58 2H, d), 7.05 (1H, d), 6.83 (2H, d), 6.51 (1H, d), 6.44 (1H, dd), 3.80 - 3.88 (4H, m), 3.72 (3H, s), 3.71 (3H, s), 3.20 (2H, t).

### Reference

### Example 21 1-[3-(4-Hydroxy-phenyl)-4,5-dihydro-pyrazol-1-yl]-2-(2,4,6-trimethoxy-phenyl)-ethanone (31)

4-(4,5-Dihydro-1*H*-pyrazol-3-yl)-phenol (2) (97 mg, 0.6 mmol) was treated with 2,4,6-trimethoxyphenyl acetic acid (113 mg, 0.5 mmol) using method D to give final compound 1-[3-(4-hydroxy-phenyl)-4,5-dihydro-pyrazol-1-yl]-2-(2,4,6-trimethoxy-phenyl)-ethanone (31) which was further purified by preparative HPLC. Yield: 5 mg (3%). LC/MS *t*ᵣ 3.95 min. MS(ES+) *m*/*z* 371 (M+H). ¹H NMP δ_{H} ppm (400 MHz, D₆-DMSO): 9.94 (1H, s), 7.59 (2H, d), 6.83 (2H, d), 6.21 (2H, s), 3.76 - 3.86 (7H, m), 3.69 (6H, s), 3.20 (2H, t).

### Reference

### Example 22 2-(2,5-Dimethoxy-phenyl)-1-[3-(4-hydroxy-phenyl)-4,5-dihydro-pyrazol-1-yl]-ethanone (32)

4-(4,5-Dihydro-1*H*-pyrazol-3-yl)-phenol (2) (97 mg, 0.6 mmol) was treated with 2,5-dimethoxyphenyl acetic acid (98 mg, 0.5 mmol) using method D to give final compound 2-(2,5-dimethoxy-phenyl)-1-[3-(4-hydroxy-phenyl)-4,5-dihydro-pyrazol-1-yl]-ethanone (32). Yield: 62 mg (36%). LC/MS *t*ᵣ 3.86 min. MS(ES+) *m*/*z* 341 (M+H). ¹H NMR δ_{H} ppm (400 MHz, D₆-DMSO): 9.95 (1H, s), 7.59 (2H, d), 6.73 - 6.88 (5H, m), 3.82 - 3.91 (4H, m), 3.68 (3H, s), 3.66 (3H, s), 3.21 (2H, t).

### Reference

### Example 23 2-(2,4-Dichloro-phenyl)-1-[3-(4-hydroxy-phenyl)-4,5-dihydro-pyrazol-1-yl]-ethanone (33)

4-(4,5-Dihydro-1*H*-pyrazol-3-yl)-phenol (2) (97 mg, 0.6 mmol) was treated with 2,4-dichlorophenyl acetic acid (102 mg, 0.5 mmol) using method D to give the final compound 2-(2,4-dichloro-phenyl)-1-[3-(4-hydroxy-phenyl)-4,5-dihydro-pyrazol-1-yl]-ethanone (33). Yield: 81mg (47%). LC/MS *t*ᵣ 4.39 min. MS(ES+) *m*/*z* 349 (M+H). ¹H NMR δ_{H} ppm (400 MHz, D₆-DMSO): 9.99 (1H, s), 7.58 - 7.62 (3H, m), 7.38 - 7.44 (2H, m), 6.83 (2H, d), 4.12 (2H, s), 3.87 (2H, t), 3.24 (2H, t).

### Reference

### Example 24 1-[3-(4-Hydroxy-phenyl)-4,5-dihydro-pyrazol-1-yl]-2-(2,3,4-trimethoxy-phenyl)-ethanone (34)

4-(4,5-Dihydro-1*H*-pyrazol-3-yl)-phenol (2) (97 mg, 0.6 mmol) was treated with 2,3,4-trimethoxyphenyl acetic acid (113 mg, 0.5 mmol) using method D to give final compound 1-[3-(4-hydroxy-phenyl)-4,5-dihydro-pyrazol-1-yl]-2-(2,3,4-trimethoxy-phenyl)-ethanone (34). Yield: 5 mg (3%). LC/MS *t*ᵣ 3.80 min. MS(ES+) *m*/*z* 371 (M+H). ¹H NMR δ_{H} ppm (400 MHz, D₆-DMSO): 10.03 (1H, s), 7.68 (2H, s), 6.89 - 6.99 (3H, m), 6.79 (1H, d), 3.90 - 3.98 (4H, m), 3.83 (3H, s), 3.80 (6H, s), 3.30 (2H, t).

### Reference

### Example 25 2-(2,3-Dimethoxy-phenyl)-1-[3-(4-hydroxy-phenyl)-4,5-dihydro-pyrazol-1-yl]-ethanone (35)

4-(4,5-Dihydro-1*H*-pyrazol-3-yl)-phenol (2) (97 mg, 0.6 mmol) was treated with 2,3-dimethoxyphenyl acetic acid (98 mg, 0.5 mmol) using method D. The product was further purified by recrystalisation (50% MeCN / H₂O) to give the final compound 2-(2,3-Dimethoxy-phenyl)-1-[3-(4-hydroxy-phenyl)-4,5-dihydro-pyrazol-1-yl]-ethanone (35). Yield: 33 mg (19%). LC/MS *t*ᵣ 3.82 min. MS(ES+) *m*/*z* 341 (M+H). ¹H NMR δ_{H} ppm (400 MHz, D₆-DMSO): 9.97 (1H, s), 7.60 (2H, d), 6.90 - 7.00 (2H, m), 6.78 - 6.84 (3H, m), 3.96 (2H, s), 3.86 (2H, t), 3.78 (3H, s), 3.67 (3H, s), 3.23 (2H, t).

### Reference

### Example 26 1-[3-(4-Hydroxy-phenyl)-4,5-dihydro-pyrazol-1-yl]-2-(2-tritluoromethyl-phenyl)-ethanone (36)

4-(4,5-Dihydro-1*H*-pyrazol-3-yl)-phenol (2) (97 mg, 0.6 mmol) was treated with 2-trifluoromethyl phenyl acetic acid (102 mg, 0.5 mmol) using method D. The product was further purified by preparative HPLC to give the final compound 1-[3-(4-hydroxy-phenyl)-4,5-dihydro-pyrazol-1-yl]-2-(2-trifluoromethyl-phenyl)-ethanone (36). Yield: 7 mg (4%). LC/MS *t*ᵣ 4.17 min. MS(ES+) *m*/*z* 349 (M+H). ¹H. NMR δ_{H} ppm (400 MHz, D₆-DMSO): 9.99 (1H, s), 7.57 - 7.71 (4H, m), 7.48 (2H, d), 6.83 (2H, d), 4.20 (2H, s), 3.87 (2H, t), 3.25 (2H, t).

### Reference

### Example 27 1-[3-(4-Hydroxy-phenyl)-4,5-dihydro-pyrazol-1-yl]-2-(2-methoxy-phenyl)-ethanone (37)

4-(4,5-Dihydro-1*H*-pyrazol-3-yl)-phenol (2) (97 mg, 0.6 mmol) was treated with 2-methoxyphenyl acetic acid (83 mg, 0.5 mmol) using method D. The product was further purified by preparative HPLC to give final compound 1-[3-(4-hydroxy-phenyl)-4,5-dihydro-pyrazol-1-yl]-2-(2-methoxyphenyl)-ethanone (37). Yield: 5 mg (3%). LC/MS *t*ᵣ 3.88 min. MS(ES+) *m*/*z* 311 (M+H). ¹H NMR δ_{H} ppm (400 MHz, D₆-DMSO): 9.95 (1H, s), 7.58 (2H, d), 7.13 - 7.24 (2H, m), 6.81 - 6.96 (4H, m), 3.93 (2H, s), 3.86 (2H, t), 3.73 (3H, s), 3.21 (2H, t).

### Reference

### Example 28 3-(4-Hydroxy-phenyl)-4,5-dihydro-pyrazole-1-carboxylic acid (3,4-dimethoxy-phenyl)-amide (38)

A solution of 4-(4,5-Dihydro-1*H*-pyrazol-3-yl)-phenol (2) (97 mg, 0.6 mmol) and 3,4-dimethoxyphenyl isocyanate (107 mg, 0.6 mmol) in THF (1 mL) was stirred at RT for 18 h. The reaction mixture was quenched with H₂O (1 mL) and the resulting precipitate was filtered. The solid was washed with H₂O (2 mL), EtOAc (15 mL) and DCM (15 mL) to give final compound 3-(4-hydroxy-phenyl)-4,5-dihydro-pyrazole-1-carboxylic acid (3,4-dimethoxy-phenyl)-amide (38). Yield: 8 mg (4%). LC/MS *t*ᵣ 3.71 min. MS(ES+) *m*/*z* 342 (M+H). ¹H NMR δ_{H} ppm (400 MHz, D₆-DMSO): 8.67 (1H, s), 7.71 (2H, d), 7.32 (1H, d), 7.18 (1H, dd), (3H, m), 3.86 (2H, t), 3.72 (3H, s), 3.70 (3H, s), 3.21 (2H, t).

### Reference

### Example 29 1-[3-(4-Hydroxy-phenyl)-4,5-dihydro-pyrazol-1-yl]-2-(2-phenoxymethyl-phenyl)-ethanone (39)

2-Benzyloxyphenyl acetic acid (75 mg, 0.31 mmol) was treated with oxalyl chloride (0.03 mL, 0.31 mmol) and 4-(4,5-dihydro-1*H*-pyrazol-3-yl)-phenol (2) (50 mg, 0.31 mmol) using Method G to give final compound 1-[3-(4-Hydroxy-phenyl)-4,5-dihydro-pyrazol-1-yl]-2-(2-phenoxymethyl-phenyl)-ethanone (39). Yield: 38 mg (32%). LC/MS *t*ᵣ 4.34 min (91%). MS(ES+) *m*/*z* 387 (M+H). ¹H NMR δ_{H} ppm (250 MHz, D₆-DMSO): 10.02 (1H, brs), 7.64 (2H, d), 7.49-7.45 (2H, m), 7.32-7.25 (5H, m), 7.10 (1H, d), 6.99-6.87 (3H, m), 5.14 (2H, s), 4.09 (2H, s), 3.87 (2H, t), 3.23 (2H, t).

### Reference

### Example 30 1-(3-(4-hydroxyphenyl)-4,5-dihydro-1H-pyrazol-1-yl)-2-(4-methoxy-3-methylphenyl)ethanone (40)

4-Methoxy-3-methylphenyl acetic acid (56 mg, 0.31 mmol) was treated with oxalyl chloride (0.03 mL), 0.31 mmol) and 4-(4,5-Dihydro-1*H*-pyrazol-3-yl)-phenol (2) (50 mg, 0.31 mmol) using Method G, except acetone dissolved the solid. The evaporated filtrate was triturated with EtOAc, filtered, washed with EtOAc and dried to give final compound 1-(3-(4-hydroxyphenyl)-4,5-dihydro-1H-pyrazol-1-yl)-2-(4-methoxy-3-methylphenyl)ethanone (40). Yield: 19 mg (19%). LC/MS *t*ᵣ 4.05 min (98%). MS(ES+) *m*/*z* 325 (M+H). ¹H NMR δ_{H} ppm (250 MHz, D₆-DMSO): 9.95 (1H, brs), 7.61 (2H, d), 7.09-7.06 (2H, m), 6.85-6.82 (3H, m), 3.84-3.72 (7H, m), 3.19 (2H, t), 2.09 (3H, s).

### Reference

### Example 31 2-(2-Chloro-5-fluoro-phenyl)-1-[3-(4-hydroxy-phenyl)-4,5-dihydro-pyrazol-1-yl]-ethanone (41)

2-Chloro-5-fluorophenyl acetic acid (120 mg, 0.62 mmol) was treated with oxalyl chloride (0.06 mL, 0.62 mmol) and 4-(4,5-dihydro-1*H*-pyrazol-3-yl)-phenol (2) (100 mg, 0.62 mmol) using Method G to give final compound 2-(2-chloro-5-fluoro-phenyl)-1-[3-(4-hydroxy-phenyl)-4,5-dihydro-pyrazol-1-yl]-ethanone (41). Yield: 83 mg (40%). LC/MS *t*ᵣ 4.14 min (98%). MS(ES+) *m*/*z* 333 (M+H). ¹H NMR δ_{H} ppm (250 MHz, D₆-DMSO): 10.01 (1H, brs), 7.61 (2H, d), 7.49-7.44 (1H, m), 7.31-7.26 (1H, m), 7.19-7.11 (1H, m), 6.83 (2H, d), 4.13 (2H, s), 3.87 (2H, t), 3.24 (2H, t).

### Reference

### Example 32 2-(2-Chloro-5-trifluoromethyl-phenyl)-1-[3-(4-hydroxy-phenyl)-4,5-dihydro-pyrazol-1-yl]-ethanone (42)

2-Chloro-5-(trifluoromethyl)phenyl acetic acid (74 mg, 0.31 mmol) was treated with oxalyl chloride (0.03 mL), 0.31 mmol) and 4-(4,5-dihydro-1*H*-pyrazol-3-yl)-phenol (2) (50 mg, 0.31 mmol) using Method G to give final compound 2-(2-chloro-5-trifluoromethyl-phenyl)-1-[3-(4-hydroxy-phenyl)-4,5-dihydro-pyrazol-1-yl]-ethanone (42). Yield: 5 mg (4%). LC/MS *t*ᵣ 4.42 min (100%). MS(ES+) *m*/*z* 383 (M+H). ¹H NMR δ_{H} ppm (250 MHz, D₆-DMSO): 9.99 (1H, brs), 7.84 (1H, s), 7.67-7.59 (4H, m), 6.83 (2H, d), 4.25 (2H, s), 3.88 (2H, t), 3.23 (2H, t).

### Reference

### Example 33 2-(2,5-dihydroxyphenyl)-1-(3-(4-hydroxyphenyl)-4,5-dihydro-1H-pyrazol-1-yl)ethanone (45)

To a solution of homogentisic acid (0.2 g, 1.19 mmol) in 2M NaOH (2.38 mL) cooled externally was added benzoyl chloride (0.28 mL, 2.44 mmol) dropwise over 2 min. The reaction was stirred for 1 h at RT, then H₂O (5 mL) was added and the resulting precipitate was filtered and washed with H₂O (5 mL). The solid was dissolved in 0.2 M HCl (10 mL) and DCM (30 mL) and the organic phase was separated then washed with brine (1 x 20 mL), dried (MgSO₄), filtered and evaporated. The crude product was purified by dry flash chromatography (40% EtOAc in heptane) to give (2,5-dihydroxy-phenyl)-acetic acid dibenzoate (43) as an off-white solid. Yield: 0.14 g (31%). LC/MS *t*ᵣ 2.15 min (100%). MS(ES+) *m*/*z* 399 (M+Na).

(2,5-Dihydroxy-phenyl)-acetic acid dibenzoate (43) (0.13 mg, 0.35 mmol) was treated with oxalyl chloride (0.07 mL, 0.7 mmol) and 4-(4,5-dihydro-1*H*-pyrazol-3-yl)-phenol (2) (50 mg, 0.31 mmol) using Method G to give 2-(2,5-dihydroxy-phenyl)-1-[3-(4-hydroxy-phenyl)-4,5-dihydro-pyrazol-1-yl]-ethanone dibenzoate (44). Yield: 36 mg (23%). LC/MS *t*ᵣ 2.22 min (100%). MS(ES+) *m*/*z* 521 (M+H).

To suspension of 2-(2,5-dihydroxy-phenyl)-1-[3-(4-hydroxy-phenyl)-4,5-dihydro-pyrazol-1-yl]-ethanone dibenzoate (44) (30 mg, 0.058 mmol) in MeOH (1 mL) was added hydrazine hydrate (3.6 µL, 0.12 mmol) and the reaction mixture stirred for 3 h at RT. After this time, THF (1 mL), NMP (1 mL) and further hydrazine hydrate (0.014 mL), 0.46 mmol) were added over 4 days. The organic solvents were partially evaporated then H₂O (15 mL) and 2M HCl (2 mL) were added and the precipitated solid was filtered, washed with H₂O (3 x 2 mL), acetone (2 x 2 mL) and dried to give the final compound 2-(2,5-dihydroxy-phenyl)-1-[3-(4-hydroxy-phenyl)-4,5-dihydro-pyrazol-1-yl]-ethanone (45). Yield: 5 mg (28%). LC/MS *t*ᵣ 3.17 min MS(ES+) *m*/*z* 313 (M+H). ¹H NMR δ_{H} ppm (250 MHz, D₆-DMSO): 9.96 (1H, brs), 8.64 (1H, brs), 8.56 (1H, br. s), 7.59 (2H, d), 6.82 (2H, d), 6.58-6.41 (3H, m), 3.83 (2H, s).

### Reference

### Example 35 2-(5-Bromo-2-methoxy-phenyl)-1-[3-(4-hydroxy-phenyl)-4,5-dihydro-pyrazol-1-yl]-ethanone (46)

5-Bromo-2-methoxyphenyl acetic acid (150 mg, 0.62 mmol) was treated with oxalyl chloride (0.06 mL, 0.62 mmol) and 4-(4,5-dihydro-1*H*-pyrazol-3-yl)-phenol (2) (100 mg, 0.62 mmol) using Method G to give final compound 2-(5-bromo-2-methoxy-phenyl)-1-[3-(4-hydroxy-phenyl)-4,5-dihydro-pyrazol-1-yl]-ethanone (46). Yield: 58 mg (24%). LC/MS *t*ᵣ 4.21 min (100%). MS(ES+) *m*/*z* 389/391 (M+H). ¹H NMR δ_{H} ppm (250 MHz, D₆-DMSO): 9.96 (1H, s), 7.59 (2H, d), 7.38 (2H, m), 6.93 (1H, d), 6.83 (2H, d), 3.94 (2H, s), 3.86 (2H, t), 3.73 (3H, s), 3.22 (2H, t).

### Reference

### Example 36 2-(4-Fluoro-2-trifluoromethyl-phenyl)-1-[3-(4-hydroxy-phenyl)-4,5-dihydro-pyrazol-1-yl]-ethanone (47)

4-Fluoro-2-(trifluoromethyl)phenyl acetic acid (69 mg, 0.31 mmol) was treated with oxalyl chloride (0.03 mL, 0.31 mmol) and 4-(4,5-dihydro-1*H*-pyrazol-3-yl)-phenol (2) (50 mg, 0.31 mmol) using Method G to give final compound 2-(4-fluoro-2-trifluoromethyl-phenyl)-1-[3-(4-hydroxy-phenyl)-4,5-dihydro-pyrazol-1-yl]-ethanone (47). Yield: 25 mg (23%). LC/MS *t*ᵣ 4.25 min (100%). MS(ES+) *m*/*z* 367 (M+H). ¹H NMR δ_{H} ppm (250 MHz, D₆-DMSO): 9.98 (1H, brs), 7.55 (5H, m), 6.84 (2H, d), 4.20 (2H, s), 3.87 (2H, t), 3.24 (2H, t).

### Example 37 2-(4-Chloro-3-fluoro-phenyl)-1-[3-(4-hydroxy-phenyl)-4,5-dihydro-pyrazol-1-yl]-ethanone (48)

4-Chloro-3-fluorophenyl acetic acid (58 mg, 0.31 mmol) was treated with oxalyl chloride (0.03 mL, 0.31 mmol) and 4-(4,5-dihydro-1*H*-pyrazol-3-yl)-phenol (2) (50 mg, 0.31 mmol) using Method G to give final compound 2-(4-chloro-3-fluoro-phenyl)-1-[3-(4-hydroxy-phenyl)-4,5-dihydro-pyrazol-1-yl]-ethanone (48). Yield: 32 mg (32%). LC/MS *t*ᵣ 4.20 min (100%). MS(ES+) *m*/*z* 333 (M+H). ¹H NMR δ_{H} ppm (250 MHz, D₆-DMSO): 9.99 (1H, brs), 7.62 (2H, d), 7.50 (1H, t), 7.33 (1H, d), 7.16 (1H, d), 6.84 (2H, d), 4.01 (2H, s), 3.85 (2H, t), 3.22 (2H, t).

### Reference

### Example 38 2-(2,5-Dimethyl-phenyl)-1-[3-(4-hydroxy-phenyl)-4,5-dihydro-pyrazol-1-yl]-ethanone (49)

2,5-Dimethylphenyl acetic acid (100 mg, 0.62 mmol) was treated with oxalyl chloride (0.06 mL, 0.62 mmol) and 4-(4,5-dihydro-1*H*-pyrazol-3-yl)-phenol (2) (100 mg, 0.62 mmol) using Method G to give final compound 2-(2,5-dimethyl-phenyl)-1-[3-(4-hydroxy-phenyl)-4,5-dihydro-pyrazol-1-yl]-ethanone (49). Yield: 42 mg (22%). LC/MS *t*ᵣ 4.23 min (85%). MS(ES+) *m*/*z* 309 (M+H). ¹H NMR δ_{H} ppm (250 MHz, D₆-DMSO): 7.60 (2H, d), 7.03 - 6.90 (3H, m), 3.93 (2H, s), 3.85 (2H, t), 3.21 (2H, t), 2.21 (6H, s).

### Reference

### Example 39 2-(2-Bromo-5-chloro-phenyl)-1-[3-(4-hydroxy-phenyl)-4,5-dihydro-pyrazol-1-yl]-ethanone (50)

2-Bromo-5-chlorophenyl acetic acid (150 mg, 0.62 mmol) was treated with oxalyl chloride (0.06 mL, 0.62 mmol) and 4-(4,5-dihydro-1*H*-pyrazol-3-yl)-phenol (2) (100 mg, 0.62 mmol) using Method G to give final compound 2-(2-bromo-5-chloro-phenyl)-1-[3-(4-hydroxy-phenyl)-4,5-dihydro-pyrazol-1-yl]-ethanone (50). Yield: 120 mg (50%). LC/MS *t*ᵣ 4.39 min (100%). MS(ES+) *m*/*z* 393/395 (M+H). ¹H NMR δ_{H} ppm (250 MHz, D₆-DMSO): 10.00 (1H, brs), 7.61 (3H, m), 7.50 (1H, s), 7.27 (1H, d), 6.84 (2H, d), 4.15 (2H, s), 3.88 (2H, t), 3.24 (2H, t).

### Reference

### Example 40 2-(5-Chloro-2-trifluoromethyl-phenyl)-1-[3-(4-hydroxy-phenyl)-4,5-dihydro-pyrazol-1-yl]-ethanone (51)

5-Chloro-2-(trifluoromethyl)phenyl acetic acid (74 mg, 0.31 mmol) was treated with oxalyl chloride (0.03 mL, 0.31 mmol) and 4-(4,5-dihydro-1*H*-pyrazol-3-yl)-phenol (2) (50 mg, 0.31 mmol) using Method G to give final compound 2-(5-chloro-2-trifluoromethyl-phenyl)-1-[3-(4-hydroxy-phenyl)-4,5-dihydro-pyrazol-1-yl]-ethanone (51). Yield: 35 mg (29%). LC/MS *t*ᵣ 4.44 min (95%). MS(ES+) *m*/*z* 383 (M+H). ¹H NMR δ_{H} ppm (250 MHz, D₆-DMSO): 9.98 (1H, brs), 7.72 (1H, d), 7.58 (4H, m), 6.84 (2H, d), 4.23 (2H, s), 3.87 (2H, t), 3.25 (2H, t).

### Example 41 2-(2,4-Dichloro-5-fluoro-phenyl)-1-[3-(4-hydroxy-phenyl)-4,5-dihydro-pyrazol-1-yl]-ethanone (52)

2,4-Dichloro-5-fluorophenyl acetic acid (69 mg, 0.31 mmol) was treated with oxalyl chloride (0.03 mL, 0.31 mmol) and 4-(4,5-dihydro-1*H*-pyrazol-3-yl)-phenol (2) (50 mg, 0.31 mmol) using Method G to give final compound 2-(2,4-dichloro-5-fluoro-phenyl)-1-[3-(4-hydroxy-phenyl)-4,5-dihydro-pyrazol-1-yl]-ethanone (52). Yield: 20 mg (18%). LC/MS *t*ᵣ 4.43 min (93%). MS(ES+) *m*/*z* 367/369 (M+H). ¹H NMR δ_{H} ppm (250 MHz, D₆-DMSO): 10.00 (1H, brs), 7.79 (1H, d), 7.63-7.51 (3H, m), 6.83 (2H, d), 4.14 (2H, s), 3.87 (2H, t), 3.25 (2H, t).

### Reference

### Example 42 1-[3-(4-Hydroxy-phenyl)-4,5-dihydro-pyrazol-1-yl]-2-(2,4,6-trimethyl-phenyl)-ethanone (53)

Mesityl acetic acid (110 mg, 0.62 mmol) was treated with oxalyl chloride (0.06 mL, 0.62 mmol) and 4-(4,5-dihydro-1*H*-pyrazol-3-yl)-phenol (2) (100 mg, 0.62 mmol) using Method G to give final compound 1-[3-(4-hydroxy-phenyl)-4,5-dihydro-pyrazol-1-yl]-2-(2,4,6-trimethyl-phenyl)-ethanone (53). Yield: 94 mg (47%). LC/MS *t*ᵣ 4.41 min (100%). MS(ES+) *m*/*z* 323 (M+H). ¹H NMR δ_{H} ppm (250 MHz, D₆-DMSO): 10.00 (1H, brs), 7.62 (2H, d), 6.84 (2H, d), 6.79 (2H, s), 3.97 (2H, s), 3.86 (2H, t), 3.22 (2H, t), 2.17 (9H, m).

### Example 43 2-(2-Bromo-4,5-dimethoxy-phenyl)-1-[3-(4-hydroxy-phenyl)-4,5-dihydro-pyrazol-1-yl]-ethanone (54)

2-Bromo-4,5-dimethoxyphenyl acetic acid (85 mg, 0.31 mmol) was treated with oxalyl chloride (0.03 mL, 0.31 mmol) and 4-(4,5-dihydro-1*H*-pyrazol-3-yl)-phenol (2) (50 mg, 0.31 mmol) using Method G to give final compound 2-(2-bromo-4,5-dimethoxy-phenyl)-1-[3-(4-hydroxy-phenyl)-4,5-dihydro-pyrazol-1-yl]-ethanone (54). Yield: 23 mg (18%). LC/MS *t*ᵣ 3.98 min (95%). MS(ES+) *m*/*z* 419/421 (M+H). ¹H NMR δ_{H} ppm (250 MHz, D₆-DMSO): 9.97 (1H, brs), 7.62 (2H, d), 7.10 (1H, s), 7.01 (1H, s), 6.83 (2H, d), 4.04 (2H, s), 3.86 (2H, t), 3.74 (3H, s), 3.70 (3H, s), 3.22 (2H, t).

### Example 44 1-(4-{1-[2-(2-Bromo-4,5-dimethoxy-phenyl)-acetyl]-4,5-dihydro-1H-pyrazol-3-yl}-phenyl)-3-methyl-urea (63)

4-nitroacetophenone (12.26 g, 0.074 mol) was treated with paraformaldehyde (6.0 g, 0.2 mol) and dimethylamine hydrochloride (12.07 g, 0.15 mol) using method A to give 3-dimethylamino-1-(4-nitro-phenyl)-propan-1-one-hydrochloride salt (12) as an off-white solid. Yield: 13.03 g (68%).

3-Dimethylamino-1-(4-nitro-phenyl)-propan-1-one-hydrochloride salt (12) (13.0 g, 0.05 mol) was treated with hydrazine hydrate (19.50 mL, 0.402 mol) using method B to give 3-(4-nitro-phenyl)-4,5-dihydro-1*H*-pyrazole (13) as an orange solid. Yield: 7.87 g (82%). LC/MS *t*ᵣ 1.25 min (96%). MS(ES+) *m*/*z* 192 (M+H).

To a stirred suspension of 3-(4-nitro-phenyl)-4,5-dihydro-1*H*-pyrazole (13) (7.37 g, 0.039 mol) in THF (100 mL) was added boc anhydride (9.28 g, 0.043 mol) in THF (50 mL), then DMAP (0.11 g). The mixture was heated to 80 °C for 2 h then cooled to RT and H₂O (140 mL) was added. The precipitated solid was filtered, washed with H₂O (1 x 75 mL then 1 x 150 mL) and dried to give 3-(4-nitro-phenyl)-4,5-dihydro-pyrazole-1-carboxylic acid *tert*-butyl ester (55). Yield: 8.98 g (80%). LC/MS *t*ᵣ 2.06 min (100%). MS(ES+) *m*/*z* 236 (M+H-56).

A suspension of 3-(4-nitro-phenyl)-4,5-dihydro-pyrazole-1-carboxylic acid *tert*-butyl ester (55) (8.90 g, 0.031 mmol) and 10% Pd/C (0.45 g, 50% wt. water) in MeOH (180 mL) under an atmosphere of hydrogen gas was stirred at RT for 17 h. The reaction mixture was filtered through celite and the filtrate was evaporated to give 3-(4-amino-phenyl)-4,5-dihydro-pyrazole-1-carboxylic acid *tert*-butyl ester (56) as a beige solid. Yield: 7.49 g (94%). LC/MS *t*ᵣ 1.60 min (100%). MS(ES+) *m*/*z* 206 (M+H-56).

A solution of aniline 3-(4-amino-phenyl)-4,5-dihydro-pyrazole-1-carboxylic acid *tert*-butyl ester (56) (916 mg, 3.51 mmol) and methyl isocyanate (500 mg, 8.77 mmol) in acetonitrile (4 mL) was heated to 85 °C in a sealed tube for 18 h. The reaction mixture was cooled to RT and 2M NaOH (8 mL) added. The resulting precipitate was filtered to give 3-[4-(3-methyl-ureido)-phenyl]-4,5-dihydro-pyrazole-1-carboxylic acid tert-butyl ester (57). The filtrate was diluted with H₂O (5 mL) and EtOAc (25 mL) and the organic layer was separated and evaporated to give a further quantity of the compound. Yield: 1.39 g (quant). LC/MS *t*ᵣ 1.64 min. MS(ES+) *m*/*z* 659 (2M+Na). ¹H NMR δ_{H} ppm (400 MHz, D₆-DMSO): 8.89 (1H, s), 7.43 - 7.55 (4H, m), 6.24 (brs), 3.80 (2H, t), 3.15 (2H, t), 2.63 (3H, d).

TFA (1 mL) was added to a solution of 3-[4-(3-methyl-ureido)-phenyl]-4,5-dihydro-pyrazole-1-carboxylic acid tert-butyl ester (57) (712 mg, 2.24 mmol) in DCM (4 mL) and the resulting solution was stirred at RT for 18 h. The reaction mixture was evaporated to give 1-[4-(4,5-dihydro-1H-pyrazol-3-yl)-phenyl]-3-methyl-urea trifluoro-acetic acid salt (58). Yield: 743 mg (99%). LC/MS *t*ᵣ 0.59 min. MS(ES+) *m*/*z* 219 (M+H). ¹H NMR δ_{H} ppm (400 MHz, D₆-DMSO): 9.07 (1H, brs), 7.72 (2H, d), 7.54 (2H, d), 6.31 (1H, brs), 3.57 (2H, t), 3.41 (2H, t), 2.63 (3H, d).

2-Bromo-4,5-dimethoxy phenyl acetic acid (41 mg, 0.151 mmol) was treated with oxalyl chloride (0.028 mL, 0.3 mmol) and 1-[4-(4,5-Dihydro-1H-pyrazol-3-yl)-phenyl]-3-methyl-urea trifluoro-acetic acid (58) (33 mg, 0.151 mmol) using method F to give final compound 1-(4-{1-[2-(2-bromo-4,5-dimethoxy-phenyl)-acetyl]-4,5-dihydro-1*H*-pyrazol-3-yl}-phenyl)-3-methyl-urea (63). Yield: 10 mg (14%). LC/MS *t*ᵣ 4.05 min. MS(ES+) *m*/*z* 475 (M+H). ¹H NMR δ_{H} ppm (400 MHz, D₆-DMSO): 8.83 (1H, s), 7.65 (2H, d), 7.50 (2H, d), 7.10 (1H, s), 7.02 (1H, s), 6.12 (1H, t), 4.06 - 4.17 (4H, m), 3.88 (2H, t), 3.74 (3H, s), 3.70 (3H, s), 3.24 (2H, t), 2.63 (3H, d).

### Example 45 1-(4-{1-[2-(2-Chloro-4,5-dimethoxy-phenyl)-acetyl]-4,5-dihydro-1H-pyrazol-3-yl}-phenyl)-3-methyl-urea (64)

2-Chloro-4-5-dimethoxy phenyl acetic acid (44 mg, 0.190 mmol) was treated with oxalyl chloride (0.037 mL, 0.38 mmol) and (58) (70 mg, 0.211 mmol) using method F to give final compound 1-(4-{1-[2-(2-chloro-4,5-dimethoxy-phenyl)-acetyl]-4,5-dihydro-1*H*-pyrazol-3-yl}-phenyl)-3-methyl-urea (64). Yield: 12 mg (15%). LC/MS *t*ᵣ 3.79 min. MS(ES+) *m*/*z* 431 (M+H). ¹H NMR δ_{H} ppm (400 MHz, D₆-DMSO): 8.82 (1H, s), 7.64 (2H, d), 7.48 (2H, d), 7.00 (1H, s), 6.98 (1H, s), 6.11 (1H, t), 4.04 (2H, s), 3.87 (2H, t), 3.74 (3H, s), 3.70 (3H, s), 3.24 (2H, t), 2.63 (3H, d).

### Reference

### Example 46 1-(4-{1-[2-(4,5-Dimethoxy-biphenyl-2-yl)-acetyl]-4,5-dihydro-1H-pyrazol-3-yl}-phenyl)-3-methyl-urea (65)

A solution of 1-(4-{1-[2-(2-Bromo-4,5-dimethoxy-phenyl)-acetyl]-4,5-dihydro-1*H*-pyrazol-3-yl}-phenyl)-3-methyl-urea (63) (40 mg, 0.082 mmol) in MeOH (1 mL) and dioxane (2 mL) was degassed for 10 min. Pd Cl₂(dppf) (7 mg, 10 mol%), K₃PO₄ (52 mg, 0.245 mmol) and phenyl boronic acid (10 mg, 0.082 mmol) were added sequentially and the reaction mixture degassed for a further 5 min following each addition. The reaction mixture was then heated under a nitrogen atmosphere to 95 °C for 16 h. The reaction was then cooled to RT, filtered through celite and evaporated. The crude residue was purified by column chromatography (2% MeOH in EtOAc) to give final compound 1-(4-{1-[2-(4,5-dimethoxy-biphenyl-2-yl)-acetyl]-4,5-dihydro-1*H*-pyrazol-3-yl}-phenyl)-3-methyl-urea (65). Yield: 3.0 mg (8%). LC/MS *t*ᵣ 4.01 min. MS(ES+) *m*/*z* 473 (M+H). ¹H NMR δ_{H} ppm (400 MHz, CDCl₃): 7.18 - 7.47 (9H, s), 6.94 (1H, s), 6.72 (1H, s), 5.83 (1H, brs) 3.96 (2H, s), 3.81 (3H, s), 3.78 (3H, s), 3.07 (2H, t), 2.78 (3H, brs).

### Reference

### Example 47 1-Methyl-3-(4-{1-[2-(2,4,6-trimethyl-phenyl)-acetyl]-4,5-dihydro-1H-pyrazol-3-yl}-phenyl)-urea (69)

Mesityl acetic acid (53 mg, 0.30 mmol) was treated with oxalyl chloride (0.052 mL, 0.60 mmol) and 1-[4-(4,5-dihydro-1H-pyrazol-3-yl)-phenyl]-3-methyl-urea trifluoro-acetic acid salt (58) (100 mg, 0.30 mmol) using method F to give final compound 1-methyl-3-(4-{1-[2-(2,4,6-trimethylphenyl)-acetyl]-4,5-dihydro-1*H*-pyrazol-3-yl}-phenyl)-urea (69). Yield: 67 mg (61%). LC/MS *t*ᵣ 4.23 min. MS(ES+) *m*/*z* 379 (M+H). ¹H NMR δ_{H} ppm (400 MHz, D₆-DMSO): 8.82 (1H, s), 7.64 (2H, d), 7.48 (2H, d), 6.80 (2H, s), 6.07 (1H, t), 3.98 (2H, s), 3.88 (2H, t), 3.23 (2H, t), 2.63 (3H, d), 2.18 (9H, s).

### Example 48 N-(4-{1-[2-(2-Bromo-4,5-dimethoxy-phenyl)-acetyl]-4,5-dihydro-1H-pyrazol-3-yl}-phenyl)-methanesulfonamide (66)

4-Nitroacetophenone (12.26 g, 0.074 mol) was treated with paraformaldehyde (6.0 g, 0.2 mol) and dimethylamine hydrochloride (12.07 g, 0.15 mol) using method A to give 3-dimethylamino-1-(4-nitro-phenyl)-propan-1-one-hydrochloride salt (12) as an off-white solid. Yield: 13.03 g (68%). 3-Dimethylamino-1-(4-nitro-phenyl)-propan-1-one-hydrochloride (12) (13.0 g, 0.05 mol) was treated with hydrazine hydrate (19.50 mL, 0.402 mol) using method B to give 3-(4-nitro-phenyl)-4,5-dihydro-1*H*-pyrazole (13) as an orange solid. Yield: 7.87 g (82%). LC/MS *t*ᵣ 1.25 min (96%). MS(ES+) *m*/*z* 192 (M+H).

To a stirred suspension of 3-(4-nitro-phenyl)-4,5-dihydro-1*H*-pyrazole (13) (7.37 g, 0.039 mol) in THF (100 mL) was added boc anhydride (9.28 g, 0.043 mol) in THF (50 mL), then DMAP (0.11 g). The mixture was heated to 80 °C for 2 h then cooled to RT and H₂O (140 mL) was added. The precipitated solid was filtered, washed with H₂O (1 x 75 mL then 1 x 150 mL) and dried to give 3-(4-nitro-phenyl)-4,5-dihydro-pyrazole-1-carboxylic acid *tert*-butyl ester (55). Yield: 8.98 g (80%). LC/MS *t*ᵣ 2.06 min (100%). MS(ES+) *m*/*z* 236 (M+H-56).
A suspension of 3-(4-nitro-phenyl)-4,5-dihydro-pyrazole-1-carboxylic acid *tert*-butyl ester (55) (8.90 g, 0.031 mmol) and 10% Pd/C (0.45 g, 50% wt. water) in MeOH (180 mL) under an atmosphere of hydrogen gas was stirred at RT for 17 h. The reaction mixture was filtered through celite and the filtrate was evaporated to give 3-(4-amino-phenyl)-4,5-dihydro-pyrazole-1-carboxylic acid *tert*-butyl ester (56). Yield: 7.49 g (94%). LC/MS *t*ᵣ 1.60 min (100%). MS(ES+) *m*/*z* 206 (M+H-56).

To a solution of 3-(4-amino-phenyl)-4,5-dihydro-pyrazole-1-carboxylic acid *tert*-butyl ester (56) (1.50 g, 5.74 mmol) and DIPEA (1.19 mL, 6.89 mmol) in THF (12 mL) cooled externally was added methane sulphonyl chloride (0.49 mL, 6.31 mmol) in THF (3 mL) dropwise over 10 min. The reaction was stirred for 1.5 h at RT and then H₂O (75 mL) was added. The precipitated solid was filtered and washed with H₂O (1 x 25 mL), MTBE (2 x 20 mL), THF (3 x 25 mL) and dried to give 3-(4-methanesulfonylamino-phenyl)-4,5-dihydro-pyrazole-1-carboxylic acid *tert*-butyl ester (59). Yield: 0.58 g (30%). LC/MS *t*ᵣ 1.75 min (93%) MS(ES+) *m*/*z* 284 (M+H-56).
TFA (1 mL) was added to a suspension of 3-(4-methanesulfonylamino-phenyl)-4,5-dihydro-pyrazole-1-carboxylic acid *tert*-butyl ester (59) (0.25 g, 0.74 mmol) in DCM (4 mL) and the resulting suspension was stirred at RT for 75 min. The reaction mixture was evaporated and the residue triturated in EtOAc (4 mL), filtered, washed with EtOAc (2 x 4 mL) and dried to give *N*-[4-(4,5-dihydro-1*H*-pyrazol-3-yl)-phenyl]-methanesulfonamide TFA salt (60). Yield: 0.21 g (81%). LC/MS *t*ᵣ 0.28/0.55 min MS(ES+) *m*/*z* 240 (M+H).

To a stirred solution of 2-bromo-4,5-dimethoxyphenyl acetic acid (39 mg, 0.14 mmol) and DMF (1 drop) in THF (1 mL) was added oxalyl chloride (14.9 µL, 0.14 mmol). The resulting solution was stirred at RT for 1 h and then added to a mixture of *N*-[4-(4,5-dihydro-1*H*-pyrazol-3-yl)-phenyl]-methanesulfonamide (60) (50 mg, 0.14 mmol) and pyridine (23 µL, 0.28 mmol) in THF (1 mL) and NMP (0.5 mL). The suspension was stirred for 4 h after which time DCM (5 mL) was added and the organic phase was washed with H₂O (3 x 30 mL), dried (MgSO₄), filtered and evaporated to give a pale green solid. The crude residue was purified by dry flash chromatography (0 - 100% EtOAc in heptane) followed by preparative HPLC to give final compound *N*-(4-{1-[2-(2-bromo-4,5-dimethoxy-phenyl)-acetyl]-4,5-dihydro-1*H*-pyrazol-3-yl}-phenyl)-methanesulfonamide (66). Yield: 6 mg (9%). LC/MS *t*ᵣ 3.98 min (100%) MS(ES+) *m*/*z* 496, 498 (M+H). ¹H NMR δ_{H} ppm (250 MHz, D₆-DMSO): 7.77 (2H, d), 7.26 (2H, d), 7.04 (1H, s), 6.93 (2H, d), 6.63 (1H, brs), 4.20 (2H, s), 4.10 (2H, t), 3.86 (3H, s), 3.85 (3H, s), 3.24 (2H, t), 3.07 (3H, s).

### Example 49 N-(4-{1-[2-(2-Chloro-4,5-dimethoxy-phenyl)-acetyl]-4,5-dihydro-1H-pyrazol-3-yl}-phenyl)-methanesulfonamide (67)

To a solution of 2-chloro-4-5-dimethoxyphenyl acetic acid (3) (42 mg, 0.18 mmol) and DMF (1 drop) in THF (2 mL) was added oxalyl chloride (0.018 mL, 0.18 mmol). After 30 min further oxalyl chloride (0.022 mL, 0.22 mmol) was added and the solution stirred at RT for 1 h. The reaction mixture was then added to a suspension of Cs₂CO₃ (0.58 g, 1.8 mmol) and *N*-[4-(4,5-dihydro-1*H*-pyrazol-3-yl)-phenyl]-methanesulfonamide (60) (86 mg, 0.36 mmol) in THF (2 mL) and the resulting suspension stirred for 2 h at RT. The reaction mixture was poured into H₂O (20 mL) and extracted with DCM (5 mL). 6M HCl (4 mL) was added to the two-phase separation and the aqueous re-extracted with DCM (5 mL). The combined organic were washed with 0.4M HCl (2 x 32 mL), dried (MgSO₄), filtered and evaporated. The crude residue was purified by dry flash chromatography (0-100% EtOAc in heptane), then triturated in MTBE (1.5 mL), filtered, washed with MTBE (1.5 mL) and dried to give final compound *N*-(4-{1-[2-(2-chloro-4,5-dimethoxyphenyl)-acetyl]-4,5-dihydro-1*H*-pyrazol-3-yl}-phenyl)-methanesulfonamide (67) as a solid. Yield: 31 mg (38%). LC/MS *t*ᵣ 3.90 min (96%). MS(ES+) *m*/*z* 452 (M+H). ¹H NMR δ_{H} ppm (250 MHz, D₆-DMSO): 10.08 (1H, br. s), 7.74 (2H, d), 7.27 (2H, d), 7.00 (1H, s), 6.98 (1H, s), 4.05 (2H, s), 3.90 (2H, t), 3.74 (3H, s), 3.71 (3H, s), ∼3.26 (2H, t, overlap with water signal), 3.04 (3H, s).

### Reference

### Example 50 N-(4-{1-[2-(2,4,6-Trimethyl-phenyl)-acetyl]-4,5-dihydro-1H-pyrazol-3-yl}-phenyl)-methanesulfonamide (72)

To a solution of Mesityl acetic acid (32 mg, 0.18 mmol) and DMF (1 drop) in THF (2 mL) was added oxalyl chloride (0.018 mL, 0.18 mmol). After 30 min further oxalyl chloride (0.022 mL, 0.22 mmol) was added and the solution stirred at RT for 1 h. The reaction mixture was then added to a suspension of Cs₂CO₃ (0.58 g, 1.8 mmol) and *N*-[4-(4,5-dihydro-1*H*-pyrazol-3-yl)-phenyl]-methanesulfonamide (60) (86 mg, 0.36 mmol) in THF (2 mL) and the resulting suspension stirred for 2 h at RT. The reaction mixture was poured into H₂O (20 mL) and extracted with DCM (5 mL). 6M HCl (4 mL) was added to the two-phase separation and the aqueous re-extracted with DCM (5 mL). The combined organics were washed with a mixture of (1:1) water/brine (1 x 10 mL), dried (MgSO₄), filtered and evaporated. The crude residue was purified by dry flash chromatography (0-100% EtOAc in heptane) to give final compound *N*-(4-{1-[2-(2,4,6-trimethyl-phenyl)-acetyl]-4,5-dihydro-1*H*-pyrazol-3-yl}-phenyl)-methanesulfonamide (72) as a solid. Yield: 28 mg (38%). LC/MS *t*ᵣ 4.43 min (95%). MS(ES+) *m*/*z* 400 (M+H). ¹H NMR δ_{H} ppm (250 MHz, D₆-DMSO): 10.08 (1H, br. s), 7.74 (2H, d), 7.27 (2H, d), 6.80 (2H, s), 3.99 (2H, s), 3.89 (2H, t), ∼3.26 (2H, t, overlap with water signal), 3.04 (3H, s), 2.19/2.17 (9H, s).

### Example 51 1-[3-(4-Amino-phenyl)-4,5-dihydro-pyrazol-1-yl]-2-(2-bromo-4,5-dimethoxy-phenyl)-ethanone (68)

Conc. HCl (0.5 mL) was added to a solution of (61) (0.5 g, 1.40 mmol) in MeOH (5 ml). Acetonitrile (10 mL), conc. HCl (1 mL) and H₂O (5 mL) were added and at intervals TFA (1 mL and 2 mL) was also added to the stirred mixture over 2 days. The solvents were evaporated to give *N*-[4-(4,5-dihydro-1*H*-pyrazol-3-yl)-phenyl]-2,2,2-trifluoro-acetamide TFA salt (62). Yield: 0.41 g (79%). LC/MS *t*ᵣ 1.07 min (100%) MS(ES+) *m*/*z* 258 (M+H).

To a stirred solution of 2-bromo-4,5-dimethoxyphenyl acetic acid (94 mg, 0.34 mmol) and DMF (1 drop) in THF (1 mL) was added oxalyl chloride (35.6 µL, 0.41 mmol). The solution was stirred for 75 min at RT and then added to a mixture of *N*-[4-(4,5-dihydro-1*H*-pyrazol-3-yl)-phenyl]-2,2,2-trifluoro-acetamide TFA salt (62) (62) (100 mg, 0.27 mmol) in THF (1 mL). Triethylamine (0.16 mL, 1.16 mmol) was then added and the suspension stirred for 1 h. DCM (5 mL) and H₂O (30 mL) were added and the separated aqueous phase re-extracted with DCM (1 x 5 mL). The combined organics were washed with H₂O (1 x 30 mL), a mixture of H₂O / brine (3:2) (2 x 50 mL), then dried (MgSO₄), filtered and evaporated to give the crude residue. This was stirred in DCM (4 mL) and 2N NaOH (4 mL) for 8 h, then further DCM (5 mL) and H₂O (10 mL) were added to the reaction mixture. The aqueous phase was separated and re-extracted into DCM (2 x 5 mL) and the combined organics were washed with brine (1 x 10 mL), then dried (MgSO₄), filtered and evaporated. The residue was triturated in MeOH and MTBE (2 mL), filtered and dried to give final compound 1-[3-(4-amino-phenyl)-4,5-dihydro-pyrazol-1-yl]-2-(2-bromo-4,5-dimethoxy-phenyl)-ethanone (68). Yield: 10 mg (7%). LC/MS *t*ᵣ 3.98 min (100%) MS(ES+) *m*/*z* 418, 420 (M+H). ¹H NMR δ_{H} ppm (250 MHz, D₆-DMSO): 7.46 (2H, d), 7.09 (1H, s), 7.01 (1H, s), 5.65 (2H, brs), 4.02 (2H, s), 3.83 (2H, t), 3.74 (3H, s), 3.70 (3H, s), 3.18 (2H, t).

### Example 52 (E)-N-(4-{1-[2-(2-Bromo-4,5-dimethoxy-phenyl)-acetyl]-4,5-dihydro-1H-pyrazol-3-yl}-phenyl)-3-methoxy-acrylamide (71)

A solution of propiolic acid (6 µL, 0.1 mmol), 1-[3-(4-amino-phenyl)-4,5-dihydro-pyrazol-1-yl]-2-(2-bromo-4,5-dimethoxy-phenyl)-ethanone (68) (42 mg, 0.1 mmol) and EDC (19 mg, 0.1 mmol) in DMF (0.8 mL) was stirred at RT for 18 h. The reaction mixture was diluted with H₂O (4 mL) and 2M HCl (2 mL) and extracted with EtOAc (2 x 2 mL). The combined organic phases were washed with water (2 mL), dried (MgSO₄), filtered and evaporated to give the crude residue. Attempted crystallisation from warm MeOH was unsuccessful so the crude residue was purified by column chromatography (50-100% EtOAc in heptane) to give final compound (*E*)*-N-*(4-{1-[2-(2-bromo-4,5-dimethoxy-phenyl)-acetyl]-4,5-dihydro-1*H*-pyrazol-3-yl}-phenyl)-3-methoxy-acrylamide (71). Yield: 4 mg (8%). LC/MS *t*ᵣ 4.22 min. MS(ES+) *m*/*z* 502/504 (M+H). ¹H NMR δ_{H} ppm (250 MHz, CDCl₃): 7.5 - 7.7 (5H, m), 7.12 (1H, bs), 6.97 (1H, s), 6.86 (1H, s), 5.25 (1H, d, J = 12 Hz), 4.13 (2H, s), 4.00 (2H, t), 3.78 (3H, s), 3.77 (3H, s), 3.67 (3H, s), 3.16 (2H, t).

### Example 53 1-[3-(4-Amino-phenyl)-4,5-dihydro-pyrazol-1-yl]-2-(2-chloro-4,5-dimethoxy-phenyl)-ethanone (73)

To a solution of 2-chloro-4-5-dimethoxyphenyl acetic acid (3) (7.99 g, 34.6 mmol) and DMF (5 drops) in DCM (30 mL) at 0 °C was added oxalyl chloride (9.06 mL, 103.8 mmol) in DCM (10 mL) over 12 min where a gas was evolved. The resulting solution was stirred for 10 min at 0 °C then allowed to warm to RT. After 135 min the solvent and excess oxalyl chloride were evaporated. The residue was re-dissolved in DCM (40 mL) and added dropwise over 20 min to a cooled stirred suspension of 3-(4-nitro-phenyl)-4,5-dihydro-1*H*-pyrazol (13) (6.62 g, 34.6 mmol) and pyridine (5.60 mL, 69.2 mL) in DCM (120 mL). After a further 10 min, the reaction mixture was warmed to RT and stirred for 1 h. The reaction mixture was washed with H₂O (2 x 300 mL) and the organic layer was dried (MgSO₄), filtered and evaporated. The crude residue was purified by dry flash chromatography (0 - 75% EtOAc / heptane), then triturated in 50% MTBE / heptane (36 mL), filtered and washed with 50% MTBE / heptane (20 mL) then heptane (40 mL) and dried to give 2-(2-chloro-4,5-dimethoxy-phenyl)-1-[3-(4-nitro-phenyl)-4,5-dihydro-pyrazol-1-yl]-ethanone (72). Yield: 11.96 g (86%). LC/MS *t*ᵣ 2.16 min MS(ES+) m/z 404/406 (M+H).

A suspension of 2-(2-chloro-4,5-dimethoxy-phenyl)-1-[3-(4-nitro-phenyl)-4,5-dihydro-pyrazol-1-yl]-ethanone (72) (11.96 g, 29.6 mmol) and 10% Pd / C (0.6 g, 50% wt. water) in acetonitrile (240 mL) under an atmosphere of hydrogen gas was stirred at RT for 17.5 h. 6M HCl (12.5 mL) was added in parts followed by MeOH (100 mL). The reaction mixture was filtered through celite, washing with MeOH (2 x 50 mL) and the filtrate was partially evaporated precipitating a solid on standing. H₂O (150 mL) and 2M NaOH (50 mL) was added to the precipitate and stirred. The solid was filtered and washed with 50% aqueous acetonitrile (2 x 50 mL) then H₂O (2 x 50 mL) and dried to give 1-[3-(4-amino-phenyl)-4,5-dihydro-pyrazol-1-yl]-2-(2-chloro-4,5-dimethoxy-phenyl)-ethanone (73) as a solid. Yield: 10.01 g (90%). LC/MS *t*ᵣ 3.91 min. MS(ES+) *m*/*z* 374/376 (M+H). ¹H NMR δ_{H} ppm (250 MHz, D₆-DMSO): 7.45 (2H, d), 7.00 (1H, s), 6.97 (1H, s), 6.58 (2H, d), 5.65 (2H, s), 4.01 (2H, s), 3.82 (2H, t), 3.74 (3H, s), 3.70 (3H, s), 3.17 (2H, t).

### Example 54 (4-{1-[2-(2-Chloro-4,5-dimethoxy-phenyl)-acetyl]-4,5-dihydro-1H-pyrazol-3-yl}-phenyl)-urea (75)

To a stirred suspension of 1-[3-(4-amino-phenyl)-4,5-dihydro-pyrazol-1-yl]-2-(2-chloro-4,5-dimethoxy-phenyl)-ethanone (73) (0.15 g, 0.40 mmol) in acetonitrile (3 mL) was added 4-nitrophenyl chloroformate (89 mg, 0.44 mmol) in parts over 1 min at RT. After stirring for 25 h at RT, acetonitrile (6 mL) and 1.2M HCl was added. The resulting precipitate was filtered, washed with H₂O (10 mL) and acetonitrile (10 mL) then dried to give crude (4-{1-[2-(2-chloro-4,5-dimethoxy-phenyl)-acetyl]-4,5-dihydro-1*H*-pyrazol-3-yl}-phenyl)-carbamic acid 4-nitro-phenyl ester (74). Yield: 100 mg (45%).

To a stirred solution of (4-{1-[2-(2-chloro-4,5-dimethoxy-phenyl)-acetyl]-4,5-dihydro-1*H*-pyrazol-3-yl}-phenyl)-carbamic acid 4-nitro-phenyl ester (74) (50 mg, 0.093 mmol) in NMP (1 mL) was added a solution of 7M ammonia in MeOH (0.25 mL, 1.75 mmol) at RT. The solution was stirred for 45 min at RT and then added to DCM (5 mL) and H₂O (30 mL). The aqueous layer was re-extracted with DCM (5 mL) and the organics were combined. The resulting precipitate was filtered and washed with DCM (1 x 2 mL; 1 x 4 mL) then dried to give the final compound (4-{1-[2-(2-chloro-4,5-dimethoxy-phenyl)-acetyl]-4,5-dihydro-1*H*-pyrazol-3-yl}-phenyl)-urea (75) as a solid. Yield: 23 mg (59%). LC/MS *t*ᵣ 3.63 min. MS(ES+) *m*/*z* 417/419 (M+H). ¹H NMR δ_{H} ppm (250 MHz, D₆-DMSO): 8.81 (1H, brs), 7.64 (2H, d), 7.48 (2H, d), 7.00 (1H, s), 6.98 (1H, s), 5.96 (2H, brs), 4.04 (2H, s), 3.87 (2H, t), 3.74 (3H, s), 3.70 (3H, s), 3.23 (2H, obs).

### Example 55 3-(4-{1-[2-(2-Chloro-4,5-dimethoxy-phenyl)-acetyl]-4,5-dihydro-1H-pyrazol-3-yl}-phenyl)-1,1-dimethyl-urea (76)

To a stirred solution of (4-{1-[2-(2-chloro-4,5-dimethoxy-phenyl)-acetyl]-4,5-dihydro-1*H*-pyrazol-3-yl}-phenyl)-carbamic acid 4-nitro-phenyl ester (74) (50 mg, 0.093 mmol) and dimethylamine hydrochloride (38 mg, 0.47 mmol) in NMP (1 mL) at RT was added Et₃N (64.8 µL, 0.47 mmol). The solution was stirred for 45 min at RT and then added to DCM (5 mL) and H₂O (40 mL). The aqueous layer was re-extracted with DCM (2 x 5 mL) and the organics were combined, washed with H₂O (2 x 30 mL), dried (MgSO₄), filtered and evaporated to give the final compound 3-(4-{1-[2-(2-Chloro-4,5-dimethoxy-phenyl)-acetyl]-4,5-dihydro-1*H*-pyrazol-3-yl}-phenyl)-1,1-dimethyl-urea (76)as a solid. Yield: 17 mg (41%). LC/MS *t*ᵣ 3.97 min. MS(ES+) *m*/*z* 445/447 (M+H). ¹H NMR δ_{H} ppm (250 MHz, D₆-DMSO): 8.53 (1H, s), 7.67-7.56 (4H, m), 7.01 (1H, s), 6.98 (1H, s), 4.05 (2H, s), 3.88 (2H, t), 3.75 (3H, s), 3.71 (3H, s), 3.25 (2H, t), 2.93 (6H, s).

### Example 56 1-(4-{1-[2-(5-Chloro-2,4-dimethoxy-phenyl)-acetyl]-4,5-dihydro-1H-pyrazol-3-yl}-phenyl)-3-methyl-urea (78)

2,4-Dimethoxyphenyl acetic acid (196 mg, 1.0 mmol) in a solution of acetonitrile (2 mL) and H₂O (2 mL) was treated with oxone (615 mg, 1.0 mmol) and KCl (75 mg, 1 mmol). The reaction mixture was stirred for 1 h at RT. EtOAc (8 mL) was added to the reaction mixture and the organic layer separated and evaporated. The residue was redissolved in 2M NaOH (10 mL) and washed with EtOAc (2 x 15 mL). The aqueous layer was acidified with c.HCl and extracted into EtOAc (2 x 20 mL). The organic extracts were combined, dried (MgSO₄), filtered and evaporated to give (5-chloro-2,4-dimethoxy-phenyl)-acetic acid (77). Yield: 103 mg (45 %). ¹H NMR δ_{H} ppm (400 MHz, D₆-DMSO): 7.23 (1H, d), 6.75 (1H, d), 3.87 (3H, s), 3.83 (3H, s), 3.45 (2H, s).

(5-Chloro-2,4-dimethoxy-phenyl)-acetic acid (77) (76 mg, 0.3 mmol) was treated with oxalyl chloride (0.056 mL, 0.6 mmol) and (58) (99.6 mg, 0.3 mmol) using method F to give the final compound 1-(4-{1-[2-(5-Chloro-2,4-dimethoxy-phenyl)-acetyl]-4,5-dihydro-1*H*-pyrazol-3-yl}-phenyl)-3-methyl-urea (78). Yield: 9 mg (7 %). LC/MS *t*ᵣ 3.93 min. MS(ES+) *m*/*z* 431/433 (M+H). ¹H NMR δ_{H} ppm (400 MHz, D₆-DMSO): 8.89 (1H, s), 7.75 (2H, d), 7.58 (2H, d), 7.31 (1H, s), 6.81 (1H, s), 6.13 (1H, brq), 3.98 (6H, s), 3.86 (4H, m), 3.33 (2H, t), 2.70 (3H, d).

### Reference

### Example 57 1-(4-{1-[2-(2-Chloro-3,5-dimethoxy-phenyl)-acetyl]-4,5-dihydro-1H-pyrazol-3-yl}-phenyl)-3-methyl-urea (80)

3,5-Dimethoxy phenyl acetic acid (196 mg, 1.0 mmol) in a solution of acetonitrile (2 mL) and H₂O (2 mL) was treated with oxone (615 mg, 1.0 mmol) and KCl (75 mg, 1 mmol). The reaction mixture was stirred for 1 h at RT. EtOAc (8 mL) was added to the reaction mixture and the organic layer separated and evaporated. The residue was redissolved in 2M NaOH (10 mL) and washed with EtOAc (2 x 15 mL). The aqueous layer was acidified with c.HCl and extracted into EtOAc (2 x 20 mL). The organic extracts were combined, dried (MgSO₄), filtered and evaporated to give (2-chloro-3,5-dimethoxy-phenyl)-acetic acid (79). Yield: 176 mg (76 %). ¹H NMR δ_{H} ppm (400 MHz, D₆-DMSO): 6.66 (2H, m), 3.83 (3H, s), 3.74 (3H, s), 3.63 (2H, s).

(2-Chloro-3,5-dimethoxy-phenyl)-acetic acid (79) (76 mg, 0.3 mmol) was treated with oxalyl chloride (0.056 mL, 0.6 mmol) and 1-[4-(4,5-dihydro-1H-pyrazol-3-yl)-phenyl]-3-methyl-urea (58) (99.6 mg, 0.3 mmol) using method F. Purification of the crude residue by preparative HPLC gave final compound 1-(4-{1-[2-(2-chloro-3,5-dimethoxy-phenyl)-acetyl]-4,5-dihydro-1*H*-pyrazol-3-yl}-phenyl)-3-methyl-urea (80). Yield: 2 mg (2 %). LC/MS *t*ᵣ 3.91 min. MS(ES+) *m*/*z* 431/433 (M+H). ¹H NMR δ_{H} ppm (400 MHz, D₆-DMSO): 8.91 (1H, s), 7.62 (2H, d), 7.48 (2H, d), 6.59 (2H, m), 6.12 (1H, brq), 4.11 (2H, s), 3.81-3.97 (5H, m), 3.77 (3H, s), 3.24 (2H, t), 2.63 (3H, d).

### Reference

### Example 58 1-(4-{1-[2-(6-Chloro-2,3-dimethoxy-phenyl)-acetyl]4,5-dihydro-1H-pyrazol-3-yl}-phenyl)-3-methyl-urea (82)

2,3-Dimethoxy phenyl acetic acid (196 mg, 1.0 mmol) in a solution of acetonitrile (2 mL) and H₂O (2 mL) was treated with oxone (615 mg, 1.0 mmol) and KCl (75 mg, 1 mmol). The reaction mixture was stirred for 1 h at RT. EtOAc (8 mL) was added to the reaction mixture and the organic layer separated and evaporated. The residue was redissolved in 2M NaOH (10 mL) and washed with EtOAc (2 x 15 mL). The aqueous layer was acidified with c.HCl and extracted into EtOAc (2 x 20 mL). The organic extracts were combined, dried (MgSO₄), filtered and evaporated to give (6-chloro-2,3-dimethoxy-phenyl)-acetic acid (81). Yield: 149 mg (65 %). ¹H NMR δ_{H} ppm (400 MHz, D₆-DMSO): 7.16 (1H, d), 6.95 (1H, d), 3.79 (3H, s), 3.64 (3H, s), 3.63 (2H, s).

(6-Chloro-2,3-dimethoxy-phenyl)-acetic acid (81) (76 mg, 0.3 mmol) was treated with oxalyl chloride (0.056 mL, 0.6 mmol) and 1-[4-(4,5-dihydro-1H-pyrazol-3-yl)-phenyl]-3-methyl-urea (58) (99.6 mg, 0.3 mmol) using method F to give the final compound 1-(4-{1-[2-(6-Chloro-2,3-dimethoxy-phenyl)-acetyl]-4,5-dihydro-1*H*-pyrazol-3-yl}-phenyl)-3-methyl-urea (82). Yield: 9 mg (7 %). LC/MS *t*ᵣ 3.95 min. MS(ES+) *m*/*z* 431/433 (M+H). ¹H NMR δ_{H} ppm (400 MHz, D₆-DMSO): 8.81 (1H, s), 7.64 (2H, d), 7.51 (2H, d), 7.12 (1H, d), 6.99 (1H, d), 6.08 (1H, brq), 4.11 (2H, s), 3.79-3.96 (5H, m), 3.74 (3H, s), 3.23 (2H, t), 2.61 (3H, d).

### Reference

### Example 59 N-(4-{1-[2-(2,4-Dimethyl-phenyl)-acetyl]-4,5-dihydro-1H-pyrazol-3-yl}-phenyl)-methanesulfonamide (83)

To a solution of 2,4-dimethyl phenyl acetic acid (49.2 mg, 0.3 mmol) in DMF (2 mL) was added HATU (92.4 mg, 0.33 mmol), Et₃N (86 µL, 0.63 mmol) and *N*-[4-(4,5-dihydro-1*H*-pyrazol-3-yl)-phenyl]-methanesulfonamide TFA salt (60) (82.5 mg, 0.3 mmol). The resulting mixture was stirred at RT for 18 h, after which time H₂O (4 mL) was added and the resulting solution extracted with EtOAc (3 x 10 mL). The organic extracts were combined and washed with 1M HCl (10 mL), dried (MgSO₄), filtered and evaporated to give the final compound *N*-(4-{1-[2-(2,4-dimethyl-phenyl)-acetyl]-4,5-dihydro-1*H*-pyrazol-3-yl}-phenyl)methanesulfonamide (83). Yield: 12 mg (10%). LC/MS *t*ᵣ 4.20 min. MS(ES+) *m*/*z* 386 (M+H). ¹H NMR δ_{H} ppm (400 MHz, D₆-DMSO): 10.11 (1H, s), 7.72 (2H, d), 7.24 (2H, d), 7.08 (1H, d), 6.96 (1H, s), 6.82 (1H, d), 3.91 (2H, s), 3.88 (2H, t), 3.24 (2H, t), 3.04 (3H, s), 2.12 (3H, s), 2.10 (3H, s).

### Reference

### Example 60 N-(4-{1-[2-(2-Chloro-4,5-dimethyl-phenyl)-acetyl]-4,5-dihydro-1H-pyrazol-3-yl}-phenyl)-methanesulfonamide (87)

A solution of 1-chloro-3,4-dimethyl benzene (572 mg, 4.0 mmol) and methyl oxalyl chloride (539 mg, 4.4 mmol) in DCM was cooled to 0 °C and AlCl₃ (853 mg, 6.4 mmol) was added portionwise. The reaction was allowed to warm to RT and stirred for 2 h, after which time it was poured onto a mixture of ice and H₂O then extracted with DCM (3 x 50 mL). The combined organic layers were dried (MgSO₄), filtered and evaporated to give (2-chloro-4,5-dimethyl-phenyl)-oxo-acetic acid methyl ester (84). Yield: 830 mg (75 %). ¹H NMR δ_{H} ppm (400 MHz, D₆-DMSO): 7.59 (1H, s), 7.46 (1H, s), 3.86 (3H, s), 2.27 (3H, s), 2.21 (3H, s).

(2-Chloro-4,5-dimethyl-phenyl)-oxo-acetic acid methyl ester (84) (830 mg, 3.67mmol) and LiOH.H₂O (308 mg, 7.35 mmol) were stirred in THF (4 mL) and H₂O (2 mL) for 2 h. The solvent was evaporated and the residue dissolved in H₂O (10 mL). This was washed with EtOAc (20 mL) and the aqueous acidified to pH 2 with conc. HCl. This was extracted into EtOAc) (3 x 20 mL) and the combined organic layers were dried (MgSO₄), filtered and evaporated to give (2-chloro-4,5-dimethyl-phenyl)-oxo-acetic acid (85). Yield: 810 mg (100 %).

(2-Chloro-4,5-dimethyl-phenyl)-oxo-acetic acid (85) (810 mg, 3.82 mmol) was dissolved in 2-methoxyethanol (5 mL) and heated to 60°C. Hydrazine hydrate (0.48 mL, 9.55 mmol) was added dropwise and the reaction mixture stirred for 30 min at 60°C. KOH (534 mg, 9.55 mmol) was added portionwise and the temperature increased to 120 °C once the addition was complete. The reaction was stirred for 45 min before being cooled to RT. H₂O (15 mL) was added and the solution extracted with EtOAc (20 mL). The aqueous layer was acidified to pH 2 with conc. HCl and extracted into EtOAc (3 x 25 mL). The organic extracts were combined, dried (MgSO₄), filtered and evaporated to give 2-chloro-4,5-dimethyl phenyl acetic acid (86) as a yellow oil which crystallised on standing. Yield: 533 mg (70 %). ¹H NMR δ_{H} ppm (400 MHz, D₆-DMSO): 7.19 (1H, s), 7.10 (1H, s), 3.60 (2H, s), 2.19 (3H, s), 2.15 (3H, s).

2-Chloro-4,5-dimethyl phenyl acetic acid (86) (59 mg, 0.3 mmol) was treated with oxalyl chloride (56 µL, 0.6 mmol) and *N*-[4-(4,5-dihydro-1*H*-pyrazol-3-yl)-phenyl]-methanesulfonamide TFA salt (60) (82.5 mg, 0.3 mmol) using method F to give the final compound *N*-(4-{1-[2-(2-Chloro-4,5-dimethyl-phenyl)-acetyl]-4,5-dihydro-1*H-*pyrazol-3-yl}-phenyl)-methanesulfonamide (87). Yield: 9 mg (7 %). LC/MS *t*ᵣ 4.37 min. MS(ES+) *m*/*z* 420/422 (M+H). ¹H NMR δ_{H} ppm (400 MHz, CDCl₃): 7.63 (2H, d), 7.12-7.22 (2H, obs), 7.05 (2H, d), 4.11 (2H, s), 4.02 (2H, t), 3.11 (2H, t), 2.98 (3H, s), 2.12 (6H, s).

### Example 61 Propynoic acid (4-{1-[2-(2-bromo-4,5-dimethoxy-phenyl)-acetyl]-4,5-dihydro-1H-pyrazol-3-yl}-phenyl)-amide (88)

A solution of propiolic acid (4.4 µL, 0.07 mmol), 1-[3-(4-Amino-phenyl)-4,5-dihydro-pyrazol-1-yl]-2-(2-bromo-4,5-dimethoxy-phenyl)-ethanone (68) (27 mg, 0.06 mmol) and EDC (14 mg, 0.07 mmol) in DMF (0.5 mL) was stirred at RT for 18 h. The solvent was removed and the crude residue purified by column chromatography (50-100% EtOAc in heptane) to give the final compound propynoic acid (4-{1-[2-(2-bromo-4,5-dimethoxy-phenyl)-acetyl]-4,5-dihydro-1*H-*pyrazol-3-yl}-phenyl)-amide (88). Yield: 3 mg (10%). LC/MS *t*ᵣ 4.18 min. MS(ES+) *m*/*z* 470/472 (M+H). ¹H NMR δ_{H} ppm (400 MHz, CDCl₃): 7.79 (2H, d), 7.64 (3H, s+d), 7.08 (1H, s), 6.96 (1H, s), 4.23 (2H, s), 4.12 (2H, t), 3.89 (3H, s), 3.88 (3H, s), 3.27 (2H, t), 3.02 (1H, s).

### Example 62 N-(4-{1-[2-(2-Iodo-4,5-dimethoxy-phenyl)-acetyl]-4,5-dihydro-1H-pyrazol-3-yl}-phenyl)-methanesulfonamide (89)

*N-*(4-{1-[2-(3,4-dimethoxy-phenyl)-acetyl]-4,5-dihydro-1*H-*pyrazol-3-yl}-phenyl)-methane-sulfonamide (22) (104 mg, 0.25 mmol) and KI (47 mg, 0.28 mmol) were suspended in a mixture of acetonitrile (2 mL) and H₂O (0.8 mL). Oxone (154 mg, 0.25 mmol) was added portionwise over 45 min and the reaction then stirred at RT for 18 h. H₂O (2 mL) was added to the brownish suspension and the solid filtered and washed with further H₂O to give the final compound *N*-(4-{1-[2-(2-iodo-4,5-dimethoxy-phenyl)-acetyl]-4,5-dihydro-1*H*-pyrazol-3-yl}-phenyl)-methane-sulfonamide (89). Yield: 60 mg (44%) LC/MS *t*ᵣ 4.03 min. MS(ES+) *m*/*z* 544/545 (M+H). ¹H NMR δ_{H} ppm (400 MHz, CDCl₃): 7.70 (2H, d), 7.15 - 7.20 (3H, m), 6.86 (2H, brs), 4.13 (2H, s), 4.03 (2H, t), 3.77 (6H, s), 3.17 (2H, t), 2.99 (3H, s).

### Example 63 4-{1-[2-(2-Chloro-4,5-dimethoxy-phenyl)-acetyl]-4,5-dihydro-1H-pyrazol-3-yl}-phenyl-cyanamide (90)

To an externally cooled stirred suspension of 1-[3-(4-amino-phenyl)-4,5-dihydro-pyrazol-1-yl]-2-(2-chloro-4,5-dimethoxy-phenyl)-ethanone (73) (100 mg, 0.27 mmol) and triethylamine (55.9 µL, 0.40 mmol) in DCM (6 mL) was added cyanogen bromide (30 mg, 0.28 mmol) in DCM (2 mL) dropwise over 10 min. After a further 10 min the reaction mixture was warmed to RT and stirred over 3 days. H₂O (1 mL) was added to the reaction mixture cautiously and the solid was filtered and washed with DCM (2 x 5 mL). H₂O (5 mL) was added to the filtrate and the organic phase was dried (MgSO₄), filtered and evaporated. The crude product was purified by preparative HPLC to give the final compound 4-{1-[2-(2-chloro-4,5-dimethoxy-phenyl)-acetyl]-4,5-dihydro-1*H*-pyrazol-3-yl}-phenyl-cyanamide (90) as a solid. Yield: 8 mg (8%). LC/MS *t*ᵣ 4.04 min. MS(ES+) *m*/*z* 399/401 (M+H). ¹H NMR δ_{H} ppm (250 MHz, D₆-DMSO): 7.85 (2H, d), 7.16 (2H, d), 7.03 (1H, s), 6.94 (1H, s), 4.11 (2H, s), 3.99 (2H, t), 3.82 (3H, s), 3.77 (3H, s), 3.34 (2H, t).

### Reference

### Example 64 1-[3-(2-Amino-benzothiazo]-6-yl)-4,5-dihydro-pyrazol-1-yl]-2-(2-chloro-4,5-dimethoxy-phenyl)-ethanone (91)

To a stirred solution of 1-[3-(4-amino-phenyl)-4,5-dihydro-pyrazol-1-yl]-2-(2-chloro-4,5-dimethoxy-phenyl)-ethanone (73) (250 mg, 0.67 mmol) and potassium thiocyanate (200 mg, 2.07 mmol) in acetic acid (1.34 mL) at RT was added bromine (34.4 µL, 0.67 mmol) in acetic acid (0.5 mL) over 8 min. A further amount of acetic acid (0.5 mL) was added and the reaction mixture stirred for 22 h. MTBE (6 mL) was added to the reaction mixture and the solid was filtered and washed with MTBE (4 mL). The collected solid was partially dissolved in an excess of MeOH (< 100 mL) and 2M NaOH (4 mL) and combined with the MTBE extracts. The organics were then evaporated and H₂O was added. The solid was filtered, washed with H₂O (x 2) and partially dried. The solid was then triturated in DCM (5 mL), filtered, washed with DCM (1 x 5 mL) and dried to give the final compound 1-[3-(2-amino-benzothiazol-6-yl)-4,5-dihydro-pyrazol-1-yl]-2-(2-chloro-4,5-dimethoxy-phenyl)-ethanone (91)as a solid. Yield: 133 mg (46%). LC/MS *t*ᵣ 3.76 min. MS(ES+) *m*/*z* 431/433 (M+H). ¹H NMR δ_{H} ppm (250 MHz, D₆-DMSO): 8.08 (1H, s), 7.74-7.65 (3H, m), 7.36 (1H, d), 7.02 (1H, s), 6.98 (1H, s), 4.06 (2H, s), 3.90 (2H, t), 3.75 (3H, s), 3.71 (3H, s), 3.25 (2H, obs).

### Example 65 2-(2-Chloro-4,5-dimethoxy-phenyl)-1-[3-(4-methylamino-phenyl)-4,5-dihydro-pyrazol-1-yl]-ethanone (95)

Sodium hydride (60% dispersion in mineral oil, 672 mg,16.8 mmol) was added portionwise to MeOH (8 mL) and stirred until gas evolution had ceased. 3-(4-Amino-phenyl)-4,5-dihydro-pyrazole-1-carboxylic acid *tert*-butyl ester (56) (1.04 g, 3.98 mmol) was added followed by paraformaldehyde (168 mg, 5.60 mmol) in MeOH (4 mL) and the resulting solution was stirred for 5 h. NaBH₄ (144 mg, 3.81 mmol) was then added and the mixture heated to reflux for a further 1 h. To the cooled reaction mixture was added 1M KOH (5 mL) and stirred for 18 h. After this time, H₂O (25 mL) and EtOAc (25 mL) were added. The aqueous layer was re-extracted with EtOAc (1 x 25 mL) and the combined organics were washed with brine (1 x 20 mL), dried (MgSO₄), filtered and evaporated to give 3-[4-methylamino)-phenyl]-4,5-dihydro-pyrazole-1-carboxylic acid *tert-*butyl ester (92). Yield: 1.4 g, (100%). LC/MS *t*ᵣ 1.88 min. MS(ES+) *m*/*z* 573 (2M+Na), 276 (M+H), 220 (M+H - 56)

3-[4-Methylamino)-phenyl]-4,5-dihydro-pyrazole-1-carboxylic acid *tert*-butyl ester (92) (275 mg, 1.00 mmol), trifluoroacetic anhydride (232 mg, 1.10 mmol) and pyridine (2 mL) were combined and stirred at RT for 18 h. The solvent was evaporated then redissolved in EtOAc (100 mL) and washed with 5% citric acid solution (2 x 50 mL). The organic layer was dried (MgSO₄), filtered and evaporated to give the crude residue which was purified by column chromatography (100% EtOAc) to give 3-{4-[methyl-(2,2,2-trifluoro-acetyl)-amino]-phenyl}-4,5-dihydro-pyrazole-1-carboxylic acid *tert*-butyl ester (93). Yield: 172 mg, (46%). LC/MS *t*ᵣ 2.12 min. MS(ES+) *m*/*z* 765 (2M+Na), 315 (M+H - 56).

A solution of 3-{4-[methyl-(2,2,2-trifluoro-acetyl)-amino]-phenyl}-4,5-dihydro-pyrazole-1-carboxylic acid *tert*-butyl ester (93) (170 mg, 0.46 mmol) in conc. HCl (0.6 mL) and MeOH (3 mL) was heated to 60 °C for 1 h. The solvent was evaporated to give *N*-[4-(4,5-dihydro-1*H*-pyrazol-3-yl)-phenyl]-2,2,2-trifluoro-*N*-methyl-acetamide hydrochloride salt (94) as a 7:3 mixture with the des trifluoroacetyl product. Yield: crude 87 mg, (62%) LC/MS *t*ᵣ 1.21 min MS(ES+) *m*/*z* 272 (M+H).

To a stirred solution of 2-chloro-4-5-dimethoxyphenyl acetic acid (3) (27 mg, 0.12 mmol) in DCM (2 mL) was added DMF (2 drops) and oxalyl chloride (30 mg, 0.24 mmol) at RT and stirred for 1 h. The solvent and excess oxalyl chloride were evaporated and the residue was re-dissolved in THF (2 mL). To this solution was added *N*-[4-(4,5-dihydro-1*H*-pyrazol-3-yl)-phenyl]-2,2,2-trifluoro-*N-*methyl-acetamide hydrochloride salt (94) (40 mg, 0.13 mmol) and Et₃N (20 µL, 0.14 mmol) and the resulting solution was stirred for 1 h at RT. H₂O (10 mL) was added and the organics extracted into DCM (3 x 10 mL) and EtOAc (2 x 10 mL). The organic extracts were combined, dried (MgSO₄), filtered and evaporated to give the crude residue which was purified by preparative HPLC to give the final compound 2-(2-chloro-4,5-dimethoxy-phenyl)-1-[3-(4-methylamino-phenyl)-4,5-dihydro-pyrazol-1-yl]-ethanone (95). Yield: 1 mg (2%). LC/MS *t*ᵣ 4.24 min. MS(ES+) *m*/*z* 388/390 (M+H). ¹H NMR δ_{H} ppm (250 MHz, CDCl₃): 7.53 (2H, d), 6.86 (1H, s), 6.81 (1H, s), 6.54 (2H, d), 4.10 (2H, s), 3.96 (2H, t), 3.78 (7H, s), 3.12 (2H, t), 2.82 (3H, s).

### Example 66 2-(2-Bromo-4,5-dimethoxy-phenyl)-1-[3-(4-methylamino-phenyl)-4,5-dihydro-pyrazol-1-yl]-ethanone (96)

A solution of (94) (20 mg, 0.07 mmol), HATU (25 mg, 0.07 mmol), Et₃N (27 µL, 0.20 mmol) and DMF (2 mL) were combined and stirred at RT for 2 h. H₂O (4 mL) was added and the reaction mixture was extracted into EtOAc (3 x 15 mL), the combined organics were dried (MgSO₄), filtered and evaporated to give the crude residue. This was dissolved in MeOH (5 mL) then 2M NaOH (1 mL) was added and the solution stirred for 3 h at RT. The solvent was evaporated and the residue redissolved in DCM (10 mL) and washed with brine (1 x 5 mL). The organic layer was dried (MgSO₄), filtered and evaporated to give the crude product which was purified by dry flash chromatography (100% EtOAc) to give the final compound 2-(2-Bromo-4,5-dimethoxy-phenyl)-1-[3-(4-methylamino-phenyl)-4,5-dihydro-pyrazol-1-yl]-ethanone (96) as a solid. Yield: 13 mg (46%). LC/MS *t*ᵣ 4.26 min. MS(ES+) *m*/*z* 432/434 (M+H). ¹H NMR δ_{H} ppm (250 MHz, CDCl₃): 7.61 (2H, d), 7.04 (1H, s), 6.95 (1H, s), 6.61 (2H, d), 4.19 (2H, s), 4.07-3.99 (3H, m), 3.86 (3H, s), 3.85 (3H, s), 3.20 (2H, t), 2.90 (3H, d).

### Example 67 Wnt assay

10T1/2 Super8xTopflash reporter cell line was generated by stable transfection of a Super8xTopflash reporter plasmid containing 8 tandem repeats of TCF4 binding sites upstream of Luciferase gene. Cells were seeded at 20K/well density (in normal growth medium with 10% FBS and 0.4 mg/ml G418) in white 96-well plates with clear bottom (BD Falcon Microtest Optilux plate, Ref. 353947) and grown for 20-24 hours. Fesh growth medium was prepared without G418 and with appropriate Wnt3a concentration (concentration that gave 40-50% of maximum activation based on the dose-response curve performed for each new batch of protein; typically ∼0.1 ug/ml).

Old medium was removed from the 96-well plate, which was gently blotted on paper towel, then all relevant wells were replenished with fresh medium containing Wnt (100 ul/well).

The wells with highest compound concentrations (usually 10 or 30 uM) were filled separately with 150 ul of prepared medium containing both Wnt3a and the test compounds. Using a multichannel pipetman, 50 ul was drawn from these wells and dispensed and mixed gently into the next row of wells to achieve 1:3 dilution of compounds. Dilution was continued achieve the desired concentration.

After 20-24 h the cells were lysed and the luciferase signal was read using Perkin Elmer luclite assay system, Cat.# 6016911). One vial of lyophilized substrate was reconstituted in 10.5 ml substrate buffer, and mixed with 10.5 ml PBS containing 1 mM Mg²⁺ and 1 mM Ca²⁺. The medium was then removed from the 96-well plate and gently blotted on paper towel, and 100 ul prepared substrate was added to each well. Approximately 15 min later the plates were read on a TopCount machine.

Compounds of the invention that were tested in the Super8xTopflash reporter assay were found to inhibit Wnt pathway signaling activity with an IC₅₀ of less than 2µM. For example, Wnt signaling activity was inhibit by compound 42 with an IC₅₀ of 0.107 µM, compound 44 with an IC₅₀ of 0.131 µM, compound 45 with an IC₅₀ of 0.144 µM, compound 3 with an IC₅₀ of 0.155 µM, compound 32 with an IC₅₀ of 0.170 µM and compound 12 with an IC₅₀ of 0.218 µM. In a particular embodiment, compounds of the invention inhibit Wnt signaling activity with an with an IC₅₀ of less than 0.5µM. In a particular embodiment, compounds of the invention inhibit Wnt signaling activity with an with an IC₅₀ of less than 0.1µM. In a particular embodiment, compounds of the invention inhibit Wnt signaling activity with an with an IC₅₀ of less than 0.05µM. In a particular embodiment, compounds of the invention inhibit Wnt signaling activity with an with an IC₅₀ of less than 0.01µM.

## Claims

1. A compound of formula (I): 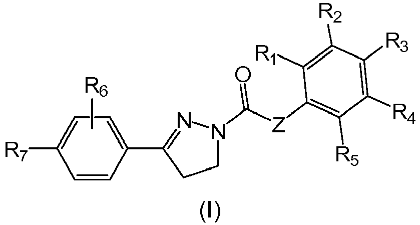 wherein:
R₁, R₄, and R₅ are H; or
R₂ and R₅ are H;
the others of R₁, R₂, R₃, R₄, and R₅ are independently halogen or alkoxy;
Z is -CH₂-;
R₆ is H, alkyl, or halogen;
R₇ is OH, -NH-Y, -NR₈-C(O)-Y, -NR₈-C(O)-NR₈-Y, or -NR₈-S(O)₂-Y;
R₈ is H or alkyl; and
Y is H, cyano, alkyl, a carbocycle or a heterocyle; wherein said alkyl is optionally substituted with halogen, hydroxyl, alkoxy, amino, nitro, cyano, carboxyl, sulfonyl, a carbocycle or a heterocycle; wherein said carbocycles and heterocycles are optionally substituted with hydroxyl, halogen, alkyl or haloalkyl.

2. A compound according claim 1, wherein R₁, R₄, and R₅ are H.

3. A compound according claim 1, wherein R₂ and R₅ are H.

4. A compound according claim 3, wherein:
R₁ is halogen; and
R₃ and R₄ are alkoxy.

5. A compound according to any one of claims 1 to 3, wherein the others of R₁, R₂, R₃, R₄, and R₅ are independently chloro, fluoro, bromo, methoxy, or ethoxy.

6. A compound according to any one of claims 1 to 5, wherein R₆ is H, Me, or F.

7. A compound according to any one of claims 1 to 5, wherein R₆ is H.

8. A compound according to any one of claims 1 to 7, wherein R₇ is -NR₈-C(O)-Y.

9. A compound according to any one of claims 1 to 7, wherein R₇ is -NR₈-C(O)-NR₈-Y.

10. A compound according to any one of claims 1 to 7, wherein R₇ is -NR₈-S(O)₂-Y.

11. A compound according to any one of claims 1 to 10, wherein R₈ is H or Me.

12. A compound according to any one of claims 1 to 7, wherein R₇ is -NH-Y.

13. A compound according to any one of claims 1 to 12, wherein Y is H, alkyl, cycloalkyl, heteroaryl or alkoxyakyl.

14. A compound according to any one of claims 1 to 12, wherein Y is H.

15. A compound according to any one of claims 1 to 12, wherein Y is alkyl.

16. A compound according to any one of claims 1 to 12, wherein Y is methyl.

17. A compound according to any one of claims 1 to 12, wherein Y is cycloalkyl.

18. A compound according to any one of claims 1 to 12, wherein Y is heteroaryl

19. A compound according to any one of claims 1 to 7, wherein R₇ is OH.

20. A pharmaceutical composition comprising a compound according to any one of claims 1 to 19, and a pharmaceutically acceptable carrier, diluent or excipient.

21. A compound according to any one of claims 1 to 19, for use in a method of treatment of the human or animal body by therapy.

22. A compound for use according to claim 21, wherein the method is a method of treatment of a disease or condition associated with Wnt pathway signaling in a mammal, cancer, colorectal cancer, or breast cancer.

23. Use of a compound according to any one of claims 1 to 19 in the manufacture of a medicament for the treatment of a disease or condition associated with Wnt pathway signaling in a mammal, cancer, colorectal cancer, or breast cancer.

## Patentansprüche

1. Verbindung der Formel (I): 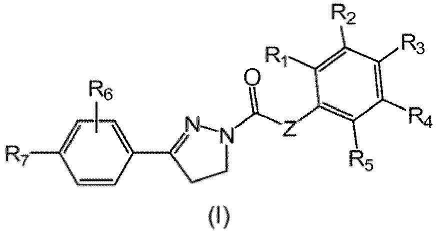 worin:
R₁, R₄ und R₅ H sind; oder
R₂ und R₅ H sind;
die anderen Reste von R₁, R₂, R₃, R₄ und R₅ unabhängig voneinander Halogen oder Alkoxy sind;
Z -CH₂- ist;
R₆ H, Alkyl oder Halogen ist;
R₇ OH, -NH-Y, -NR₈-C(O)-Y, -NR₈-C(O)-NR₈-Y oder -NR₈-S(O)₂-Y ist;
R₈ H oder Alkyl ist und
Y H, Cyano, Alkyl, ein Carbozyklus oder ein Heterozyklus ist; worin das Alkyl gegebenenfalls mit Halogen, Hydroxyl, Alkoxy, Amino, Nitro, Cyano, Carboxyl, Sulfonyl, einem Carbozyklus oder einem Heterozyklus substituiert ist; worin die Carbozyklen und Heterozyklen gegebenenfalls mit Hydroxyl, Halogen, Alkyl oder Haloalkyl substituiert sind.

2. Verbindung nach Anspruch 1, worin R₁, R₄ und R₅ H sind.

3. Verbindung nach Anspruch 1, worin R₂ und R₅ H sind.

4. Verbindung nach Anspruch 3, worin:
R₁ Halogen ist; und
R₃ und R₄ Alkoxy sind.

5. Verbindung nach einem der Ansprüche 1 bis 3, worin die anderen Reste von R₁, R₂, R₃, R₄ und R₅ unabhängig voneinander Chlor, Fluor, Brom, Methoxy oder Ethoxy sind.

6. Verbindung nach einem der Ansprüche 1 bis 5, worin R₆ H, Me oder F ist.

7. Verbindung nach einem der Ansprüche 1 bis 5, worin R₆ H ist.

8. Verbindung nach einem der Ansprüche 1 bis 7, worin R₇ -NR₈-C(O)-Y ist.

9. Verbindung nach einem der Ansprüche 1 bis 7, worin R₇ -NR₈-C(O)-NR₈-Y ist.

10. Verbindung nach einem der Ansprüche 1 bis 7, worin R₇ -NR₈-S(O)₂-Y ist.

11. Verbindung nach einem der Ansprüche 1 bis 10, worin R₈ H oder Me ist.

12. Verbindung nach einem der Ansprüche 1 bis 7, worin R₇ -NH-Y ist.

13. Verbindung nach einem der Ansprüche 1 bis 12, worin Y H, Alkyl, Cycloalkyl, Heteroaryl oder Alkoxyalkyl ist.

14. Verbindung nach einem der Ansprüche 1 bis 12, worin Y H ist.

15. Verbindung nach einem der Ansprüche 1 bis 12, worin Y Alkyl ist.

16. Verbindung nach einem der Ansprüche 1 bis 12, worin Y Methyl ist.

17. Verbindung nach einem der Ansprüche 1 bis 12, worin Y Cycloalkyl ist.

18. Verbindung nach einem der Ansprüche 1 bis 12, worin Y Heteroaryl ist.

19. Verbindung nach einem der Ansprüche 1 bis 7, worin R₇ OH ist.

20. Pharmazeutische Zusammensetzung, die eine Verbindung nach einem der Ansprüche 1 bis 19 und einen pharmazeutisch annehmbaren Träger, ein pharmazeutisch annehmbares Verdünnungsmittel oder einen pharmazeutisch annehmbaren Exzipienten umfasst.

21. Verbindung nach einem der Ansprüche 1 bis 19 zur Verwendung in einem Verfahren zur therapeutischen Behandlung des menschlichen oder tierischen Körpers.

22. Verbindung zur Verwendung nach Anspruch 21, worin das Verfahren ein Verfahren zur Behandlung einer Erkrankung oder eines Zustands in Zusammenhang mit der Wnt-Signalübertragung in einem Säugetier, von Krebs, von Kolorektalkrebs oder von Brustkrebs ist.

23. Verwendung einer Verbindung nach einem der Ansprüche 1 bis 19 zur Herstellung eines Medikaments zur Behandlung einer Erkrankung oder eines Zustands in Zusammenhang mit der Wnt-Signalübertragung in einem Säugetier, von Krebs, von Kolorektalkrebs oder von Brustkrebs.

## Revendications

1. Composé de formule (I) : 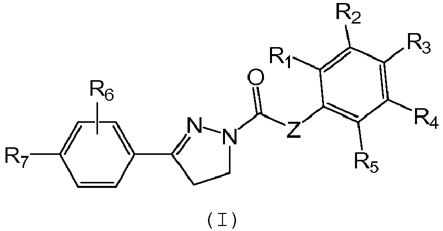 dans laquelle :
R₁, R₄ et R₅ sont H ; ou
R₂ et R₅ sont H ;
les autres parmi R₁, R₂, R₃, R₄ et R₅ sont indépendamment un halogène ou un alcoxy ;
Z est -CH₂- ;
R₆ est H, un alkyle ou un halogène ;
R₇ est OH, -NH-Y, -NR₈-C(O)-Y, -NR₈-C(O)-NR₈-Y ou -NR₈-S(O)₂-Y ;
R₈ est H ou un alkyle ; et
Y est H, cyano, un alkyle, un carbocycle ou un hétérocycle ; ledit alkyle étant éventuellement substitué par un halogène, hydroxyle, alcoxy, amino, nitro, cyano, carboxyle, sulfonyle, carbocycle ou hétérocycle ; et lesdits carbocycles et hétérocycles étant éventuellement substitués par un halogène, hydroxyle, alkyle ou halogénoalkyle.

2. Composé selon la revendication 1, dans lequel R₁, R₄ et R₅ sont H.

3. Composé selon la revendication 1, dans lequel R₂ et R₅ sont H.

4. Composé selon la revendication 3, dans lequel :
R₁ est un halogène ; et
R₃ et R₄ sont des radicaux alcoxy.

5. Composé selon l'une quelconque des revendications 1 à 3, dans lequel les autres parmi R₁, R₂, R₃, R₄ et R₅ sont indépendamment chloro, fluoro, bromo, méthoxy ou éthoxy.

6. Composé selon l'une quelconque des revendications 1 à 5, dans lequel R₆ est H, Me ou F.

7. Composé selon l'une quelconque des revendications 1 à 5, dans lequel R₆ est H.

8. Composé selon l'une quelconque des revendications 1 à 7, dans lequel R₇ est -NR₈-C(O)-Y.

9. Composé selon l'une quelconque des revendications 1 à 7, dans lequel R₇ est -NR₈-C(O)-NR₈-Y.

10. Composé selon l'une quelconque des revendications 1 à 7, dans lequel R₇ est -NR₈-S(O)₂-Y.

11. Composé selon l'une quelconque des revendications 1 à 10, dans lequel R₈ est H ou Me.

12. Composé selon l'une quelconque des revendications 1 à 7, dans lequel R₇ est -NH-Y.

13. Composé selon l'une quelconque des revendications 1 à 12, dans lequel Y est H ou un alkyle, cycloalkyle, hétéroaryle ou alcoxyalkyle.

14. Composé selon l'une quelconque des revendications 1 à 12, dans lequel Y est H.

15. Composé selon l'une quelconque des revendications 1 à 12, dans lequel Y est un alkyle.

16. Composé selon l'une quelconque des revendications 1 à 12, dans lequel Y est méthyle.

17. Composé selon l'une quelconque des revendications 1 à 12, dans lequel Y est un cycloalkyle.

18. Composé selon l'une quelconque des revendications 1 à 12, dans lequel Y est un hétéroaryle.

19. Composé selon l'une quelconque des revendications 1 à 7, dans lequel R₇ est OH.

20. Composition pharmaceutique comprenant un composé selon l'une quelconque des revendications 1 à 19 et un véhicule, diluant ou excipient pharmaceutiquement acceptable.

21. Composé selon l'une quelconque des revendications 1 à 19, pour une utilisation dans une méthode de traitement thérapeutique du corps humain ou animal.

22. Composé pour une utilisation selon la revendication 21, dans lequel la méthode est une méthode de traitement d'une maladie ou d'un état associé à la signalisation de la voie de Wnt chez un mammifère, d'un cancer, d'un cancer colorectal, ou d'un cancer du sein.

23. Utilisation d'un composé selon l'une quelconque des revendications 1 à 19 dans la fabrication d'un médicament destiné au traitement d'une maladie ou d'un état associé à la signalisation de la voie de Wnt chez un mammifère, d'un cancer, d'un cancer colorectal, ou d'un cancer du sein.
